# DEMANDE DE BREVET EUROPEEN

(11) **EP 1 849 869 A2**
(43) Date de publication de la demande: **31.10.2007**
(21) Numéro de dépôt: 07003648.8
(22) Date de dépôt: 20.10.1995
(51) Int. Cl.: C12N 15/49, C07K 14/16, A61K 39/21, C12N 5/10, C12N 15/86, C12N 7/00, G01N 33/50, C12Q 1/70

(54) **Séquences nucléotidiques d'antigènes rétroviraux VIH-1 groupe (ou sous-groupe) 0**

(30) Priorité: 20.10.1994 FR 9412554; 03.03.1995 FR 9502526
(62) Demande divisionnaire de: 95935996.9
(71) Demandeur: INSTITUT PASTEUR, 75724 Paris Cédex 15 (FR)
(72) Inventeur: Charneau, Pierre, 75005 Paris (FR); Clavel, François, 75016 Paris (FR); Borman, Andrew, 28210 Nogent le Roi (FR); Quillent, Caroline, 94400 Viry sur Seine (FR); Guetard, Denise, 75015 Paris (FR); Montagnier, Luc, 92000 Le Plessis-Robinson (FR); Donjon de Saint-Martin, Jacqueline, 92140 Clamart (FR); Cohen, Jacques H.M., 51100 Reims (FR)
(74) Mandataire: Desaix, Anne

(57) **Abrégé**

L'invention concerne une protéine de rétrovirus VIH-1 groupe (ou sous-groupe) O, ou polypeptide ou peptide naturel ou synthétique comprenant au moins une partie de ladite protéine, susceptible d'être reconnue par des anticorps isolables à partir de sérum obtenu suite à une infection par une souche VIH-1 groupe O VAU, ou une souche VIH-1 groupe (ou sous-groupe) O DUR.

### Annex to the application documents - subsequently filed sequences listing

SEQUENCE LISTING

<110>  INSTITUT PASTEUR

<120>  SEQUENCES NUCLEOTIDIQUES D'ANTIGENES RETROVIRAUX VIH-1 GROUPE (OU
        SOUS-GROUPE) O

<130>  B2482AAA-AD/LV/SDU

<140>  EP 07003648.8
<141>  2007-02-22

<150>  FR 9412554
<151>  1994-10-20

<150>  FR 9502526
<151>  1995-03-03

<150>  EP 95935996.9
<151>  1995-10-20

<150>  PCT/FR95/01391
<151>  1995-10-20

<160>  103

<170>  PatentIn version 3.3

<210>  1
<211>  23
<212>  DNA
<213>  Artificial

<220>
<223>  Amorce 4506

<400>  1
agtggatwya tagaagcaga agt                                              23


<210>  2
<211>  20
<212>  DNA
<213>  Artificial

<220>
<223>  Amorce 5011

<400>  2
actgcycctt chcctttcca                                                  20


<210>  3
<211>  28
<212>  DNA
<213>  Artificial

<220>
<223>  Amorce TH2

<400>  3
gctctagatg gggatctccc atggcagg                                         28


<210>  4
<211>  30
<212>  DNA
<213>  Artificial

<220>
<223>  Amorce UH2

<400>  4
gctctagatc agggaagaat ccctgagtgt                                    30


<210>  5
<211>  2631
<212>  DNA
<213>  Human immunodeficiency virus VAU

<400>  5
atgacagcga ttatgaaagc aatggggaag aggaacagga agttagggat ctggtgcttg      60

attttggctt tgataatccc atgtttgagc tgtaaccaac tatatgccac agtctattct     120

ggggtacctg tatgggaaga tgcaaaacca acattgttct gtgcttcaga tgctaacttg     180

acaagcactg aacagcataa tatttgggca acacaagcct gtgttcccac agaccccagt     240

ccaaatgaat atgagctaaa aaatgtgaca ggtaaattca atatatggaa aaattatata     300

gtagaccaaa tgcacgaaga cattatagat ttgtgggacc agagtttaaa accttgtgtt     360

caaatgactt tcttgtgtgt acaaatgaat tgtacagata tcaaaaatag tattaatacc     420

acaaacagtc ccttaaactc aaacaataca aaagaggtga aacagtgtga ctttaatgta     480

actacagtgc tcaaagacaa acaggagaaa aaacaggctc tattctatgt gacagatttg     540

gttaagatta acgccacatc aaatgaaaca atgtatagat taattaattg taactccaca     600

accatcaggc aggcctgtcc aaaggtatct tttgagccca ttcccataca ctattgtgct     660

ccagcgggat gtgccatctt taagtgtaat gaaacaggat ttaatggaac aggtctctgt     720

aaaaacgtta cagtagttac ttgtacacat ggcatcaaac caacagtaag tacccaacta     780

atactaaatg ggacactctc taaaggaaat ataacaatca tgggaaagaa tatttcagac     840

agtggggaga acatcctaat aaccctaaat actaatataa caatagcatg tgagagacca     900

ggaaatcaga caatacaaaa gataatggca ggtccaatgg cttggtacag catggccctt     960

agtaatacaa agggggatac aagggcagct tattgtaatt atagtgccac tgactggaac    1020

aaagccttaa aaaacataac tgaaagatat ttagaacttg tagaatataa tcaaactgat    1080

gttaccatga aattcggtaa tcacagtggt gaagatgcag aagtaacaaa tttctttttt    1140

aactgtcatg gagaattctt ttattgtaac acaaatcggc tgtttaatca taccttttcc    1200

tgcaagaaga atatgaccaa taacaagatc aattgtacta atattagcaa taatagcaat    1260

ggcactcagg caataccttg caggttgaga caagtagtaa gggactggat gaggggagga    1320

tcgggacttt atgcacctcc catcccagga aacctagtat gcaggtcaaa cataactgga    1380

atgattctac aattggacac gccatggaat aaaacacatc ctaacagcac cacccttaga    1440

ccaggagggg gagatatgaa agatatatgg agaactcaat tgttcaaata taaagtagta    1500

agagtaaaac cttttagtgt agcaccaaca aaaattgcaa ggccaactat aggaactaga    1560

tctcatagag agaaaagagc agcaggtttg gcaatgctat tcttggggat tctaagtgca    1620

gcaggaagca ctatgggcgc agcggcaaca gcgctgacgg tacggaccca gcatctgata 1680

aagggtatag tgcaacagca ggataacctg ctaagagcaa tacaggccca gcaacacttg 1740

ctgaggccat ctgtatgggg tattagacaa ctccgagctc gcctgctagc cttagaaacc 1800

tttatacaga atcagcaact ccttaacctg tggggctgca agaatagact aatctgctac 1860

acatcagtaa agtggaataa aacatgggga ggagataatg aatcaatttg ggatgagtta 1920

acatggcagc agtgggatca acagataaac aacgtaagct ccttcatata tgaaaaaata 1980

caagaggcac aagaacaaca ggagaaaaat gagaaagaat tgctggagtt agatgaatgg 2040

gcctctattt ggaattggct tgacataact aaatggttgt ggtatataaa aatagctata 2100

atcatagtag gagcactaat aggtgtaaga gtagttatga tagtacttaa tctagtaaag 2160

aacattaggc agggatatca acccctctcg ttacagatcc ccatccaaca acaagcggaa 2220

gtaggaacgc caggaggaac aggagaagga ggtggagacg aagacaggcg caggtggact 2280

ccattgccgc aagggttctt gcatctgttg tacacggacc tcaggacaat aatcttgtgg 2340

atttaccacc tcttgagcaa cttagcctca gagatccaga agttgatcag acacctggga 2400

cttggactat ggatcatagg gcagaggaca attgaagctt gcagactctt taaagctata 2460

atacaatact ggctacaaga attgcaaact agtgctacaa atctactaga tactgttgca 2520

gtggcagttg ctaattggac tgacagcaca atcttaggca tacaaagcat agggagaggg 2580

attcttaaca taccaagaag gattagacag ggccttgaac gactcctgtt a 2631

<210> 6
<211> 877
<212> PRT
<213> Human immunodeficiency virus VAU

<400> 6

Met Thr Ala Ile Met Lys Ala Met Gly Lys Arg Asn Arg Lys Leu Gly
1               5                   10                  15

Ile Trp Cys Leu Ile Leu Ala Leu Ile Ile Pro Cys Leu Ser Cys Asn
            20                  25                  30

Gln Leu Tyr Ala Thr Val Tyr Ser Gly Val Pro Val Trp Glu Asp Ala
        35                  40                  45

Lys Pro Thr Leu Phe Cys Ala Ser Asp Ala Asn Leu Thr Ser Thr Glu
    50                  55                  60

Gln His Asn Ile Trp Ala Thr Gln Ala Cys Val Pro Thr Asp Pro Ser
65                  70                  75                  80

Pro Asn Glu Tyr Glu Leu Lys Asn Val Thr Gly Lys Phe Asn Ile Trp
                85                  90                  95

Lys Asn Tyr Ile Val Asp Gln Met His Glu Asp Ile Ile Asp Leu Trp
            100                 105                 110

Asp Gln Ser Leu Lys Pro Cys Val Gln Met Thr Phe Leu Cys Val Gln
        115                 120                 125

Met Asn Cys Thr Asp Ile Lys Asn Ser Ile Asn Thr Thr Asn Ser Pro
    130                 135                 140

Leu Asn Ser Asn Asn Thr Lys Glu Val Lys Gln Cys Asp Phe Asn Val
145                 150                 155                 160

Thr Thr Val Leu Lys Asp Lys Gln Glu Lys Lys Gln Ala Leu Phe Tyr
                165                 170                 175

Val Thr Asp Leu Val Lys Ile Asn Ala Thr Ser Asn Glu Thr Met Tyr
            180                 185                 190

Arg Leu Ile Asn Cys Asn Ser Thr Thr Ile Arg Gln Ala Cys Pro Lys
        195                 200                 205

Val Ser Phe Glu Pro Ile Pro Ile His Tyr Cys Ala Pro Ala Gly Cys
    210                 215                 220

Ala Ile Phe Lys Cys Asn Glu Thr Gly Phe Asn Gly Thr Gly Leu Cys
225                 230                 235                 240

Lys Asn Val Thr Val Val Thr Cys Thr His Gly Ile Lys Pro Thr Val
                245                 250                 255

Ser Thr Gln Leu Ile Leu Asn Gly Thr Leu Ser Lys Gly Asn Ile Thr
            260                 265                 270

Ile Met Gly Lys Asn Ile Ser Asp Ser Gly Glu Asn Ile Leu Ile Thr
        275                 280                 285

Leu Asn Thr Asn Ile Thr Ile Ala Cys Glu Arg Pro Gly Asn Gln Thr
    290                 295                 300

Ile Gln Lys Ile Met Ala Gly Pro Met Ala Trp Tyr Ser Met Ala Leu
305                 310                 315                 320

Ser Asn Thr Lys Gly Asp Thr Arg Ala Ala Tyr Cys Asn Tyr Ser Ala
                325                 330                 335

Thr Asp Trp Asn Lys Ala Leu Lys Asn Ile Thr Glu Arg Tyr Leu Glu
            340                 345                 350

Leu Val Glu Tyr Asn Gln Thr Asp Val Thr Met Lys Phe Gly Asn His
        355                 360                 365

Ser Gly Glu Asp Ala Glu Val Thr Asn Phe Phe Phe Asn Cys His Gly
    370                 375                 380

Glu Phe Phe Tyr Cys Asn Thr Asn Arg Leu Phe Asn His Thr Phe Ser
385                 390                 395                 400

Cys Lys Lys Asn Met Thr Asn Asn Lys Ile Asn Cys Thr Asn Ile Ser
                405                 410                 415

Asn Asn Ser Asn Gly Thr Gln Ala Ile Pro Cys Arg Leu Arg Gln Val
            420                 425                 430

Val Arg Asp Trp Met Arg Gly Gly Ser Gly Leu Tyr Ala Pro Pro Ile
        435                 440                 445

Pro Gly Asn Leu Val Cys Arg Ser Asn Ile Thr Gly Met Ile Leu Gln
    450                 455                 460

Leu Asp Thr Pro Trp Asn Lys Thr His Pro Asn Ser Thr Thr Leu Arg
465                 470                 475                 480

Pro Gly Gly Gly Asp Met Lys Asp Ile Trp Arg Thr Gln Leu Phe Lys
                485                 490                 495

Tyr Lys Val Val Arg Val Lys Pro Phe Ser Val Ala Pro Thr Lys Ile
            500                 505                 510

Ala Arg Pro Thr Ile Gly Thr Arg Ser His Arg Glu Lys Arg Ala Ala
        515                 520                 525

Gly Leu Ala Met Leu Phe Leu Gly Ile Leu Ser Ala Ala Gly Ser Thr
    530                 535                 540

Met Gly Ala Ala Ala Thr Ala Leu Thr Val Arg Thr Gln His Leu Ile
545                 550                 555                 560

Lys Gly Ile Val Gln Gln Gln Asp Asn Leu Leu Arg Ala Ile Gln Ala
                565                 570                 575

Gln Gln His Leu Leu Arg Pro Ser Val Trp Gly Ile Arg Gln Leu Arg
            580                 585                 590

Ala Arg Leu Leu Ala Leu Glu Thr Phe Ile Gln Asn Gln Gln Leu Leu
        595                 600                 605

Asn Leu Trp Gly Cys Lys Asn Arg Leu Ile Cys Tyr Thr Ser Val Lys
    610                 615                 620

Trp Asn Lys Thr Trp Gly Gly Asp Asn Glu Ser Ile Trp Asp Glu Leu
625                 630                 635                 640

Thr Trp Gln Gln Trp Asp Gln Gln Ile Asn Asn Val Ser Ser Phe Ile
                645                 650                 655

Tyr Glu Lys Ile Gln Glu Ala Gln Glu Gln Gln Glu Lys Asn Glu Lys
            660                 665                 670

Glu Leu Leu Glu Leu Asp Glu Trp Ala Ser Ile Trp Asn Trp Leu Asp
        675                 680                 685

Ile Thr Lys Trp Leu Trp Tyr Ile Lys Ile Ala Ile Ile Ile Val Gly
    690                 695                 700

Ala Leu Ile Gly Val Arg Val Val Met Ile Val Leu Asn Leu Val Lys
705                 710                 715                 720

Asn Ile Arg Gln Gly Tyr Gln Pro Leu Ser Leu Gln Ile Pro Ile Gln
                725                 730                 735

Gln Gln Ala Glu Val Gly Thr Pro Gly Gly Thr Gly Glu Gly Gly Gly
            740                 745                 750

Asp Glu Asp Arg Arg Arg Trp Thr Pro Leu Pro Gln Gly Phe Leu His
        755                 760                 765

Leu Leu Tyr Thr Asp Leu Arg Thr Ile Ile Leu Trp Ile Tyr His Leu
    770                 775                 780

Leu Ser Asn Leu Ala Ser Glu Ile Gln Lys Leu Ile Arg His Leu Gly
785                 790                 795                 800

Leu Gly Leu Trp Ile Ile Gly Gln Arg Thr Ile Glu Ala Cys Arg Leu
                805                 810                 815

Phe Lys Ala Ile Ile Gln Tyr Trp Leu Gln Glu Leu Gln Thr Ser Ala
            820                 825                 830

Thr Asn Leu Leu Asp Thr Val Ala Val Ala Val Ala Asn Trp Thr Asp
        835                 840                 845

Ser Thr Ile Leu Gly Ile Gln Ser Ile Gly Arg Gly Ile Leu Asn Ile
    850                 855                 860

Pro Arg Arg Ile Arg Gln Gly Leu Glu Arg Leu Leu Leu
865                 870                 875

<210>  7
<211>  453
<212>  DNA
<213>  Human immunodeficiency virus VAU

<400>  7
gcagagacag gacaggaaac tgcctacttc ctgttaaaat tagcagcaag atggcctatt      60

aaaatactac atacagacaa tgggcctaac tttacaagtg cagccatgaa agctgcatgt     120

tggtggacaa acatacaaca tgagtttgga ataccataca atccacaaag tcaaggagta 180
gtagaagcca tgaacaagga attaaaatca atcatacagg tgagggacca agcagagcac 240
ttaaggacag cagtacaaat ggcagtattt gttcacaatt ttaaaagaaa aggggggatt 300
ggggggtaca ctgcaggaga gagattaata gacatattag catcacaaat acaaacaaca 360
gaactacaaa aacaaatttt aaaaattcaa aattttcggg tctattacag agacagcaga 420
gaccctattt ggaaaggacc ggcacagctc ctg 453

<210> 8
<211> 351
<212> PRT
<213> Human immunodeficiency virus VAU

<400> 8

Ala Ala Gly Leu Ala Met Leu Phe Leu Gly Ile Leu Ser Ala Ala Gly
1               5                   10                  15

Ser Thr Met Gly Ala Ala Ala Thr Ala Leu Thr Val Arg Thr Gln His
            20                  25                  30

Leu Ile Lys Gly Ile Val Gln Gln Gln Asp Asn Leu Leu Arg Ala Ile
        35                  40                  45

Gln Ala Gln Gln His Leu Leu Arg Pro Ser Val Trp Gly Ile Arg Gln
    50                  55                  60

Leu Arg Ala Arg Leu Leu Ala Leu Glu Thr Phe Ile Gln Asn Gln Gln
65                  70                  75                  80

Leu Leu Asn Leu Trp Gly Cys Lys Asn Arg Leu Ile Cys Tyr Thr Ser
                85                  90                  95

Val Lys Trp Asn Lys Thr Trp Gly Gly Asp Asn Glu Ser Ile Trp Asp
            100                 105                 110

Glu Leu Thr Trp Gln Gln Trp Asp Gln Gln Ile Asn Asn Val Ser Ser
        115                 120                 125

Phe Ile Tyr Glu Lys Ile Gln Glu Ala Gln Glu Gln Gln Glu Lys Asn
    130                 135                 140

Glu Lys Glu Leu Leu Glu Leu Asp Glu Trp Ala Ser Ile Trp Asn Trp
145                 150                 155                 160

Leu Asp Ile Thr Lys Trp Leu Trp Tyr Ile Lys Ile Ala Ile Ile Ile
                165                 170                 175

Val Gly Ala Leu Ile Gly Val Arg Val Val Met Ile Val Leu Asn Leu
            180                 185                 190

Val Lys Asn Ile Arg Gln Gly Tyr Gln Pro Leu Ser Leu Gln Ile Pro
        195                 200                 205

Ile Gln Gln Gln Ala Glu Val Gly Thr Pro Gly Gly Thr Gly Glu Gly
    210                 215                 220

Gly Gly Asp Glu Asp Arg Arg Arg Trp Thr Pro Leu Pro Gln Gly Phe
225                 230                 235                 240

Leu His Leu Leu Tyr Thr Asp Leu Arg Thr Ile Ile Leu Trp Ile Tyr
                245                 250                 255

His Leu Leu Ser Asn Leu Ala Ser Glu Ile Gln Lys Leu Ile Arg His
            260                 265                 270

Leu Gly Leu Gly Leu Trp Ile Ile Gly Gln Arg Thr Ile Glu Ala Cys
        275                 280                 285

Arg Leu Phe Lys Ala Ile Ile Gln Tyr Trp Leu Gln Glu Leu Gln Thr
    290                 295                 300

Ser Ala Thr Asn Leu Leu Asp Thr Val Ala Val Ala Val Ala Asn Trp
305                 310                 315                 320

Thr Asp Ser Thr Ile Leu Gly Ile Gln Ser Ile Gly Arg Gly Ile Leu
                325                 330                 335

Asn Ile Pro Arg Arg Ile Arg Gln Gly Leu Glu Arg Leu Leu Leu
            340                 345                 350

<210>  9
<211>  513
<212>  DNA
<213>  Artificial

<220>
<223>  Region GAG de HIV 1-O souche DUR

<400>  9
cagggacaaa tggtacatca ggccatctcc cccagaactt tatatgtatg ggtaaaggca      60

gtagaagaaa aggcctttaa ccctgaaatt atccctatgt ttatggcact atcagaagga     120

gctgttccct atgatatcaa tgttatgcta aatgccatag gaggacacca aggggcttta     180

caagtattaa aagaagtaat caatgatgaa gcagcagact gggatagagc tcacccacaa     240

caggcagggc cgttaccacc agggcagata agggaaccaa caggaagtga cattgctgga     300

acaactagca cacagcaaga gcaaattctc tggactacta gggcaggtaa ccctatccca     360

gttggagaca tctataggaa atggatagtg ttgggtctaa acaaaatggt aaaaatgtat     420

agtccagtga gcatcttaga tattaggcag ggaccaaaag aaccatttag agattatgta     480

gacaggttct acaaaacatt aagagctgag cag                                  513

<210>  10
<211>  525
<212>  DNA
<213>  Artificial

<220>
<223>  Boucle V3 de gp120 souche DUR

<400>  10
attccaatac actattgtgc tccagcagga tatgctatct ttaaatgcaa caacgaggag      60

tttactggaa aaggcccatg taacaacatt tcagtagtta cctgtacaca gggtatcaag     120

ccaacagtaa gcactcatct aatattcaat gggacaatct ctgaaagaaa aataagaatt     180

atgggaaaga acatctcgag caactcaggt aatatcctag tgaccctaaa ttctactata     240

aacatgacct gtgtgaggcc aggaaataat tcagtacagg agataaaaat aggtccaatg     300

gcttggtaca gtatgcaaat tgagcgagag ggaaaaggag caaattcaag aacagctttt     360

tgtacctata atgccacgga ctggagaaaa accttgcaag ggatagctga aaggtattta     420

gaacttgtaa ataaaacaag tccgactgaa ataatgttca ataaaagcaa tggtggagat     480

gcagaaataa cccgtttgca ttttaactgt catggagaat tcttt                     525


<210>  11
<211>  312
<212>  DNA
<213>  Artificial

<220>
<223>  Region immunodominante de GP41 de HIV 1-O DUR

<400>  11
atagtgcaac agcaggacaa cctgctgaga gcaatacagg cccagcaaca tctgctgagg      60

ttatctgtat ggggtattag acaactccga gctcgcctgc tagccttaga aacccttatg     120

cagaatcagc aactcctaaa cctgtggggt tgtagaggaa aagcaatctg ctacacatca     180

gtacaatgga atgaaacatg gggaggaaat gactcaattt gggacaggtt aacatggcag     240

caatgggatc aacagatagc caatgtaagc tcttttatat atgacaaaat acaagaagca     300

caagaacaac aa                                                         312


<210>  12
<211>  861
<212>  PRT
<213>  Human immunodeficiency virus LAI

<400>  12

Met Arg Val Lys Glu Lys Tyr Gln His Leu Trp Arg Trp Gly Trp Lys
1               5                   10                  15


Trp Gly Thr Met Leu Leu Gly Ile Leu Met Ile Cys Ser Ala Thr Glu
            20                  25                  30


Lys Leu Trp Val Thr Val Tyr Tyr Gly Val Pro Val Trp Lys Glu Ala
        35                  40                  45

Thr Thr Thr Leu Phe Cys Ala Ser Asp Ala Lys Ala Tyr Asp Thr Glu
    50                  55                  60

Val His Asn Val Trp Ala Thr His Ala Cys Val Pro Thr Asp Pro Asn
65                  70                  75                  80

Pro Gln Glu Val Val Leu Val Asn Val Thr Glu Asn Phe Asn Met Trp
                85                  90                  95

Lys Asn Asp Met Val Glu Gln Met His Glu Asp Ile Ile Ser Leu Trp
            100                 105                 110

Asp Gln Ser Leu Lys Pro Cys Val Lys Leu Thr Pro Leu Cys Val Ser
        115                 120                 125

Leu Lys Cys Thr Asp Leu Gly Asn Ala Thr Asn Thr Asn Ser Ser Asn
    130                 135                 140

Thr Asn Ser Ser Ser Gly Glu Met Met Met Glu Lys Gly Glu Ile Lys
145                 150                 155                 160

Asn Cys Ser Phe Asn Ile Ser Thr Ser Ile Arg Gly Lys Val Gln Lys
                165                 170                 175

Glu Tyr Ala Phe Phe Tyr Lys Leu Asp Ile Ile Pro Ile Asp Asn Asp
            180                 185                 190

Thr Thr Ser Tyr Thr Leu Thr Ser Cys Asn Thr Ser Val Ile Thr Gln
        195                 200                 205

Ala Cys Pro Lys Val Ser Phe Glu Pro Ile Pro Ile His Tyr Cys Ala
    210                 215                 220

Pro Ala Gly Phe Ala Ile Leu Lys Cys Asn Asn Lys Thr Phe Asn Gly
225                 230                 235                 240

Thr Gly Pro Cys Thr Asn Val Ser Thr Val Gln Cys Thr His Gly Ile
                245                 250                 255

Arg Pro Val Val Ser Thr Gln Leu Leu Leu Asn Gly Ser Leu Ala Glu
            260                 265                 270

Glu Glu Val Val Ile Arg Ser Ala Asn Phe Thr Asp Asn Ala Lys Thr
        275                 280                 285

Ile Ile Val Gln Leu Asn Gln Ser Val Glu Ile Asn Cys Thr Arg Pro
    290                 295                 300

Asn Asn Asn Thr Arg Lys Ser Ile Arg Ile Gln Arg Gly Pro Gly Arg
305                 310                 315                 320

Ala Phe Val Thr Ile Gly Lys Ile Gly Asn Met Arg Gln Ala His Cys
                325                 330                 335

Asn Ile Ser Arg Ala Lys Trp Asn Ala Thr Leu Lys Gln Ile Ala Ser
            340                 345                 350

Lys Leu Arg Glu Gln Phe Gly Asn Asn Lys Thr Ile Ile Phe Lys Gln
        355                 360                 365

Ser Ser Gly Gly Asp Pro Glu Ile Val Thr His Ser Phe Asn Cys Gly
    370                 375                 380

Gly Glu Phe Phe Tyr Cys Asn Ser Thr Gln Leu Phe Asn Ser Thr Trp
385                 390                 395                 400

Phe Asn Ser Thr Trp Ser Thr Glu Gly Ser Asn Asn Thr Glu Gly Ser
                405                 410                 415

Asp Thr Ile Thr Leu Pro Cys Arg Ile Lys Gln Phe Ile Asn Met Trp
            420                 425                 430

Gln Glu Val Gly Lys Ala Met Tyr Ala Pro Pro Ile Ser Gly Gln Ile
        435                 440                 445

Arg Cys Ser Ser Asn Ile Thr Gly Leu Leu Leu Thr Arg Asp Gly Gly
    450                 455                 460

Asn Asn Asn Asn Gly Ser Glu Ile Phe Arg Pro Gly Gly Gly Asp Met
465                 470                 475                 480

Arg Asp Asn Trp Arg Ser Glu Leu Tyr Lys Tyr Lys Val Val Lys Ile
                485                 490                 495

Glu Pro Leu Gly Val Ala Pro Thr Lys Ala Lys Arg Arg Val Val Gln
            500                 505                 510

Arg Glu Lys Arg Ala Val Gly Ile Gly Ala Leu Phe Leu Gly Phe Leu
        515                 520                 525

Gly Ala Ala Gly Ser Thr Met Gly Ala Arg Ser Met Thr Leu Thr Val
    530                 535                 540

Gln Ala Arg Gln Leu Leu Ser Gly Ile Val Gln Gln Gln Asn Asn Leu
545                 550                 555                 560

Leu Arg Ala Ile Glu Ala Gln Gln His Leu Leu Gln Leu Thr Val Trp
                565                 570                 575

Gly Ile Lys Gln Leu Gln Ala Arg Ile Leu Ala Val Glu Arg Tyr Leu
            580                 585                 590

Lys Asp Gln Gln Leu Leu Gly Ile Trp Gly Cys Ser Gly Lys Leu Ile
        595                 600                 605

Cys Thr Thr Ala Val Pro Trp Asn Ala Ser Trp Ser Asn Lys Ser Leu
    610                 615                 620

Glu Gln Ile Trp Asn Asn Met Thr Trp Met Glu Trp Asp Arg Glu Ile
625                 630                 635                 640

Asn Asn Tyr Thr Ser Leu Ile His Ser Leu Ile Glu Glu Ser Gln Asn
                645                 650                 655

Gln Gln Glu Lys Asn Glu Gln Glu Leu Leu Glu Leu Asp Lys Trp Ala
            660                 665                 670

Ser Leu Trp Asn Trp Phe Asn Ile Thr Asn Trp Leu Trp Tyr Ile Lys
        675                 680                 685

Ile Phe Ile Met Ile Val Gly Gly Leu Val Gly Leu Arg Ile Val Phe
    690                 695                 700

Ala Val Leu Ser Ile Val Asn Arg Val Arg Gln Gly Tyr Ser Pro Leu
705                 710                 715                 720

Ser Phe Gln Thr His Leu Pro Thr Pro Arg Gly Pro Asp Arg Pro Glu
                725                 730                 735

Gly Ile Glu Glu Glu Gly Gly Glu Arg Asp Arg Asp Arg Ser Ile Arg
            740                 745                 750

Leu Val Asn Gly Ser Leu Ala Leu Ile Trp Asp Asp Leu Arg Ser Leu
        755                 760                 765

Cys Leu Phe Ser Tyr His Arg Leu Arg Asp Leu Leu Leu Ile Val Thr
    770                 775                 780

Arg Ile Val Glu Leu Leu Gly Arg Arg Gly Trp Glu Ala Leu Lys Tyr
785                 790                 795                 800

Trp Trp Asn Leu Leu Gln Tyr Trp Ser Gln Glu Leu Lys Asn Ser Ala
                805                 810                 815

Val Ser Leu Leu Asn Ala Thr Ala Ile Ala Val Ala Glu Gly Thr Asp
            820                 825                 830

Arg Val Ile Glu Val Val Gln Gly Ala Cys Arg Ala Ile Arg His Ile
        835                 840                 845

Pro Arg Arg Ile Arg Gln Gly Leu Glu Arg Ile Leu Leu
    850                 855                 860

<210>  13
<211>  171
<212>  PRT
<213>  Artificial

<220>
<223>  Region GAG du virus HIV 1-O souche DUR

<400>  13

Gln Gly Gln Met Val His Gln Ala Leu Ser Pro Arg Thr Leu Asn Ala
1               5                   10                  15

Trp Val Lys Ala Val Glu Glu Lys Ala Phe Asn Pro Glu Ile Ile Pro
            20                  25                  30

Met Phe Met Ala Leu Ser Glu Gly Ala Val Pro Tyr Asp Ile Asn Val
        35                  40                  45

Met Leu Asn Ala Ile Gly Gly His Gln Gly Ala Leu Gln Val Leu Lys
    50                  55                  60

Glu Val Ile Asn Asp Glu Ala Ala Asp Trp Asp Arg Ala His Pro Gln
65                  70                  75                  80

Gln Ala Gly Pro Leu Pro Pro Gly Gln Ile Arg Glu Pro Thr Gly Ser
                85                  90                  95

Asp Ile Ala Gly Thr Thr Ser Thr Gln Gln Glu Gln Ile Leu Trp Thr
            100                 105                 110

Thr Arg Ala Gly Asn Pro Ile Pro Val Gly Asp Ile Tyr Arg Lys Trp
        115                 120                 125

Ile Val Leu Gly Leu Asn Lys Met Val Lys Met Tyr Ser Pro Val Ser
    130                 135                 140

Ile Leu Asp Ile Arg Gln Gly Pro Lys Glu Pro Phe Arg Asp Tyr Val
145                 150                 155                 160

Asp Arg Phe Tyr Lys Thr Leu Arg Ala Glu Gln
                165                 170


<210>  14
<211>  175
<212>  PRT
<213>  Artificial

<220>
<223>  Boucle V3 de gp120 souche DUR

<400>  14

Ile Pro Ile His Tyr Cys Ala Pro Ala Gly Tyr Ala Ile Phe Lys Cys
1               5                   10                  15

Asn Asn Glu Glu Phe Thr Gly Lys Gly Pro Cys Asn Asn Ile Ser Val
            20                  25                  30

Val Thr Cys Thr Gln Gly Ile Lys Pro Thr Val Ser Thr His Leu Ile
        35                  40                  45

Phe Asn Gly Thr Ile Ser Glu Arg Lys Ile Arg Ile Met Gly Lys Asn
    50                  55                  60

Ile Ser Ser Asn Ser Gly Asn Ile Leu Val Thr Leu Asn Ser Thr Ile
65                  70                  75                  80

Asn Met Thr Cys Val Arg Pro Gly Asn Asn Ser Val Gln Glu Ile Lys
                85                  90                  95

Ile Gly Pro Met Ala Trp Tyr Ser Met Gln Ile Glu Arg Glu Gly Lys
            100                 105                 110

Gly Ala Asn Ser Arg Thr Ala Phe Cys Thr Tyr Asn Ala Thr Asp Trp
        115                 120                 125

Arg Lys Thr Leu Gln Gly Ile Ala Glu Arg Tyr Leu Glu Leu Val Asn
    130                 135                 140

Lys Thr Ser Pro Thr Glu Ile Met Phe Asn Lys Ser Asn Gly Gly Asp
145                 150                 155                 160

Ala Glu Ile Thr Arg Leu His Phe Asn Ser Cys Gly Glu Phe Phe
                165                 170                 175

<210>  15
<211>  104
<212>  PRT
<213>  Artificial

<220>
<223>  Region immunodominante de GP 41 de HIV 1-O DUR

<400>  15

Ile Val Gln Gln Gln Asp Asn Leu Leu Arg Ala Ile Gln Ala Gln Gln
1               5                   10                  15

His Leu Leu Arg Leu Ser Val Trp Gly Ile Arg Gln Leu Arg Ala Arg
            20                  25                  30

Leu Leu Ala Leu Glu Thr Leu Met Gln Asn Gln Gln Leu Leu Asn Leu
        35                  40                  45

Trp Gly Cys Arg Gly Lys Ala Ile Cys Tyr Thr Ser Val Gln Trp Asn
    50                  55                  60

Glu Thr Trp Gly Gly Asn Asp Ser Ile Trp Asp Arg Leu Thr Trp Gln

65                  70                  75                  80

Gln Trp Asp Gln Gln Ile Ala Asn Val Ser Ser Phe Ile Tyr Asp Lys
                85                  90                  95

Ile Gln Glu Ala Gln Glu Gln Gln
            100

<210>  16
<211>  7
<212>  PRT
<213>  Artificial

<220>
<223>  Peptide VAU

<400>  16

Cys Lys Asn Arg Leu Ile Cys
1               5

<210>  17
<211>  26
<212>  PRT
<213>  Artificial

<220>
<223>  Peptide VAU

<400>  17

Arg Leu Leu Ala Leu Glu Thr Phe Ile Gln Asn Trp Trp Leu Leu Asn
1               5                   10                  15

Leu Trp Gly Cys Lys Asn Arg Leu Ile Cys
            20                  25

<210>  18
<211>  37
<212>  PRT
<213>  Artificial

<220>
<223>  Peptide VAU

<400>  18

Arg Ala Arg Leu Leu Ala Leu Glu Thr Phe Ile Gln Asn Gln Gln Leu
1               5                   10                  15

Leu Asn Leu Trp Gly Cys Lys Asn Arg Leu Ile Cys Tyr Thr Ser Val
            20                  25                  30

Lys Trp Asn Lys Thr
        35

<210>  19
<211>  6
<212>  PRT

<213>  Artificial

<220>
<223>  Peptide 5 bis

<400>  19

Ala His Pro Gln Gln Ala
1               5


<210>  20
<211>  9
<212>  PRT
<213>  Artificial

<220>
<223>  Peptide 5 ter

<400>  20

Leu Trp Thr Thr Arg Ala Gly Asn Pro
1               5


<210>  21
<211>  32
<212>  PRT
<213>  Artificial

<220>
<223>  Peptide 1

<400>  21

Ser Pro Arg Thr Leu Asn Ala Trp Val Lys Ala Val Glu Glu Lys Ala
1               5                   10                  15


Phe Asn Pro Glu Ile Ile Pro Met Phe Met Ala Leu Ser Glu Gly Ala
            20                  25                  30


<210>  22
<211>  20
<212>  PRT
<213>  Artificial

<220>
<223>  Peptide 2

<400>  22

Met Leu Asn Ala Ile Gly Gly His Gln Gly Ala Leu Gln Val Leu Lys
1               5                   10                  15


Glu Val Ile Asn
            20


<210>  23
<211>  27
<212>  PRT
<213>  Artificial

<220>
<223>  Peptide 3

<400>  23

Gly Pro Leu Pro Pro Gly Gln Ile Arg Glu Pro Thr Gly Ser Asp Ile
1               5                   10                  15

Ala Gly Thr Thr Ser Thr Gln Gln Glu Gln Ile
            20                  25

<210>  24
<211>  30
<212>  PRT
<213>  Artificial

<220>
<223>  Peptide 4

<400>  24

Ile Pro Val Gly Asp Ile Tyr Arg Lys Trp Ile Val Leu Gly Leu Asn
1               5                   10                  15

Lys Met Val Lys Met Tyr Ser Pro Val Ser Ile Leu Asp Ile
            20                  25                  30

<210>  25
<211>  21
<212>  PRT
<213>  Artificial

<220>
<223>  Peptide 5

<400>  25

Gln Gly Pro Lys Glu Pro Phe Arg Asp Tyr Val Asp Arg Phe Tyr Lys
1               5                   10                  15

Thr Lys Leu Ala Glu
            20

<210>  26
<211>  10
<212>  PRT
<213>  Artificial

<220>
<223>  Peptide 6

<400>  26

Ser Pro Arg Thr Leu Asn Ala Trp Val Lys
1               5                   10

<210>  27
<211>  10
<212>  PRT
<213>  Artificial

<220>
<223>  Peptide 7

<400>  27

Gly Ser Asp Ile Ala Gly Thr Thr Ser Thr
1               5                   10


<210>  28
<211>  14
<212>  PRT
<213>  Artificial

<220>
<223>  Peptide 8

<400>  28

Gln Gly Pro Lys Glu Pro Phe Arg Asp Tyr Val Asp Arg Phe
1               5                   10


<210>  29
<211>  4
<212>  PRT
<213>  Artificial

<220>
<223>  Peptide 9

<400>  29

Asn Pro Glu Ile
1


<210>  30
<211>  16
<212>  PRT
<213>  Artificial

<220>
<223>  Peptide 10

<400>  30

Ala Val Glu Glu Lys Ala Phe Asn Pro Glu Ile Ile Pro Met Phe Met
1               5                   10                  15


<210>  31
<211>  9
<212>  PRT
<213>  Artificial

<220>
<223>  Peptide 23

<400>  31

Ile Gly Gly His Gln Gly Ala Leu Gln
1               5


<210>  32
<211>  8
<212>  PRT
<213>  Artificial

<220>
<223>  Peptide 24

<400>  32

Arg Glu Pro Thr Gly Ser Asp Ile
1               5


<210>  33
<211>  9
<212>  PRT
<213>  Artificial

<220>
<223>  Peptide 25

<400>  33

Ile Asn Asp Glu Ala Ala Asp Trp Asp
1               5


<210>  34
<211>  5
<212>  PRT
<213>  Artificial

<220>
<223>  Peptide 12

<400>  34

Lys Glu Ile Lys Ile
1               5


<210>  35
<211>  8
<212>  PRT
<213>  Artificial

<220>
<223>  Peptide 13

<400>  35

Glu Arg Glu Gly Lys Gly Ala Asn
1               5


<210>  36
<211>  14
<212>  PRT
<213>  Artificial

<220>
<223>  Peptide 14

<400>  36

Cys Val Arg Pro Gly Asn Asn Ser Val Lys Glu Ile Lys Ile
1               5                   10


<210>  37
<211>  12
<212>  PRT

<213>  Artificial

<220>
<223>  Peptide 15

<400>  37

Gln Ile Glu Arg Glu Gly Lys Gly Ala Asn Ser Arg
1               5                   10


<210>  38
<211>  38
<212>  PRT
<213>  Artificial

<220>
<223>  Peptide 11

<400>  38

Cys Val Arg Pro Gly Asn Asn Ser Val Lys Glu Ile Lys Ile Gly Pro
1               5                   10                  15


Met Ala Trp Tyr Ser Met Gln Ile Glu Arg Glu Gly Lys Gly Ala Asn
            20                  25                  30


Ser Arg Thr Ala Phe Cys
        35


<210>  39
<211>  8
<212>  PRT
<213>  Artificial

<220>
<223>  Peptide 16

<400>  39

Gly Pro Met Ala Trp Tyr Ser Met
1               5


<210>  40
<211>  14
<212>  PRT
<213>  Artificial

<220>
<223>  Peptide 17

<400>  40

Arg Leu Leu Ala Leu Glu Thr Leu Met Gln Asn Gln Gln Leu
1               5                   10


<210>  41
<211>  23
<212>  PRT
<213>  Artificial

<220>
<223>  Peptide 18

<400>  41

Leu Asn Leu Trp Gly Cys Arg Gly Lys Ala Ile Cys Tyr Thr Ser Val
1               5                   10                  15

Gln Trp Asn Glu Thr Trp Gly
            20

<210>  42
<211>  6
<212>  PRT
<213>  Artificial

<220>
<223>  Peptide 19

<400>  42

Cys Arg Gly Lys Ala Ile
1               5

<210>  43
<211>  5
<212>  PRT
<213>  Artificial

<220>
<223>  Peptide 20

<400>  43

Ser Val Gln Trp Asn
1               5

<210>  44
<211>  28
<212>  PRT
<213>  Artificial

<220>
<223>  Peptide 21

<400>  44

Arg Leu Leu Ala Leu Glu Thr Leu Met Asn Gln Gln Leu Leu Asn Leu
1               5                   10                  15

Trp Gly Cys Arg Gly Lys Ala Ile Cys Tyr Thr Ser
            20                  25

<210>  45
<211>  24
<212>  PRT
<213>  Artificial

<220>
<223>  Peptide 22

<400>  45

Gln Asn Gln Gln Leu Leu Asn Leu Trp Gly Cys Arg Gly Lys Ala Ile

1                 5                   10                  15

Cys Tyr Thr Ser Val Gln Trp Asn
            20

<210>  46
<211>  8
<212>  PRT
<213>  Artificial

<220>
<223>  Peptide DUR

<400>  46

Arg Ile Leu Ala Val Glu Arg Tyr
1               5

<210>  47
<211>  6
<212>  PRT
<213>  Artificial

<220>
<223>  Peptide VIH-1 LAI

<400>  47

Gly Pro Gly Arg Ala Phe
1               5

<210>  48
<211>  6
<212>  PRT
<213>  Artificial

<220>
<223>  Peptide VIH-1 VAU

<400>  48

Gly Pro Met Ala Trp Tyr
1               5

<210>  49
<211>  6
<212>  PRT
<213>  Artificial

<220>
<223>  Peptide HIV MVP5180

<400>  49

Gly Pro Met Arg Trp Arg
1               5

<210>  50
<211>  5
<212>  PRT
<213>  Artificial

<220>
<223>  Peptide VIH-1 VAU

<400>  50

Thr Phe Ile Gln Asn
1               5


<210>  51
<211>  6
<212>  PRT
<213>  Artificial

<220>
<223>  Peptide VIH-1 VAU

<400>  51

Trp Gly Cys Lys Asn Arg
1               5


<210>  52
<211>  24
<212>  DNA
<213>  Artificial

<220>
<223>  Amorce oligonucléotidique dur V3a

<400>  52
attccaatac actattgtgc tcca                                              24


<210>  53
<211>  24
<212>  DNA
<213>  Artificial

<220>
<223>  Amorce oligonucléotidique dur V3r

<400>  53
aaagaattct ccatgactgt taaa                                              24


<210>  54
<211>  24
<212>  DNA
<213>  Artificial

<220>
<223>  Amorce nucléotidique dur 41a

<400>  54
ggtatagtgc aacagcagga caac                                              24


<210>  55
<211>  21
<212>  DNA
<213>  Artificial

<220>
<223>  Amorce nucléotidique dur 41r

<400>  55

agaggcccat tcatctaact c                                                21

<210>  56
<211>  7
<212>  PRT
<213>  Artificial

<220>
<223>  Région homologue de la région gp41


<220>
<221>  misc_feature
<222>  (3)..(3)
<223>  Xaa can be any naturally occurring amino acid

<400>  56

Arg Leu Xaa Ala Leu Glu Thr
1               5


<210>  57
<211>  4
<212>  PRT
<213>  Artificial

<220>
<223>  Région homologue de la région gp41

<400>  57

Gln Asn Gln Gln
1


<210>  58
<211>  4
<212>  PRT
<213>  Artificial

<220>
<223>  Région homologue de la région gp41

<400>  58

Leu Trp Gly Cys
1


<210>  59
<211>  5
<212>  PRT
<213>  Artificial

<220>
<223>  Région homologue de la région gp41

<400>  59

Cys Tyr Thr Ser Val
1               5


<210>  60
<211>  26
<212>  PRT

<213>  Artificial

<220>
<223>  Epitope immunodominant de HIV-1 lai

<400>  60

Arg Ile Leu Ala Val Glu Arg Tyr Leu Lys Asp Gln Gln Leu Leu Gly
1               5                   10                  15

Ile Trp Gly Cys Ser Gly Lys Leu Ile Cys
            20                  25

<210>  61
<211>  26
<212>  PRT
<213>  Artificial

<220>
<223>  Epitope immunodominant de HIV-1 Z321

<400>  61

Arg Ile Leu Ala Val Glu Arg Tyr Leu Lys Asp Gln Gln Leu Leu Gly
1               5                   10                  15

Ile Trp Gly Cys Ser Gly Lys Ile Ile Cys
            20                  25

<210>  62
<211>  26
<212>  PRT
<213>  Artificial

<220>
<223>  Epitope immunodominant de HIV-1 eli

<400>  62

Arg Ile Leu Ala Val Glu Arg Tyr Leu Lys Asp Gln Gln Leu Leu Gly
1               5                   10                  15

Ile Trp Gly Cys Ser Gly Lys His Ile Cys
            20                  25

<210>  63
<211>  26
<212>  PRT
<213>  Artificial

<220>
<223>  Epitope immunodominant de HIV-1 JRCSF

<400>  63

Arg Val Leu Ala Val Glu Arg Tyr Leu Lys Asp Gln Gln Leu Met Gly
1               5                   10                  15

Ile Trp Gly Cys Ser Gly Lys Leu Ile Cys
            20                  25

<210>  64
<211>  26
<212>  PRT
<213>  Artificial

<220>
<223>  Epitope immunodominant de HIV-1 WMJ

<400>  64

Arg Val Leu Ala Val Glu Arg Tyr Leu Arg Asp Gln Gln Leu Leu Gly
1               5                   10                  15

Ile Trp Gly Cys Ser Gly Lys Leu Ile Cys
            20                  25

<210>  65
<211>  26
<212>  PRT
<213>  Artificial

<220>
<223>  Epitope immunodominant de HIV-1 NDK

<400>  65

Arg Val Leu Ala Val Glu Arg Tyr Leu Arg Asp Gln Gln Leu Leu Gly
1               5                   10                  15

Ile Trp Gly Cys Ser Gly Arg His Ile Cys
            20                  25

<210>  66
<211>  26
<212>  PRT
<213>  Artificial

<220>
<223>  Epitope immunodominant de HIV-1 mal

<400>  66

Arg Val Leu Ala Val Glu Arg Tyr Leu Gln Asp Gln Arg Leu Leu Gly
1               5                   10                  15

Met Trp Gly Cys Ser Gly Lys His Ile Cys
            20                  25

<210>  67
<211>  26
<212>  PRT
<213>  Artificial

<220>
<223>  Epitope immunodominant de SIV CPZ GAB

<400>  67

Arg Leu Leu Ala Val Glu Arg Tyr Leu Gln Asp Gln Gln Ile Leu Gly
1               5                   10                  15

Leu Trp Gly Cys Ser Gly Lys Ala Val Cys
            20                  25

<210>  68
<211>  26
<212>  PRT
<213>  Artificial

<220>
<223>  Epitope immunodominant de HIV-1 vau

<400>  68

Arg Leu Leu Ala Leu Glu Thr Phe Ile Gln Asn Gln Gln Leu Leu Asn
1               5                   10                  15

Leu Trp Gly Cys Lys Asn Arg Leu Ile Cys
            20                  25

<210>  69
<211>  26
<212>  PRT
<213>  Artificial

<220>
<223>  Epitope immunodominant de mvp5180

<400>  69

Arg Leu Gln Ala Leu Glu Thr Leu Ile Gln Asn Gln Gln Arg Leu Asn
1               5                   10                  15

Leu Trp Gly Cys Lys Gly Lys Leu Ile Cys
            20                  25

<210>  70
<211>  26
<212>  PRT
<213>  Artificial

<220>
<223>  Epitope immunodominant de ant70

<400>  70

Arg Leu Leu Ala Leu Glu Thr Leu Leu Gln Asn Gln Gln Leu Leu Ser
1               5                   10                  15

Leu Trp Gly Cys Lys Gly Lys Leu Val Cys
            20                  25

<210>  71
<211>  26
<212>  PRT
<213>  Artificial

<220>
<223>  Epitope immunodominant de HIV-2 rod

<400>  71

Arg Val Thr Ala Ile Glu Lys Tyr Leu Gln Asp Gln Ala Arg Leu Asn
1               5                   10                  15

Ser Trp Gly Cys Ala Phe Arg Gln Val Cys
            20                  25

<210>  72
<211>  26
<212>  PRT
<213>  Artificial

<220>
<223>  Epitope immunodominant de HIV-2 D194

<400>  72

Arg Val Thr Ala Ile Glu Lys Tyr Leu Lys Asp Gln Ala Gln Leu Asn
1               5                   10                  15

Ser Trp Gly Cys Ala Phe Arg Gln Val Cys
            20                  25

<210>  73
<211>  170
<212>  PRT
<213>  Artificial

<220>
<223>  Région GAG DUR

<220>
<221>  DOMAIN
<222>  (10)..(41)
<223>  Région discriminative 1

<220>
<221>  DOMAIN
<222>  (49)..(68)
<223>  Région discriminative 2

<220>
<221>  DOMAIN
<222>  (83)..(109)
<223>  Région discriminative 3

<220>
<221>  DOMAIN
<222>  (121)..(143)
<223>  Région discriminative 4

<400>  73

Gln Gly Gln Met Val His Gln Ala Leu Ser Pro Arg Thr Leu Asn Ala
1               5                   10                  15

Trp Val Lys Ala Val Glu Glu Lys Ala Phe Asn Pro Glu Ile Ile Pro
            20                  25                  30

Met Phe Met Ala Leu Ser Glu Gly Ala Val Pro Tyr Asp Ile Asn Val
        35                  40                  45

Met Leu Asn Ala Ile Gly Gly His Gln Gly Ala Leu Gln Val Leu Lys
    50                  55                  60

Glu Val Ile Asn Asp Glu Ala Ala Asp Trp Asp Arg Ala His Pro Gln
65                  70                  75                  80

Gln Ala Gly Pro Leu Pro Pro Gly Gln Ile Arg Glu Pro Thr Gly Ser
                85                  90                  95

Asp Ile Ala Gly Thr Thr Ser Thr Gln Gln Glu Gln Ile Leu Trp Thr
            100                 105                 110

Thr Arg Ala Gly Asn Pro Ile Pro Val Gly Asp Ile Tyr Arg Lys Trp
        115                 120                 125

Ile Val Leu Gly Leu Asn Lys Met Val Lys Met Tyr Ser Pro Val Ser
    130                 135                 140

Ile Leu Asp Ile Arg Gln Gly Pro Lys Glu Pro Phe Arg Asp Tyr Val
145                 150                 155                 160

Asp Arg Phe Tyr Lys Thr Lys Leu Ala Glu
                165                 170

<210>  74
<211>  170
<212>  PRT
<213>  Artificial

<220>
<223>  Région GAG ANT


<220>
<221>  DOMAIN
<222>  (10)..(41)
<223>  Région discriminative 1

<220>
<221>  DOMAIN
<222>  (49)..(68)
<223>  Région discriminative 2

<220>
<221>  DOMAIN
<222>  (83)..(109)
<223>  Région discriminative 3

<220>
<221>  DOMAIN
<222>  (121)..(143)
<223>  Région discriminative 4

<400>  74

Gln Gly Gln Met Val His Gln Ala Ile Ser Pro Arg Thr Leu Asn Ala
1               5                   10                  15

Trp Val Lys Ala Val Glu Glu Lys Ala Phe Asn Pro Glu Ile Ile Pro
            20                  25                  30

Met Phe Met Ala Leu Ser Glu Gly Ala Ile Ser Tyr Asp Ile Asn Thr
        35                  40                  45

Met Leu Asn Ala Ile Gly Gly His Gln Gly Ala Leu Gln Val Leu Lys
    50                  55                  60

Glu Val Ile Asn Glu Glu Ala Val Glu Trp Asp Arg Thr His Pro Pro
65                  70                  75                  80

Pro Val Gly Pro Leu Pro Pro Gly Gln Ile Arg Glu Pro Thr Gly Ser
                85                  90                  95

Asp Ile Ala Gly Thr Thr Ser Thr Gln Gln Glu Gln Ile His Trp Thr
            100                 105                 110

Thr Arg Pro Asn Gln Pro Ile Pro Val Gly Asp Ile Tyr Arg Lys Trp
        115                 120                 125

Ile Val Leu Gly Leu Asn Lys Met Val Lys Met Tyr Ser Pro Val Ser
    130                 135                 140

Ile Leu Asp Ile Lys Gln Gly Pro Lys Glu Pro Phe Arg Asp Tyr Val
145                 150                 155                 160

Asp Arg Phe Tyr Lys Thr Lys Leu Ala Glu
                165                 170

<210>  75
<211>  170
<212>  PRT
<213>  Artificial

<220>
<223>  Région GAG MVP

<220>
<221>  DOMAIN
<222>  (10)..(41)
<223>  Région discriminative 1

<220>
<221>  DOMAIN
<222>  (49)..(68)
<223>  Région discriminative 2

<220>
<221>  DOMAIN
<222>  (83)..(109)
<223>  Région discriminative 3

<220>
<221>  DOMAIN
<222>  (121)..(143)

<223>  Région discriminative 4

<400>  75

Gln Gly Gln Met Val His Gln Ala Ile Ser Pro Arg Thr Leu Asn Ala
1               5                   10                  15

Trp Val Lys Ala Val Glu Glu Lys Ala Phe Asn Pro Glu Ile Ile Pro
            20                  25                  30

Met Phe Met Ala Leu Ser Glu Gly Ala Val Pro Tyr Asp Ile Asn Thr
        35                  40                  45

Met Leu Asn Ala Ile Gly Gly His Gln Gly Ala Leu Gln Val Leu Lys
    50                  55                  60

Glu Val Ile Asn Glu Glu Ala Ala Glu Trp Asp Arg Thr His Pro Pro
65                  70                  75                  80

Ala Met Gly Pro Leu Pro Pro Gly Gln Ile Arg Glu Pro Thr Gly Ser
                85                  90                  95

Asp Ile Ala Gly Thr Thr Ser Thr Gln Gln Glu Gln Ile Ile Trp Thr
            100                 105                 110

Thr Arg Gly Ala Asn Ser Ile Pro Val Gly Asp Ile Tyr Arg Lys Trp
        115                 120                 125

Ile Val Leu Gly Leu Asn Lys Met Val Lys Met Tyr Ser Pro Val Ser
    130                 135                 140

Ile Leu Asp Ile Arg Gln Gly Pro Lys Glu Pro Phe Arg Asp Tyr Val
145                 150                 155                 160

Asp Arg Phe Tyr Lys Thr Lys Leu Ala Glu
                165                 170

<210>  76
<211>  169
<212>  PRT
<213>  Artificial

<220>
<223>  Région GAG LAI

<220>
<221>  DOMAIN
<222>  (10)..(41)
<223>  Région discriminative 1

<220>
<221>  DOMAIN
<222>  (49)..(68)
<223>  Région discriminative 2

<220>

<221>  DOMAIN
<222>  (83)..(109)
<223>  Région discriminative 3

<220>
<221>  DOMAIN
<222>  (121)..(143)
<223>  Région discriminative 4

<400>  76

Gln Gly Gln Met Val His Gln Ala Ile Ser Pro Arg Thr Leu Asn Ala
1               5                   10                  15

Trp Val Lys Val Val Glu Glu Lys Ala Phe Ser Pro Glu Val Ile Pro
            20                  25                  30

Met Phe Ser Ala Leu Ser Glu Gly Ala Thr Pro Gln Asp Leu Asn Thr
        35                  40                  45

Met Leu Asn Thr Val Gly Gly His Gln Ala Ala Met Gln Met Leu Lys
    50                  55                  60

Glu Thr Ile Asn Glu Glu Ala Ala Glu Trp Asp Arg Val His Pro Val
65                  70                  75                  80

His Ala Gly Pro Ile Ala Pro Gly Gln Met Arg Glu Pro Arg Gly Ser
                85                  90                  95

Asp Ile Ala Gly Thr Thr Ser Thr Leu Gln Glu Gln Ile Gly Trp Met
            100                 105                 110

Thr Asn Asn Pro Pro Ile Pro Val Gly Glu Ile Tyr Lys Arg Trp Ile
        115                 120                 125

Ile Leu Gly Leu Asn Lys Ile Val Arg Met Tyr Ser Pro Thr Ser Ile
    130                 135                 140

Leu Asp Ile Arg Gln Gly Pro Lys Glu Pro Phe Arg Asp Tyr Val Asp
145                 150                 155                 160

Arg Phe Tyr Lys Thr Lys Leu Ala Glu
                165

<210>  77
<211>  169
<212>  PRT
<213>  Artificial

<220>
<223>  Région GAG MAL

<220>
<221>  DOMAIN
<222>  (10)..(41)
<223>  Région discriminative 1

<220>
<221>  DOMAIN
<222>  (49)..(68)
<223>  Région discriminative 2

<220>
<221>  DOMAIN
<222>  (83)..(109)
<223>  Région discriminative 3

<220>
<221>  DOMAIN
<222>  (121)..(143)
<223>  Région discriminative 4

<400>  77

Gln Gly Gln Met Ile His Gln Ala Ile Ser Pro Arg Thr Leu Asn Ala
1               5                   10                  15

Trp Val Lys Val Ile Glu Glu Lys Ala Phe Ser Pro Glu Val Ile Pro
            20                  25                  30

Met Phe Ser Ala Leu Ser Glu Gly Ala Thr Pro Gln Asp Leu Asn Met
        35                  40                  45

Met Leu Asn Ile Val Gly Gly His Gln Ala Ala Met Gln Met Leu Lys
    50                  55                  60

Asp Thr Ile Asn Glu Glu Ala Ala Asp Trp Asp Arg Val His Pro Val
65                  70                  75                  80

His Ala Gly Pro Ile Pro Pro Gly Gln Met Arg Glu Pro Arg Gly Ser
                85                  90                  95

Asp Ile Ala Gly Thr Thr Ser Thr Leu Gln Glu Gln Ile Gly Trp Met
            100                 105                 110

Thr Ser Asn Pro Pro Ile Pro Val Gly Asp Ile Tyr Lys Arg Trp Ile
        115                 120                 125

Ile Leu Gly Leu Asn Lys Ile Val Arg Met Tyr Ser Pro Val Ser Ile
    130                 135                 140

Leu Asp Ile Arg Gln Gly Pro Lys Glu Pro Phe Arg Asp Tyr Val Asp
145                 150                 155                 160

Arg Phe Phe Lys Thr Lys Leu Ala Glu
                165

<210>  78
<211>  169
<212>  PRT
<213>  Artificial

<220>

<223>  Région GAG CPZ

<220>
<221>  DOMAIN
<222>  (10)..(41)
<223>  Région discriminative 1

<220>
<221>  DOMAIN
<222>  (49)..(68)
<223>  Région discriminative 2

<220>
<221>  DOMAIN
<222>  (83)..(109)
<223>  Région discriminative 3

<220>
<221>  DOMAIN
<222>  (121)..(143)
<223>  Région discriminative 4

<400>  78

Gln Gly Gln Met Val His Gln Ala Ile Ser Pro Arg Thr Leu Asn Ala
1               5                   10                  15

Trp Val Lys Val Val Glu Glu Lys Ala Phe Ser Pro Glu Val Ile Pro
            20                  25                  30

Met Phe Ser Ala Leu Ser Glu Gly Ala Leu Pro Gln Asp Val Asn Thr
        35                  40                  45

Met Leu Asn Ala Val Gly Gly His Gln Gly Ala Met Gln Val Leu Lys
    50                  55                  60

Glu Val Ile Asn Glu Glu Ala Ala Glu Trp Asp Arg Leu His Pro Thr
65                  70                  75                  80

His Ala Gly Pro Ile Ala Pro Gly Gln Leu Arg Glu Pro Arg Gly Ser
                85                  90                  95

Asp Ile Ala Gly Thr Thr Ser Thr Leu Gln Glu Gln Ile Gly Trp Thr
            100                 105                 110

Thr Ala Asn Pro Pro Ile Pro Val Gly Asp Val Tyr Arg Arg Trp Val
        115                 120                 125

Ile Leu Gly Leu Asn Lys Val Val Arg Met Tyr Cys Pro Val Ser Ile
    130                 135                 140

Leu Asp Ile Arg Gln Gly Pro Lys Glu Pro Phe Arg Asp Tyr Val Asp
145                 150                 155                 160

Arg Phe Tyr Lys Thr Lys Leu Ala Glu
                165

<210>  79
<211>  35
<212>  PRT
<213>  Artificial

<220>
<223>  Région de la boucle V3 de GP120 de MAD

<400>  79

Cys Thr Arg Pro Tyr Lys Asn Thr Arg Gln Arg Thr Gly Ile Gly Pro
1               5                   10                  15

Gly Gln Ala Leu Tyr Thr Thr His Arg Ile Ile Gly Asp Ile Arg Gln
            20                  25                  30

Ala His Cys
        35

<210>  80
<211>  37
<212>  PRT
<213>  Artificial

<220>
<223>  Région immunodominante de GP41 de MAD

<400>  80

Arg Val Leu Ala Val Glu Arg Tyr Leu Gln Asp Gln Gln Leu Leu Gly
1               5                   10                  15

Ile Trp Gly Cys Ser Gly Lys His Ile Cys Thr Thr Thr Val Pro Trp
            20                  25                  30

Asn Ser Ser Trp Ser
        35

<210>  81
<211>  36
<212>  PRT
<213>  Artificial

<220>
<223>  Région de la boucle V3 de GP120 de LAI

<400>  81

Cys Thr Arg Pro Asn Asn Asn Thr Arg Lys Ser Ile Arg Ile Gln Arg
1               5                   10                  15

Gly Pro Gly Arg Ala Phe Val Thr Ile Gly Lys Ile Gly Asn Met Arg
            20                  25                  30

Gln Ala His Cys
        35

<210>  82

<211>  37
<212>  PRT
<213>  Artificial

<220>
<223>  Région immunodominante de GP41 de LAI

<400>  82

Arg Ile Leu Ala Val Glu Arg Tyr Leu Lys Asp Gln Gln Leu Leu Gly
1               5                   10                  15

Ile Trp Gly Cys Ser Gly Lys Leu Ile Cys Thr Thr Ala Val Pro Trp
            20                  25                  30

Asn Ala Ser Trp Ser
        35

<210>  83
<211>  37
<212>  PRT
<213>  Artificial

<220>
<223>  Région de la boucle V3 de GP120 de OYI

<400>  83

Cys Thr Arg Pro Asn Asn Asn Thr Arg Asn Arg Ile Ser Ile Gln Arg
1               5                   10                  15

Gly Pro Gly Arg Ala His Val Thr Thr Lys Gln Ile Ile Gly Asp Ile
            20                  25                  30

Arg Gln Ala His Cys
        35

<210>  84
<211>  37
<212>  PRT
<213>  Artificial

<220>
<223>  Région immunodominante de GP41 de OYI

<400>  84

Arg Val Leu Ala Val Glu Arg Tyr Leu Lys Asp Gln Gln Leu Leu Gly
1               5                   10                  15

Ile Trp Gly Cys Ser Gly Lys Leu Ile Cys Thr Thr Thr Val Pro Trp
            20                  25                  30

Asn Ala Ser Trp Ser
        35

<210>  85
<211>  34
<212>  PRT

<213>  Artificial

<220>
<223>  Région de la boucle V3 de GP120 de ELI

<400>  85

Cys Ala Arg Pro Tyr Gln Asn Thr Arg Gln Arg Thr Pro Ile Gly Leu
1               5                   10                  15

Gly Gln Ser Leu Tyr Thr Thr Arg Ser Arg Ser Ile Ile Gly Gln Ala
            20                  25                  30

His Cys

<210>  86
<211>  37
<212>  PRT
<213>  Artificial

<220>
<223>  Région immunodominante de GP41 de ELI

<400>  86

Arg Ile Leu Ala Val Glu Arg Tyr Leu Lys Asp Gln Gln Leu Leu Gly
1               5                   10                  15

Ile Trp Gly Cys Ser Gly Lys His Ile Cys Thr Thr Asn Val Pro Trp
            20                  25                  30

Asn Ser Ser Trp Ser
        35

<210>  87
<211>  33
<212>  PRT
<213>  Artificial

<220>
<223>  Région de la boucle V3 de GP120 de MAL

<400>  87

Cys Thr Arg Pro Gly Asn Asn Thr Arg Arg Gly Ile His Phe Gly Pro
1               5                   10                  15

Gly Gln Ala Leu Tyr Thr Thr Gly Val Gly Asp Ile Arg Arg Ala Tyr
            20                  25                  30

Cys

<210>  88
<211>  37
<212>  PRT
<213>  Artificial

<220>
<223>  Région immunodominante de GP41 de MAL

<400>  88

Arg Val Leu Ala Val Glu Arg Tyr Leu Gln Asp Gln Arg Leu Leu Gly
1               5                   10                  15

Met Trp Gly Cys Ser Gly Lys His Ile Cys Thr Thr Phe Val Pro Trp
            20                  25                  30

Asn Ser Ser Trp Ser
        35

<210>  89
<211>  36
<212>  PRT
<213>  Artificial

<220>
<223>  Région de la boucle V3 de GP120 de 455

<400>  89

Cys Ser Arg Pro Tyr Asn Thr Arg Lys Asn Ile Arg Arg Tyr Ser Ile
1               5                   10                  15

Gly Ser Gly Gln Ala Phe Tyr Val Thr Gly Lys Ile Gly Asp Ile Arg
            20                  25                  30

Gln Ala His Cys
        35

<210>  90
<211>  37
<212>  PRT
<213>  Artificial

<220>
<223>  Région immunodominante de GP41 de 455

<400>  90

Arg Val Leu Ala Val Glu Arg Tyr Leu Gln Asp Gln Gln Leu Leu Gly
1               5                   10                  15

Ile Trp Gly Cys Ser Gly Lys Leu Ile Cys Thr Thr Thr Val Pro Trp
            20                  25                  30

Asn Ser Ser Trp Ser
        35

<210>  91
<211>  35
<212>  PRT
<213>  Artificial

<220>
<223>  Région CPZ de la boucle V3 de GP120

<400>  91

Cys His Arg Pro Gly Asn Asn Thr Arg Gly Glu Val Gln Ile Gly Pro
1               5                   10                  15

Gly Met Thr Phe Tyr Asn Ile Glu Asn Val Val Gly Asp Thr Arg Ser
            20                  25                  30

Ala Tyr Cys
        35

<210>  92
<211>  37
<212>  PRT
<213>  Artificial

<220>
<223>  Région immunodominante de GP41 de CPZ

<400>  92

Arg Leu Leu Ala Val Glu Arg Tyr Leu Gln Asp Gln Gln Ile Leu Gly
1               5                   10                  15

Leu Trp Gly Cys Ser Gly Lys Ala Val Cys Tyr Thr Thr Val Pro Trp
            20                  25                  30

Asn Asn Ser Trp Pro
        35

<210>  93
<211>  36
<212>  PRT
<213>  Artificial

<220>
<223>  Région de la boucle V3 de GP120 de ANT

<400>  93

Cys Glu Arg Pro Gln Ile Asp Ile Gln Glu Met Arg Ile Gly Pro Met
1               5                   10                  15

Ala Trp Tyr Ser Met Gly Ile Gly Gly Thr Ala Gly Asn Ser Ser Arg
            20                  25                  30

Gln Ala Tyr Cys
        35

<210>  94
<211>  37
<212>  PRT
<213>  Artificial

<220>
<223>  Région immunodominante de GP41 de ANT

<400>  94

Arg Leu Leu Ala Leu Glu Thr Leu Leu Gln Asn Gln Gln Leu Leu Ser
1               5                   10                  15

Leu Trp Gly Cys Lys Gly Lys Leu Val Cys Tyr Thr Ser Val Lys Trp
            20                  25                  30

Asn Arg Thr Trp Ile
        35

<210>  95
<211>  36
<212>  PRT
<213>  Artificial

<220>
<223>  Région de la boucle V3 de GP120 de MVP

<400>  95

Cys Ile Arg Glu Gly Ile Ala Glu Val Gln Asp Ile Tyr Thr Gly Pro
1               5                   10                  15

Met Arg Trp Arg Ser Met Thr Leu Ile Arg Ser Asn Asn Thr Ser Arg
            20                  25                  30

Val Ala Tyr Cys
        35

<210>  96
<211>  37
<212>  PRT
<213>  Artificial

<220>
<223>  Région immunodominante de GP41 de MVP

<400>  96

Arg Leu Gln Ala Leu Glu Thr Leu Ile Gln Asn Gln Gln Arg Leu Asn
1               5                   10                  15

Leu Trp Gly Cys Lys Gly Lys Leu Ile Cys Tyr Thr Ser Val Lys Trp
            20                  25                  30

Asn Arg Thr Trp Ile
        35

<210>  97
<211>  37
<212>  PRT
<213>  Artificial

<220>
<223>  Région de la boucle V3 de GP120 de  VAU

<400>  97

Cys Glu Arg Pro Gly Asn Gln Thr Ile Gln Lys Ile Met Ala Gly Pro

1               5                   10                  15

Met Ala Trp Tyr Ser Met Ala Leu Ser Asn Thr Lys Gly Asp Thr Ser
            20                  25                  30

Arg Ala Ala Tyr Cys
        35

<210>  98
<211>  37
<212>  PRT
<213>  Artificial

<220>
<223>  Région immunodominante de GP41 de VAU

<400>  98

Arg Leu Leu Ala Leu Glu Thr Phe Ile Gln Asn Gln Gln Leu Leu Asn
1               5                   10                  15

Leu Trp Gly Cys Lys Asn Arg Leu Ile Cys Tyr Thr Ser Val Lys Trp
            20                  25                  30

Asn Lys Thr Trp Gly
        35

<210>  99
<211>  40
<212>  PRT
<213>  Artificial

<220>
<223>  Consensus de la région de la boucle V3 de GP120 de HIV-O

<220>
<221>  misc_feature
<222>  (2)..(2)
<223>  Xaa can be any naturally occurring amino acid

<220>
<221>  misc_feature
<222>  (4)..(11)
<223>  Xaa can be any naturally occurring amino acid

<220>
<221>  misc_feature
<222>  (13)..(16)
<223>  Xaa can be any naturally occurring amino acid

<220>
<221>  misc_feature
<222>  (20)..(20)
<223>  Xaa can be any naturally occurring amino acid

<220>
<221>  misc_feature
<222>  (22)..(22)
<223>  Xaa can be any naturally occurring amino acid

<220>

<221>  misc_feature
<222>  (25)..(34)
<223>  Xaa can be any naturally occurring amino acid

<220>
<221>  misc_feature
<222>  (37)..(37)
<223>  Xaa can be any naturally occurring amino acid

<220>
<221>  misc_feature
<222>  (39)..(39)
<223>  Xaa can be any naturally occurring amino acid

<400>  99

Cys Xaa Arg Xaa Xaa Xaa Xaa Xaa Xaa Xaa Xaa Ile Xaa Xaa Xaa Xaa
1               5                   10                  15

Gly Pro Met Xaa Trp Xaa Ser Met Xaa Xaa Xaa Xaa Xaa Xaa Xaa Xaa
            20                  25                  30

Xaa Xaa Ser Arg Xaa Ala Xaa Cys
        35                  40

<210>  100
<211>  37
<212>  PRT
<213>  Artificial

<220>
<223>  Consensus de la région de GP41 de HIV-O

<220>
<221>  misc_feature
<222>  (3)..(3)
<223>  Xaa can be any naturally occurring amino acid

<220>
<221>  misc_feature
<222>  (8)..(9)
<223>  Xaa can be any naturally occurring amino acid

<220>
<221>  misc_feature
<222>  (14)..(14)
<223>  Xaa can be any naturally occurring amino acid

<220>
<221>  misc_feature
<222>  (16)..(16)
<223>  Xaa can be any naturally occurring amino acid

<220>
<221>  misc_feature
<222>  (21)..(25)
<223>  Xaa can be any naturally occurring amino acid

<220>
<221>  misc_feature
<222>  (31)..(31)
<223>  Xaa can be any naturally occurring amino acid

<220>

<221>  misc_feature
<222>  (34)..(34)
<223>  Xaa can be any naturally occurring amino acid

<220>
<221>  misc_feature
<222>  (37)..(37)
<223>  Xaa can be any naturally occurring amino acid

<400>  100

Arg Leu Xaa Ala Leu Glu Thr Xaa Xaa Gln Asn Gln Gln Xaa Leu Xaa
1               5                   10                  15

Leu Trp Gly Cys Xaa Xaa Xaa Xaa Xaa Cys Tyr Thr Ser Val Xaa Trp
            20                  25                  30

Asn Xaa Thr Trp Xaa
        35

<210>  101
<211>  38
<212>  PRT
<213>  Artificial

<220>
<223>  Région de la boucle V3 de GP120 de DUR

<400>  101

Cys Val Arg Pro Gly Asn Asn Ser Val Lys Glu Ile Lys Ile Gly Pro
1               5                   10                  15

Met Ala Trp Tyr Ser Met Gln Ile Glu Arg Glu Gly Lys Gly Ala Asn
            20                  25                  30

Ser Arg Thr Ala Phe Cys
        35

<210>  102
<211>  37
<212>  PRT
<213>  Artificial

<220>
<223>  Région immunodominante de GP41 de DUR

<400>  102

Arg Leu Leu Ala Leu Glu Thr Leu Met Gln Asn Gln Gln Leu Leu Asn
1               5                   10                  15

Leu Trp Gly Cys Arg Gly Lys Ala Ile Cys Tyr Thr Ser Val Gln Trp
            20                  25                  30

Asn Glu Thr Trp Gly
        35

<210>  103

<211>  35
<212>  PRT
<213>  Artificial

<220>
<223>  Peptide VAU

<400>  103

Arg Leu Leu Ala Leu Glu Thr Phe Ile Gln Asn Gln Gln Leu Leu Asn
1               5                   10                  15

Leu Trp Gly Cys Lys Asn Arg Leu Ile Cys Tyr Thr Ser Val Lys Trp
            20                  25                  30

Asn Lys Thr
        35

## Description

L'invention concerne les antigènes obtenus par expression de séquences nucléotidiques ou par synthèse chimique par exemple en utilisant des synthétiseurs de marque Applied Biosystems, présents dans les variants d'VIH-1 groupe (ou sous-groupe) O et particulièrement les antigènes correspondant à ceux isolables des particules virales. A titre d'exemple de virus VIH-1 du sous-groupe O, on se réfère à l'isolat VIH-1_{(VAU)} ainsi qu'à l'isolat VIH-1_{(DUR)}.

Rappelons que la désignation HIV utilisée dans ce texte correspondant à la désignation VIH également utilisée.

L'invention concerne également les anticorps, polyclonaux ou monoclonaux, induits par ces antigènes.

L'invention est également relative à des séquences d'ADN clonées soit présentant une analogie de séquence, soit une complémentarité avec l'ARN génomique du virus précité. Elle concerne aussi des procédés de préparation de ces séquences d'ADN clonées. L'invention concerne également les polypeptides contenant des séquences d'acides aminés codés par les séquences d'ADN clonées.

De plus, l'invention concerne des applications des antigènes mentionnés précédemment à la détection in vitro chez l'homme de potentialité de certaines formes du SIDA et, en ce qui concerne certains d'entre eux, à la production de compositions immunogènes et de compositions vaccinantes contre ce rétrovirus. De même, l'invention concerne les applications aux mêmes fins des susdits anticorps et, pour certains d'entre eux, leur application à la production de principes actifs de médicaments contre ce sida humain.

L'invention concerne également l'application des séquences d'ADN clonées et de polypeptides obtenus à partir de ces séquences comme sondes ou amorces pour l'amplification génique, dans des trousses de diagnostics.

L'invention est également relative à des compositions d'antigènes, qui peuvent être obtenus par synthèse chimique ou par expression dans un hôte cellulaire recombinant et permettant le diagnostic d'une infection due à un rétrovirus humain de type HIV indépendamment du sous-type VIH-1 ou VIH-2. De telles compositions comprennent au moins un peptide choisi parmi les peptides antigéniques communs aux virus VIH-1, VIH-2, VIH-1 _{(DUR)} et VIH-1_{(VAU)} ou des variants de ces peptides antigéniques possédant des caractéristiques immunogènes semblables.

L'invention vise également des compositions permettant le diagnostic spécifique d'une infection due à un rétrovirus humain de type VIH-1, plus particulièrement le VIH-1, groupe M, le VIH-2 ou le VIH-1 groupe (ou sous-groupe) 0 et comprenant au moins un peptide antigénique spécifique du virus VIH-1, un peptide antigénique spécifique du virus VIH-2 et un peptide antigénique spécifique du virus VIH-1 groupe (ou sous-groupe) O ou des variants de ces peptides antigéniques possédant des caractéristiques immunogènes semblables. Plus particulièrement, les peptides antigéniques sont dérivés de la protéine d'enveloppe des virus VIH-1 groupe M, VIH-2 et VIH-1 groupe (ou sous-groupe) O.

L'invention vise par ailleurs un peptide permettant une détection d'anticorps anti-HIV que les peptides de l'art antérieur ne permettaient pas toujours de détecter en se basant notamment sur la découverte d'une nouvelle souche VIH-1: VIH-1 DUR. L'antisérum dirigé contre elle ne présente pas toujours de réactivité avec les peptides du consensus HIV tel qu'il est utilisé de nos jours. Le terme «consensus HIV » se réfère aux régions conservées entre isolats et dont la mise en évidence est essentielle à la conception de vaccins ou de réactifs diagnostiques, et dont les mutations confèrent la résistance aux médicaments antiviraux. Le terme «peptide» utilisé dans le présent texte, définit aussi bien les oligopeptides que des polypeptides.

### Etat de la technique

Deux types de virus de l'immunodéficience humaine (HIV) ayant une responsabilité dans le développement d'un SLA ou du SIDA ont été isolés et caractérisés. Un premier virus dénommé LAV-1 ou VIH-1 a été isolé et décrit dans la demande de brevet GB 8324.800 et la demande EP 84401.834 du 14/09/1964. Ce virus a également été décrit par F. Barré-Sinoussi et al dans Science (1983), 220. 868-871.

Le rétrovirus HIV de type 2 appartient à une classe distincte et n'a qu'une parenté immunologique réduite avec des rétrovirus HIV de type 1. Les rétrovirus VIH-2 ont été décrits dans la demande de brevet européen n° 87.400.151.4 publiée sous le numéro 239.425.

Le rétrovirus VIH-1 est le plus courant et sa présence est prédominante dans plusieurs régions du monde. Quant au rétrovirus VIH-2, il est le plus souvent retrouvé en Afrique de l'Ouest, bien que sa propagation à l'extérieur de cette région ait été récemment documentée par Grez et al, (1994) J. Virol. 68, 2161-2168.

L'ensemble des lentivirus de l'immunodéficience des primates, comprenant les virus du type 1 et du type 2 de l'immunodéficience humaine ainsi que plusieurs types de virus de primates non humains, augmente en taille et en complexité. Le plus courant de ces virus, le VIH-1, se répand actuellement sous forme d'épidémie mondiale et il est responsable d'un problème de santé public majeur. Peu après l'identification et la caractérisation moléculaire de ce virus, il a été reconnu comme étant très variable, et comprend actuellement plusieurs sous-types (Myers, 1994, Louwagie, et al. 1993, Louwagie, et al. 1992, Myers, G. (1994) VIH-1 subtypes and phylogenetics trees. In: Human Retrovirus and AIDS 1994; Myers, G., Korber, B., Wain-Hobson, S., Smith, R.F. and Pavlakis, G.N., eds. Los Alamos National Laboratory, Los Alamos, NM. III-2 - III-9.). Cette distinction de sous-types est surtout basée sur la divergence des gènes gag et *env.* Au moins 6 sous-types ont été identifiés, désignés A à F, mais plusieurs sont encore susceptibles d'émerger de l'enquête mondiale poussée en cours sur les isolats du VIH-1. Il a été trouvé que ces différents sous-types sont équidistants les uns des autres, dans un profil phylogénétique dit phylogénie en étoile, ce qui suggère que les différents sous-types du VIH-1 auraient évolué et divergé de façon synchrone à partir d'un ancêtre commun.

Récemment, deux virus distincts de ce groupe de virus VIH-1 ont été isolés et caractérisés. Ces deux virus ont été obtenus de patients vivant au Cameroun, en Afrique de l'Ouest Centrale (Gürtler, et al. 1994, Vanden Heasevelde, et al. 1994). Leur séquence, plus particulièrement la séquence de leur gène env (enveloppe), montre clairement que ces virus appartenaient à une catégorie distincte de virus apparentés au VIH-1, désignée VIH-1 groupe O(NKENGASONG et al., 1993).

Toutefois, la diversité des isolats à l'intérieur de ce groupe de virus apparentés au VIH-1 n'est pas connue, et sa propagation en dehors de l'Afrique n'a pas été documentée.

Une contrainte générale, dans la mise au point de tests sérologiques HIV, est d'éviter aussi bien les réactions faussement positives - ou faussement négatives - tout en conservant ou améliorant la sensibilité que permettent les tests antérieurs en détection de séropositivité.

Les tests basés sur l'emploi de peptide(s) consensus, essentiellement dérivés du gène « *env»,* ont été considérés comme une solution presque idéale jusqu'à ce que la découverte du variant VIH-1-O fasse entrevoir la possibilité de résultats faussement négatifs (Genomic cloning and complete sequence analysis of a highly divergent African human immuno-deficiency virus isolate. J. Virol. 1994; 68: 1586-96 ; a new subtype of human immuno-deficiency virus type 1 (MPV-5180) from Cameroon. J. Virol. 1994; 68:1581-85).

La non-réactivité de certains tests à antigène peptidique *«env»,* chez des patients présentant quand même certains syndromes cliniques caractéristiques du SIDA ou des syndromes lymphadénopathiques qui parfois les précèdent, est, à ce jour, parfois imputée à une infection du groupe VIH-1-O (VIH-1/VIH-2 seronegativity in VIH-1 subtype O infected patients, Lancet 1994; 343:1393-94 ; New VIH-1 subtype in Switzerland. Lancet 1994; 344:270-71).

### Description de l'invention

La présente invention a pour but de mettre à disposition des laboratoires de diagnostic des moyens, notamment des peptides spécifiques permettant une détection d'anticorps anti-HIV, qui étaient jusqu'à ce jour susceptibles d'être indétectables. Elle concerne également des mélanges de peptides issus de VIH-1 DUR et de peptides correspondants d'autres HIVs, de façon à éviter les résultats « faux négatifs » potentiels.

Elle concerne par ailleurs un procédé de détection et de discrimination dans un échantillon biologique entre des anticorps caractéristiques d'un rétrovirus du type VIH-1-M et des anticorps caractéristiques d'un rétrovirus VIH-1 groupe (ou sous-groupe) O.

L'invention découle des observations faites sur une femme séropositive, ayant séjourné au Cameroun et ayant révélé une réactivité sérologique atypique, au cours de plusieurs tests de dépistages de l'infection HIV, ces derniers ayant été confirmés par des techniques de « Western blot ».

Du fait de cette réactivité sérologique atypique, en particulier du manque de réactivité sur certains tests de troisième génération, même modifiés pour le type O, les inventeurs ont jugé intéressant d'entreprendre un séquencage de certaines parties du génome de cette souche VIH-1 DUR, plus spécifiquement des gènes GAG et ENV.

Cependant, les amplifications géniques par PCR à l'aide d'amorces issues du groupe M et d'amorces connues du groupe O ont été sans résultat pour les parties codant la boucle V3 de gp120, et pour la région immunodominante de gp41. Seule la région GAG était amplifiable à l'aide d'amorces connues dans l'art antérieur (Loussert-Ajaka I, Lancet 1994 ; 343:1393). Un autre but de la présente invention est par conséquent la détermination d'amorces susceptibles de palier ce problème. Ces amorces pourront indifféremment être dénommées « primers » par la suite.

On a déterminé des séquences partielles des glycoprotéines gp41 et de gp120 ainsi que de protéines de capside (gène GAG) à partir d'ADN lymphocytaire et de cultures virales, indiquant que cette souche VIH-1 DUR appartient d'une part au groupe VIH-1-O, et qu'elle diffère de façon importante du groupe M, plus particulièrement pour gp41 et gp120.

Ainsi, on a pu mettre en évidence, plus particulièrement à propos de la séquence GAG de VIH-1 (CUR), l'existence de séquences consensus dans le groupe O, sur plusieurs zones, distinctes de séquences consensus du groupe M sur les mêmes zones.

Le clonage des séquences codant les fragments de GAG, gp41 et gp120 de VIH-1_{(DUR)} a été effectué dans un plasmide Bluescript® contenant un site PST1. Les produits d'amplification ont été clonés soit selon les techniques classiques utilisant les amorces universelles T3 et T7, soit directement séquencés par l'utilisation des amorces de la précédente amplification. Les séquences ont ensuite été déterminées par le dispositif automatique de séquençage Applied Biosystems 373A (ESGS Montigny le Bretonneux, France).

Dans le contexte de la présente invention, les inventeurs ont isolé et séquencé le gène *env* d'un isolat du groupe O, le VIH-1_{(VAU)}, obtenu d'une patiente française qui n'a jamais voyagé en dehors de l'Europe et qui est morte du SIDA en 1992. Selon sa séquence d'enveloppe, le VIH-1_{(VAU)} est apparenté aux deux virus camerounais HIV_{ANT70} et HNM_{MVP5180} récemment caractérisés. L'analyse phylogénétique des séquences *env* révèle que les trois virus semblent constituer un groupe distinct, qui sera appelé ici VIH-1 groupe O. L'isolement du VIH-1_{(VAU)} de cette patiente indique également qu'une certaine propagation des VIH-1 du groupe O s'est déjà produite en dehors de l'Afrique.

### Isolement du virus VIH-1 _{(VAU)}

VIH-1_{(VAU)} a été isolé en 1992 d'une patiente française âgée de 41 ans atteinte du SIDA. Cette patiente présentait en 1986 une leuco-neutropénie sévère associée à un carcinome du col utérin. Néanmoins, elle a progressivement montré des signes d'infections opportunistes, avec une baisse du nombre de cellules T CD4⁺ circulantes et elle est morte du SIDA en 1992. Des anticorps anti-VIH-1 ont d'abord été détectés par ELISA (Elavia, SANOFI DIAGNOSTICS PASTEUR, et Test ABBOTT) en 1990.

La patiente n'avait jamais voyagé en dehors de l'Europe, n'avait pas utilisé de médicaments par voie intraveineuse et n'avait subi aucune transfusion sanguine connue. Aucun partenaire sexuel originaire de l'Afrique n'a été identifié. Elle a donné naissance à un enfant en bonne santé en 1971, mais un fils, né en 1980, est mort à l'âge d'un an suite à un épisode clinique très suggestif de SIDA néo-natal. Son troisième enfant né en 1983 et son mari sont actuellement en bonne santé et non infectés.

L'isolement du virus a été réalisé de la manière suivante : les cellules CD8+ présentes dans les PBMC (lymphocytes sanguins périphériques) de la patiente ont été éliminées à l'aide de billes revêtues d'anticorps IOT8 (Immunotech). Ces PBMC restantes ont été stimulées à la PHA, puis cocultivées avec des PBMC épuisées en CD8 provenant d'un donneur sain et stimulées à la PHA La croissance virale dans la coculture a été suivie par dosage de l'activité transcriptase inverse (RT) du surnageant et par test ELISA de la p24 du. VIH-1 (coffret de diagnostic commercialisé par DuPont de Nemours). Le virus obtenu de la coculture initiale a été soumis à plusieurs passages dans des cultures de PBMC épuisées en CD8 et stimulées à la PHA. Plusieurs tentatives ont été réalisées pour infecter par le VIH-1_{(VAU)} diverses lignées cellulaires transformées, dont des cellules MT4 (Harada, et al. 1985) et CEM (Rey, et al. 1989), ainsi que la lignée P4-2 de cellules Hela-CD4-LTRLacZ (Clavel et Chameau 1994).

### Caractérisation biologique du VIH-1_{(VAU)}

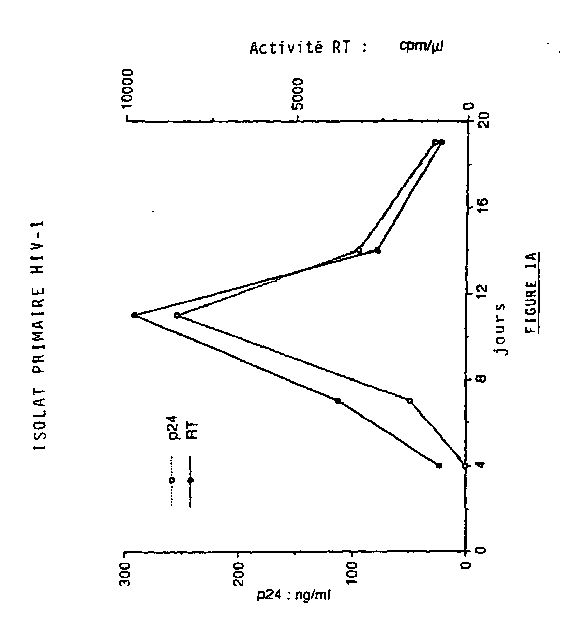

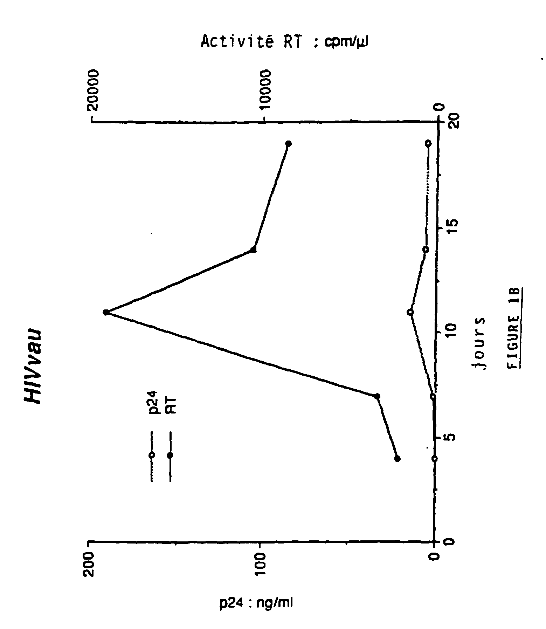

Deux semaines après la coculture des PBMC de la patiente épuisées en CD8, stimulées à la PHA, avec des cellules similaires d'un donneur sain, la production de virus a été détectée sous la forme d'un pic d'activité RT dans le surnageant de culture. Ce virus pouvait alors être soumis à des passages en série sur des PBMC normales, épuisées en CD8, stimulées à la PHA. Sur la figure 1, le panneau A représente la production du VIH-1_{(VAU)} dans des sumageants de cultures de PBMC infectées, contrôlées par dosage RT (cercles pleins) et ELISA de capture d'antigène p24 VIH-1 (cercles vides). La concentration de p24 VIH-1 est exprimée en ng/ml et l'activité RT en cpm/µl. Dans le panneau B, la même expérience a été effectuée avec un isolat primaire standard du VIH-1 d'un patient atteint du SIDA.

Bien que la croissance du VIH-1_{(VAU)} ait été facilement détectée à l'aide du dosage RT, la détection de virus dans les surnageants de culture à l'aide de l'ELISA p24 VlH-1 (DuPont) était nettement moins sensible. La figure 1 montre la comparaison entre les profils d'infection productive des PBMC avec soit le VIH-1 _{(VAU)} soit un isolat primaire du VIH-1 d'un patient atteint du SIDA, dosés en RT ou p24. Pour des quantités équivalentes de particules, déterminées par dosage de l'activité RT dans les sumageants dosés, approximativement 25 fois moins de p24 a été détecté pour le VIH-1(_{VAU}) que pour l'autre isolat VIH-1. La différence peut être consécutive au fait que l'anticorps monoclonal spécifique de la p24 du VIH-1, qui est utilisé pour revêtir les plaques ELISA, n'a qu'une faible affinité pour les produits gag du VIH-1 (VAU)

Plusieurs tentatives négatives ont été faites pour propager le VIH-1_{(VAU)} sur des lignées de cellules T humaines transformées, sensibles au VIH-1. En particulier, des cocultures entre des PBMC infectées par le VIH-1(VAU) et soit des cellules MT4 soit des cellules CEM n'ont pas conduit à la propagation du virus. Il a également été trouvé que ce virus n'était pas capable d'infecter des cellules HeLa CD4⁺ (P4-2) (Clavel et Chameau 1994) portant un gène lacZ inductible par le gène tat. De même, aucune réplication du VIH-1_{(VAU)} ne pouvait être détectée dans des lymphocytes sanguins périphériques activés de plusieurs chimpanzés.

L'analyse de la séquence d'enveloppe VIH-1_{(VAU)}, qui sera décrite en détail plus loin, et sa comparaison avec celle des deux isolats camerounais récemment décrits indiquent que tous les trois virus appartiennent au même groupe de virus apparentés au VIH-1. De plus, cette comparaison indique que ces trois variantes du virus sont approximativement phylogénétiquement équidistantes les unes des autres. Par conséquent, chacune des trois variantes de virus constitue en soi un sous-type, distinct, de leur groupe, qui est maintenant appelé VIH-1 groupe O. Ce groupe est distinct du groupe des autres isolats VIH-1, identifiés jusqu'ici, que les inventeurs appellent ici VIH-1, groupe M.

L'apparition de ce nouveau groupe pose la question de son origine : est-ce que le groupe O a évolué à partir des virus du groupe M (ou inversement) ou est-ce que chaque groupe a un passé distinct ? Les inventeurs pensent que dans la mesure où à la fois le groupe M et le groupe O présentent un profil de divergence interne similaire, il est vraisemblable qu'ils correspondent chacun à la diversification d'ancêtres distincts de virus dans des populations humaines distinctes. On ne peut pas apprécier à partir des données phylogénétiques et virologiques actuellement disponibles si l'ancêtre de l'un ou l'autre des deux groupes affectait les humains de manière naturelle ou a été introduit chez les humains à partir d'autres espèces. Le seul virus proche du VIH-1 présent chez un primate non humain est l'isolat SIVCPZGAB (Huet, et al. 1990), isolé à partir d'un chimpanzé apparemment infecté de façon naturelle, qui est clairement distinct à la fois du groupe M et du groupe O, et pour lequel aucun équivalent humain n'a été trouvé. Il est peu probable que les virus du groupe O aient récemment évolué à partir d'un virus de chimpanzé dans la mesure où le VIH-1_{(VAU)} n'a pas réussi à se répliquer dans des lymphocytes de chimpanzés.

Pourquoi l'épidémie du groupe O n'apparait-elle que maintenant, quelques 15 à 20 ans plus tard que le groupe M ? Il y a trois explications possibles : premièrement, l'introduction de l'ancêtre des virus du groupe O chez les humains se serait produite plus récemment que celle du groupe M; deuxièmement, il se pourrait qu'on ait permis au groupe M de se propager plus tôt que le groupe O en raison de différentes conditions sociales dans leur région d'origine; et troisièmement, les virus du groupe O auraient une capacité de transmission plus réduite, comparée à celle des virus du groupe M. Il a été proposé qu'une telle propriété explique l'absence de propagation mondiale significative du VIH-2, pour lequel une charge virale plus faible chez les sujets infectés est liée à une transmissibilité réduite (De Cock, et al. 1993). A cet égard, bien que nous n'ayons aucune donnée sur la charge virale chez les patients infectés par un VIH-1 du groupe O, le pouvoir pathogène de ces virus ne semble pas être différent de celui du VIH-1. La patiente chez qui le VIH-1_{(VAU)} a été isolé est morte du SIDA, comme le patient chez qui l'isolat HIV_{MVP5180} du groupe O a été obtenu.

Toutefois, l'histoire naturelle de l'infection de la patiente VIH-1 _{(VAU)} n'est toujours pas claire, mais il existe plusieurs indications que cette patiente a été infectée avant 1980, comme le suggère la mort à cette date de son deuxième enfant atteint d'un syndrome ressemblant au SIDA.

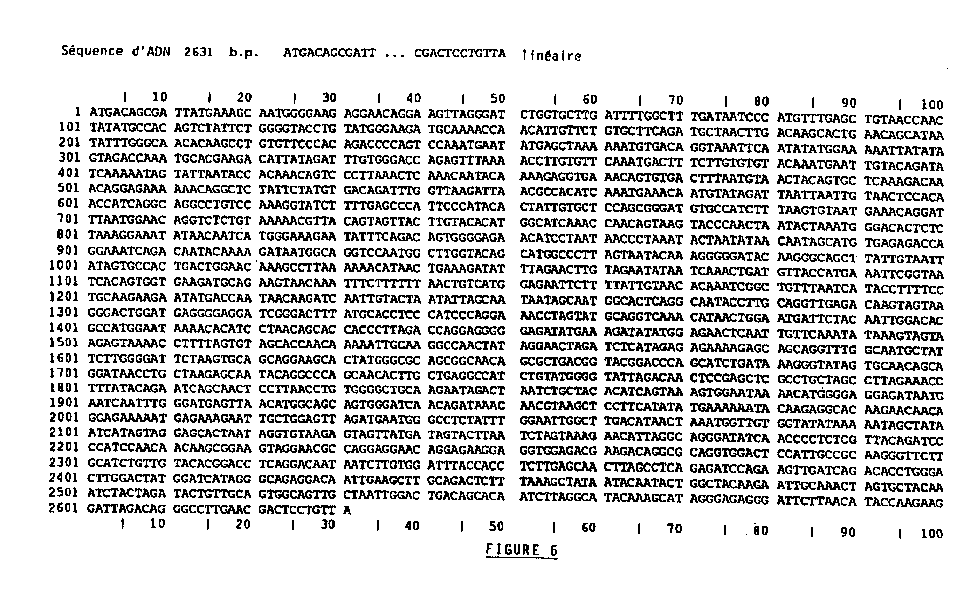

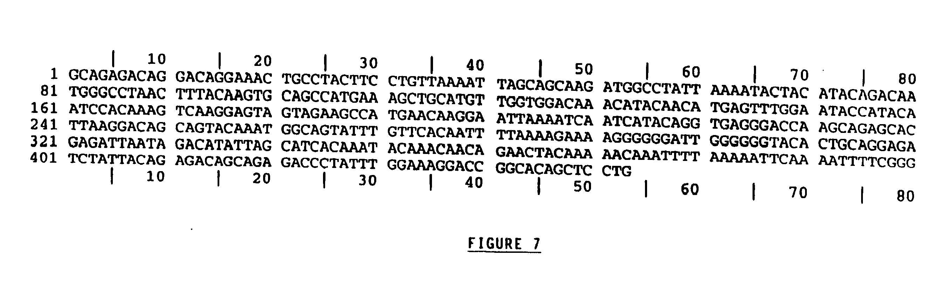

L'invention concerne tout variant des séquences d'acide nucléique du virus VIH-1_{(VAU)} ou de tout virus équivalent du groupe O, contenant des protéines de structure qui présentent les mêmes propriétés immunologiques que les protéines de structure codées par le gène env comprenant la séquence décrite à la figure 6 et dénommée « vau *»,* désignée encore par la SEC ID N° 5.

La présente invention est également relative à des compositions contenant soit des antigènes selon l'invention, soit un mélange d'antigènes selon l'invention associés à des extraits originaires d'un ou plusieurs VIH-1 groupe O ou d'autres virus variants, d'une part, et d'un ou plusieurs VIH-2 et/ou VIH-1, d'autre part, ces compositions étant éventuellement marquées. On peut utiliser tous types de marqueur approprié : enzymatiques, fluorescents, radioactifs, etc.

### Acides nucléiques

L'invention concerne les ADN ou fragments d'ADN, plus particulièrement ADN et fragments d'ADN clonés obtenus à partir de l'ARN, d'ADN_{c} ou d'amorces utilisables en PCR, ou autres méthodes d'amplification génique dérivés de l'ARN ou de l'ADN du rétrovirus VIH-1_{(VAU)}. L'invention concerne encore particulièrement tous les ADN équivalents, notamment tout ADN présentant des homologies de séquence avec l'ADN du VIH-1_{(VAU)}, en particulier avec la séquence codant la région *env* de la souche VIH-1_{(VAU)} comprenant la séquence correspondant à la SEQ ID N° 5 représentée à la figure 6 et appelée « vau ». L'homologie avec le VIH-1 groupe M est au moins égale à 50%, de préférence à 70% et plus avantageusement encore à environ 90%. D'une façon générale, l'invention concerne tout ADN (ou ARN) équivalent capable de s'hybrider avec l'ADN ou l'ARN d'un rétrovirus VIH-1 du groupe 0.

L'invention concerne aussi les séquences d'ARN correspondant aux séquences d'ADN définies ci-dessus.

L'invention concerne également le gène de l'intégrase du virus VIH-1_{(VAU)}.comprenant la séquence identifiée par la désignation SEQ ID N° 7 ou s'hybridant avec la SEQ ID N° 7. L'invention vise aussi les ARN correspondant à l'ADN ci-dessus décrit.

L'invention a aussi pour objet des compositions contenant les peptides ou polypeptides codés par l'ADN susdite ou des fragments d'ADN.

Des oligonucléotides dérivés de la séquence VAU ou encore du gène de l'intégrase du virus VIH-1_{(VAU)} particulièrement des oligonucléotides comprenant au moins 9 nucléotides, peuvent être utilisés pour la détection de séquences ADN ou ARN de virus VIH-1 du groupe O dans des prélèvements biologiques, des cultures cellulaires, ou des extraits de cellules, par la technique de la PCR ou toute autre technique d'amplification génique. Ces séquences pourraient être utilisées soit comme amorces d'amplification génique soit comme sondes en vue de la détection spécifique des produits d'amplification génique. Sont utilisables également comme sonde d'hybridation, les produits d'amplification, ou leur séquence correspondante synthétique, obtenus par synthèse chimique (Applied Biosystems).

L'invention couvre également tout fragment d'au moins 100 nucléotides utilisable comme sonde dans des réactions d'hybridation et capable de permettre une réaction avec une partie du génome d'un variant VIH-1_{(VAU)} dans des conditions d'hybridation de forte stringence.

### Clonage et séquençage du gène env VIH-1_{(VAU)}

Pour l'amplification initiale par PCR de l'ADN du VIH-1_{(VAU)}, l'ADN total a été extrait de PBMC infectées par le VIH-1_{(VAU)} et un segment du gène pol (région intégrase) a été amplifié à l'aide d'amorces dégénérées :
amorce 4506: 5'AGTGGAT(A/T)(T/C)ATAGAAGCAGAAGT3'; Seq. ID N°1;
amorce 5011 : 5'ACTGC(C/T)CCTTC(A/C/T)CCTTTCCA3'; Seq. ID N°2;

Le milieu de réaction incluant KCI 50 mM, Tris-HCl 10 mM (pH 8,9), MgCl₂ 1,5 mM, gélatine 0,1 mg/ml, dNTP 0,2 mM, Taq Polymérase 1 U (Amersham). La PCR a été effectuée pendant 43 cycles thermiques à 92°C pendant 10 secondes, 50°C pendant 1 minute, 72°C pendant 40 secondes.

Le produit d'amplification résultant a été cloné dans un vecteur pBluescript, engendrant le clone ph4 déposé à la CNCM le 20 octobre 1994 sous le n° I-1486 qui a ultérieurement été utilisé comme sonde pour cribler une banque lambda d'ADN de faible poids moléculaire, digéré par EcoRI, issu de cellules infectées par le VIH-1_{(vAU)}. Brièvement, les PBMC infectées par le VIH-1_{(VAU)} ont été cocultivées pendant 24 heures avec des PBMC neuves stimulées à la PHA et épuisées en cellules CD8⁺, après quoi un effet cytopathogène poussé (ECP) était visible. L'ADN de faible poids moléculaire a alors été extrait suivant la méthode de Hirt (Hirt 1967), et digéré avec l'enzyme EcoRI. Une analyse précédente en Southern-blot précédente de cet ADN avait bien montré que le génome VIH-1_{(VAU)} contenait un seul site EcoRI, permettant le clonage d'espèces d'ADN circulaire non intégré représentant la totalité du génome viral. Le produit de digestion résultant a été soumis à une électrophorèse sur gel d'agarose, et la population de fragments d'ADN d'une taille de 8-12 kb approximativement a été purifiée et ligaturée à de l'ADN lambda Zap digéré par EcoRI (Stratagene). Après l'encapsidation, l'étalement et le criblage par hybridation avec de l'ADN ph4 marqué au ³²P, un clone, λ H34, a été identifié comme étant positif, et amplifié. L'inserat EcoRI a été purifié, soniqué, et cloné par "shotgun" dans le vecteur M13mp18 traité à la phosphatase, digéré avec l'enzyme Smal. Cent cinquante des clones obtenus ont été séquencés sur un séquenceur d'ADN 373A (Applied Biosystems), et les séquences résultantes ont été rassemblées en une seule séquence à l'aide du progiciel d'analyse d'ADN GCG-Wisconsin.

L'analyse de cette séquence a révélé de nombreux codons non-sens dans tous les cadres de lecture, ce qui est très suggestif d'un génome hypermuté (Vartanian, et al. 1991). Cette séquence étant inutilisable, il a par conséquent été décidé d'amplifier par la PCR le gène *env* du VIH-1_{(VAU)} à l'aide de l'ADN total de PBMC infectées par le VIH-1(_{VAU}), et des amorces oligonucléotidiques dérivées de la séquence λH34 :
amorce TH2 5'GCTCTAGATGGGGATCTCCCATGGCAGG3' Seq. ID N° 3;
amorce UH2 5'GCTCTAGATCAGGGAAGAATCCCTGAGTGT3'. Seq. ID N° 4.

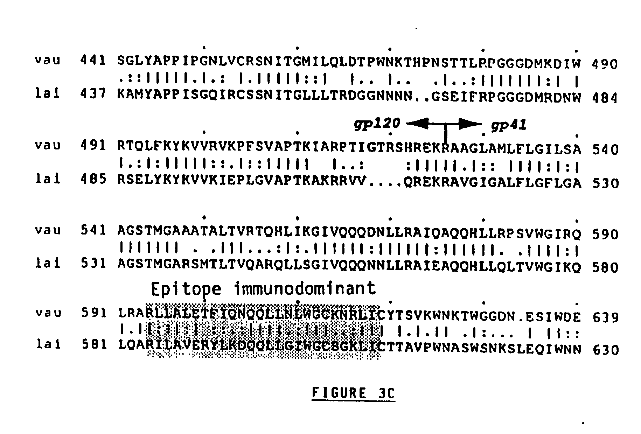

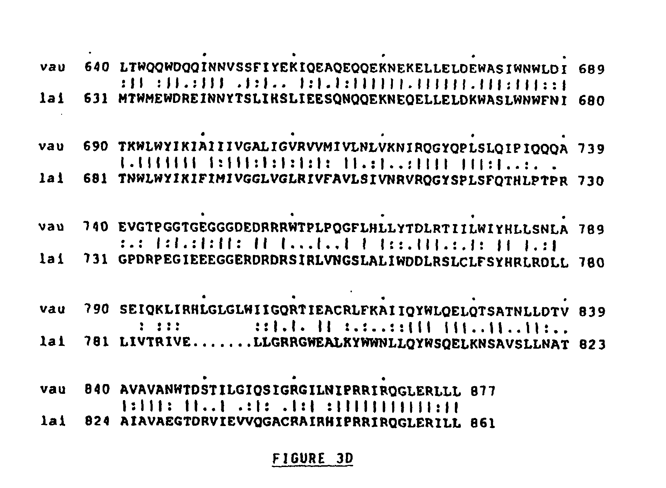

L'amplification par la PCR a été effectuée pendant 35 cycles thermiques à 92°C pendant 15 secondes, 52°C pendant 1 minute, 60°C pendant 2 minutes et 72°C pendant 2 minutes. Le produit d'amplification résultant, d'une taille de 3,5 kb, a été cloné dans le vecteur M13mp18 et séquencé par réactions successives, en utilisant d'abord l'amorce universelle de séquençage M13, puis des amorces déduites des séquences amont. L'analyse des séquences nucléotidiques et peptidiques a été effectuée à l'aide du progiciel d'analyse d'ADN GCG-Wisconsin. Le gène *env* VIH-1_{(VAU)} code 877 acides aminés au total, incluant le peptide signal. La séquence nucléotidique du gène *env* VIH-1_{(VAU)} correspond à la Seq. ID N° 5 (voir figure 3).

### Utilisation d'acides nucléiques à titre de sondes

L'invention concerne naturellement aussi l'utilisation des ADN, ADN_{c} ou de leurs fragments, ou de plasmides recombinants ou autres vecteurs équivalents contenant ces fragments, en tant que sondes, pour la détection de la présence ou non du virus VIH-1_{(VAU)} dans des échantillons de sérum ou autres liquides ou tissus biologiques obtenus à partir de patients suspectés d'être porteurs du virus VIH-1_{(VAU)}. Ces sondes sont éventuellement marquées (marqueurs radioactifs, enzymatiques, fluorescents, etc.). Des sondes particulièrement intéressantes pour la mise en oeuvre du procédé de détection du virus VIH-1_{(VAU)} ou d'un variant VIH-1_{(VAU)} peuvent être caractérisées en ce qu'elles comprennent la totalité ou une fraction de l'ADN complémentaire du génome du virus VIH-1_{(VAU)} ou encore notamment les fragments contenus dans divers clones. On mentionnera plus particulièrement une fraction de l'ADN_{c} du VIH-1_{(VAU)} contenant tout ou partie de la région *env.*

Les sondes mises en oeuvre dans ce procédé de détection du virus VIH-1_{(VAU)} ou dans des trousses de diagnostic, ne sont en aucune façon limitées aux sondes décrites précédemment. Elles comprennent toutes les séquences nucléotidiques issues du génome du virus VIH-1 (VAU), d'un variant VIH-1_{(VAU)} ou d'un virus proche par sa structure, dès lors qu'elles permettent la détection, à partir de fluides biologiques de personnes susceptibles de présenter un sida, d'un virus VIH-1 du groupe O, en particulier VIH-1_{(VAU)} par hybridation avec de l'ADN ou de l'ARN du virus VIH-1_{(VAU)}.

Particulièrement avantageuses sont les sondes qui, lors de l'hybridation avec VIH-1, donnent une réaction forte avec les HIV appartenant au groupe O et une réaction faible avec les VIH-1 appartenant au groupe M. A titre d'exemple non limitatif, une sonde fabriquée à partir de la séquence du gène de l'intégrase du virus VIH-1_{(VAU)} (SEQ ID N° 7) donne, lorsqu'elle est hybridée avec VIH-1 dans des conditions d'hybridation telles que celles décrites dans le brevet EP 178 978, une réaction forte avec les HIV du groupe O et une réaction faible avec les VIH-1 du groupe M.

La détection peut être réalisée de toute façon en soi connue, notamment :
par une mise en contact de ces sondes soit avec les acides nucléiques obtenus à partir de cellules contenues dans des fluides biologiques (par exemple liquide céphalo-rachidien, salive, etc.), soit avec ces fluides eux-mêmes, dès lors que leurs acides nucléiques ont été rendus accessibles à l'hybridation avec ces sondes, et ce dans des conditions permettant l'hybridation entre ces sondes et ces acides nucléiques
et par une détection de l'hybridation éventuellement produite.

Le susdit diagnostic mettant en jeu des réactions d'hybridation peut également être réalisé à l'aide de mélanges de sondes respectivement dérivées du VIH-1_{(VAU)}, du VIH-1 et du VIH-2, dès lors qu'il n'est pas nécessaire de faire la distinction entre les types de virus HIV recherchés.

L'invention a aussi pour objet des vecteurs d'expression contenant la séquence codant pour les protéines d'enveloppe de VIH-1 ou contenant la séquence codant l'intégrase.

L'invention comprend des compositions pour la détection de la présence ou non de virus VIH-1_{VAU} dans des échantillons de sérum ou d'autres liquides ou tissus biologiques, obtenus à partir de patients susceptibles d'être porteurs du virus VIH-1_{VAU}. Ces compositions sont caractérisées en ce qu'elles comprennent au moins une sonde obtenue à partir d'une séquence nucléotidique issue ou dérivée du génome du virus VIH-1_{VAU}, particulièrement un fragment d'ADN VIH-1_{VAU} contenant la région ou une partie de la région codant la protéine *env* du virus VIH-1_{VAU} ou d'un variant de VIH-1_{VAU}.

Avantageusement, la composition décrite ci-dessus comprend également une sonde obtenue à partir d'une séquence nucléotidique issue de VIH-1 ou de VIH-2.

D'autres compositions de diagnostic comprennent les amorces de l'invention utilisables en amplification génique de rétrovirus de sous-groupe 0 ou variants de ces rétrovirus.

### Antigènes, notamment protéines et glycoprotéines

L'invention concerne une protéine de rétrovirus VIH-1 groupe (ou sous-groupe) O, ou polypeptide ou peptide naturel ou synthétique comprenant au moins une partie de ladite protéine, susceptibles d'être reconnus par des anticorps isolables à partir de sérum obtenu suite à une infection par une souche VIH-1 groupe 0 VAU, ou une souche VIH-1 groupe O DUR.

L'invention concerne une protéine d'enveloppe externe du rétrovirus VIH-1_{VAU} codée par le gène comprenant la séquence correspondant à la SEQ ID N° 5. Selon un mode de réalisation préféré de l'invention, cette protéine est en outre caractérisée en ce qu'elle comprend la séquence d'acides aminés correspondant à la Seq ID N° 6 représentée sur la figure 3 et comportant les résidus d'acides aminés 1 à 526. L'invention a aussi pour objet tout polypeptide ou variant, dérivé de ladite séquence, ayant un épitope susceptible d'être reconnu par des anticorps induits par le virus VIH-1_{VAU.}

La susdite protéine peut être obtenue sous forme glycosylée ou non glycosylée.

L'invention a aussi pour objet une protéine transmembranaire d'enveloppe comprenant l'enchaînement d'acides aminés SEQ ID N° 8 représenté sur la figure 3 entre les résidus d'acides aminés 527 et 877. Cette protéine transmembranaire est, dans le cadre de l'invention, sous forme glycosylée ou non.

L'invention concerne tous les antigènes, notamment protéines, glycoprotéines, polypeptides ou peptides, obtenus par expression de séquences codantes du génome VIH-1_{(VAU)} et ayant des propriétés immunologiques équivalentes à ceux ou celles du VIH-1_{(VAU)}. Les antigènes sont dits équivalents dans le cadre de la présente invention, dès lors qu'ils sont reconnaissables par les mêmes anticorps, notamment des anticorps isolables à partir de sérum obtenu d'un patient ayant été infecté par un VIH-1_{(VAU)}.

En particulier, l'invention a pour objet les peptides ou polypeptides synthétisés chimiquement et dont la séquence d'acides aminés est contenue dans celle des protéines d'enveloppe VIH-1_{(VAU)}, représentée sur la figure 3, ou les peptides ou polypeptides équivalents.

On doit aussi inclure, parmi les peptides, polypeptides, protéines ou glycoprotéines équivalents, les fragments des antigènes qui précèdent et les peptides préparés par synthèse chimique ou par génie génétique, dès lors qu'ils donnent lieu à des réactions immunologiques croisées avec les antigènes dont ils sont dérivés. En d'autres termes, l'invention concerne tout peptide ou polypeptide ayant des épitopes identiques ou semblables aux épitopes des susdits antigènes, susceptibles d'être reconnus par les mêmes anticorps. Font partie de ce dernier type de polypeptides, les produits d'expression de séquences d'ADN correspondant aux séquences des ADN codant les polypeptides ou antigènes susdits.

Plus particulièrement, les antigènes obtenus à partir du virus VIH-1_{(VAU)} ou produits par génie génétique ou synthèse chimique classique et qui présentent le plus grand intérêt dans le contexte de la présente invention sont les antigènes qui permettent d'établir une distinction claire entre les virus VIH-1_{(VAU)} de l'invention et les virus des groupes VIH-1 et VIH-2. A cet égard, des différences considérables ont été observées au niveau de la protéine d'enveloppe du virus VIH-1_{(VAU)} ainsi qu'au niveau de l'épitope immunodominant de la portion externe de la protéine PM. Il semble que les protéines gag et pol présentent davantage de similitude avec le virus VIH-1 que la protéine d'enveloppe.

L'invention concerne aussi des peptides ou polypeptides identiques à la région immunodominante de la glycoprotéine transmembranaire d'enveloppe du VIH-1_{(VAU)}. Cette région est représentée à la figure 3.

Des polypeptides préférés de cette région sont par exemple ceux qui contiennent la séquence CKNRLIC ou correspondent à cette séquence. Il peut s'agir aussi de polypeptides ou peptides répondant à la séquence RLLALETFIQNWWLLNLWGCKNRLIC ou comprenant cette séquence.

Un autre peptide préféré, identifié ci-après par la dénomination « peptide VAU», répond à la séquence suivante ou comprend cette séquence ou toute partie de cette séquence susceptible d'être reconnue par des anticorps dirigés contre le rétrovirus VIH-1_{(VAU)} RARLLALETFIQNQQLLNLWGCKNRLICYTSVKWNKT.

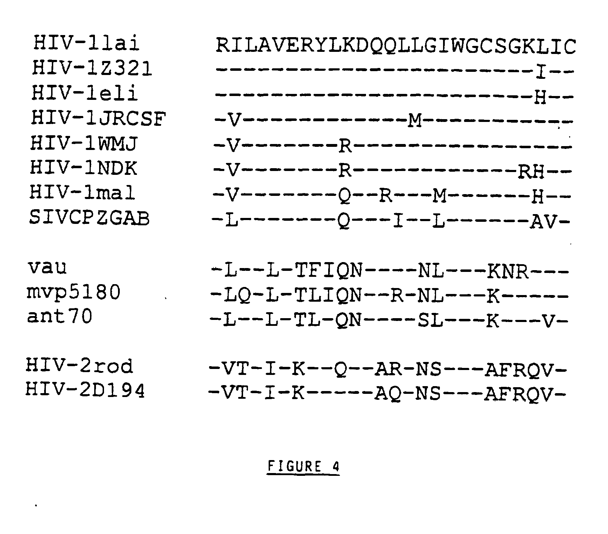

Des polypeptides variants de cette séquence sont par exemple les polypeptides représentés sur la figure 4 pour les isolats VIH-1_{(MVP5180)} et VIH-1₍ₐₙₜ₇₀₎. Ces polypeptides peuvent aussi être dérivés des précédents par insertion et/ou délétion et/ou substitution, par exemple substitution conservative par des résidus d'acides aminés.

La présente invention concerne un peptide issu du virus VIH-1-O DUR déposé le 23 février 1995 à la CNCM sous la référence I-1542, ou un peptide dont la séquence se distingue de celle du précédent par substitution, délétion ou addition d'acides aminés, ce peptide distinct conservant néanmoins les caractéristiques antigéniques du précédent.

D'autres peptides entrant dans le cadre de l'invention sont définis ci-après.

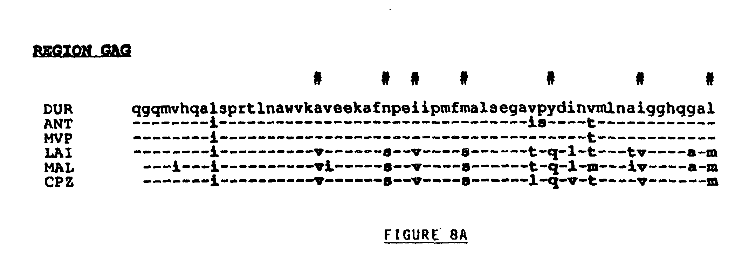

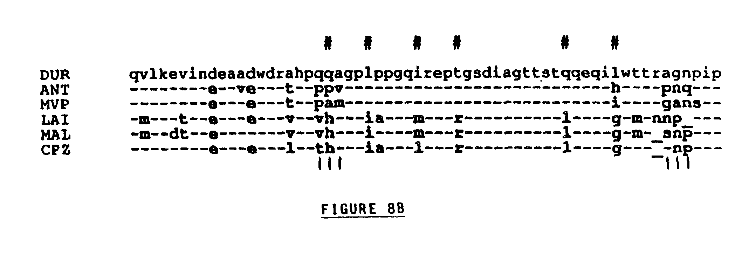

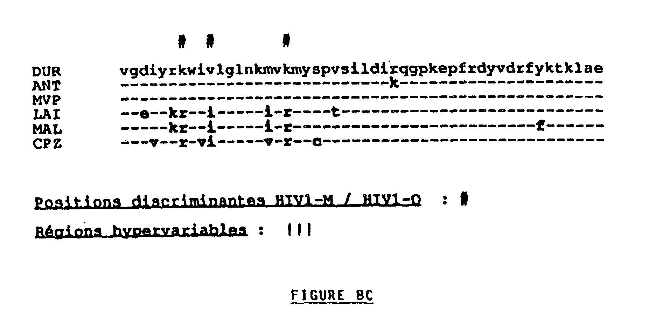

Ainsi, un peptide préféré de l'invention est un peptide comportant au moins 4 acides aminés consécutifs contenus dans la séquence GAG représentée à la figure 8 ou d'une séquence GAG immunologiquement semblable issue d'un variant du virus VIH-1-O DUR, ladite séquence immunologiquement semblable étant reconnue par des anticorps reconnaissant aussi de façon spécifique au moins l'une des séquences AHPQQA, LWTTRAGNP contenues dans la séquence GAG de la figure 8.

Préférentiellement, ce peptide consiste en un peptide dont la séquence en acides aminés est contenue soit dans l'une des séquences suivantes :
SPRTLNAWVKAVEEKAFNPEIIPMFMALSEGA (1)
MLNAIGGHQGALQVLKEVIN (2).
GPLPPGQIREPTGSDIAGTTSTQQEQI (3)
IPVGDIYRKWIVLGLNKMVKMYSPVSILDI (4)
QGPKEPFRDYVDRFYKTKLAE (5)
AHPQQA (5 bis)
LWTTRAGNP (5ter),
soit dans une séquence immunologiquement semblable correspondante, ce peptide comportant au moins 4 acides aminés consécutifs de l'une desdites séquences.

Préférentiellement encore, ce peptide consiste en un peptide dont la séquence en acides aminés est contenue soit dans l'une des séquences suivantes :
SPRTLNAWVK (6)
GSDIAGTTST (7)
QGPKEPFRDYVDRF (8)
soit dans une séquence immunologiquement semblable correspondante, ce peptide comportant au moins quatre acides aminés consécutifs de l'une desdites séquences.

Des peptides particulièrement préférés dans la présente invention sont les peptides contenant ;
- la séquence en acides aminés NPEI (9)
   ou
- la séquence en acides aminés AVEEKAFNPEIIPMFM (10), et plus particulièrement les peptides dont la séquence en acides aminés est contenue, soit dans l'une des séquences suivantes :
   IGGHQGALQ (23)
   REPTGSDI (24)
soit dans une séquence immulogiquement semblable correspondante, ce peptide comportant au moins 4 acides aminés consécutifs de l'une desdites séquences, ainsi que le peptide dont la séquence en acides aminés est contenue, dans la séquence d'acide aminés suivante :
INDEAADWD (25)
soit dans une séquence immulogiquement semblable correspondante, ce peptide comportant au moins 4 acides aminés consécutifs de ladite séquence.

La présente invention concerne les séquences d'acides nucléiques codant les peptides (23), (24) et (25) ainsi que les séquences d'acides nucléiques codant les séquences immunologiquement semblables ainsi que les compositions comprenant au moins un de ces acides nucléiques.

L'invention concerne également l'utilisation d'au moins un de ces acides nucléiques pour la détection et la discrimination entre des souches VIH-1 groupe M et HIV-1 groupe O.

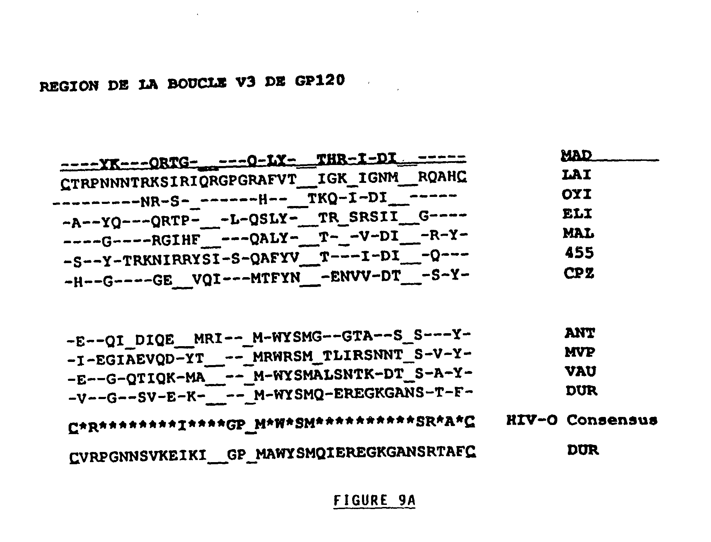

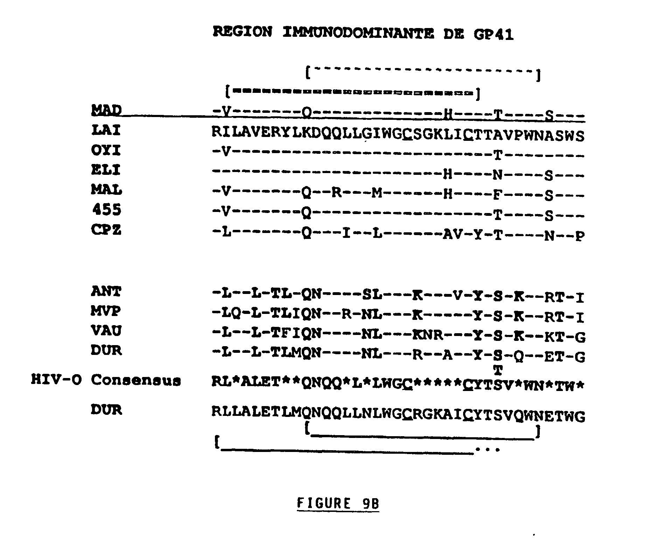

Entre également dans le cadre de la présente invention un peptide issu du virus VIH-1-O DUR défini ci-dessus, ledit peptide comportant au moins 4 acides aminés consécutifs, de la boucle V3 de gp120 représentée à la figure 9 ou de la séquence correspondante immunologiquement semblable, issue d'un variant du virus VIH-1-O DUR, ladite séquence immunologiquement semblable étant reconnue par des anticorps reconnaissant aussi de façon spécifique au moins l'une des séquences :
KEIKI (12),
EREGKGAN (13).
CVRPGNNSVKEIKI (14),
QIEREGKGANSR (15).

De façon préférée, ce peptide contient:
a) soit la séquence CVRPGNNSVKEIKIGPMAWYSMQIEREGKGANSRTAFC (11) ou une partie de cette séquence qui contienne au moins 4 acides aminés
b) soit une séquence d'acides aminés distincte de la séquence de a) dans laquelle un ou plusieurs acides aminés sont substitués par un ou plusieurs acides aminés sous réserve que le peptide conserve sa réactivité avec un antisérum contre le susdit peptide,
c) soit une séquence d'acides aminés distincte selon a) ou b) dans laquelle un ou plusieurs acides aminés ont été supprimés ou ajoutés sous réserve que le peptide conserve sa réactivité avec un antisérum contre le peptide de a),
d) soit une séquence ou partie de séquence immunologiquement semblable correspondante.

Préférentiellement encore, ce peptide contient soit
la séquence KEIKI (12),
soit
la séquence EREGKGAN (13),
soit
la séquence GPMAWYSM (16).

De façon particulièrement préférée, un peptide comme défini ci-dessus contient la séquence d'acides aminés CVRPGNNSVKEIKI (14), soit la séquence QIEREGKGANSR (15).

Entre également dans le cadre de l'invention un peptide issu du virus VIH-1-O DUR tel qu'il est défini ci-dessus, ledit peptide comportant au moins 4 acides aminés consécutifs, dont la séquence entière est contenue dans la séquence de la région immunodominante de la gp41 représentée à la figure 9 ou dans une séquence correspondante immunologiquement semblable, issue d'un variant du virus VIH-1-O DUR, ladite séquence immunologiquement semblable étant reconnue par des anticorps reconnaissant aussi de façon spécifique au moins l'une des séquences :
RLLALETLMQNQQL (17),
LNLWGCRGKAICYTSVQWNETWG (18),
CRGKAI (19),
SVQWN (20),
RLLALETLMONQQLLNLWGCRGKAICYTS (21),
QNQQLLNLWGCRGKAICYTSVQWN (22).

De façon préférée, ce peptide est un peptide contenant la séquence RLLALETLMQNQQL (17), ou LNLWGCRGKAtCYTSVQWNETWG (18) ou partie de ce peptide (18) contenant :
a) soit la séquence CRGKAI (19), soit la séquence SVQWN (20) dans laquelle Q est le cas échéant remplacé par un acide aminé différent, néanmoins différent aussi de K, soit les deux séquences à la fois,
b) soit une séquence d'acides aminés distincte de la séquence de a) dans laquelle un ou plusieurs acides aminés sont substitués par deux acides aminés sous réserve que le peptide conserve sa réactivité avec un antisérum contre le peptide de a),
c) soit une séquence d'acides aminés distincte selon a) ou b) dans laquelle un ou plusieurs acides aminés ont été supprimés ou ajoutés sous réserve que le peptide conserve sa réactivité avec un antisérum contre le peptide de a),
d) soit dans une séquence ou partie de séquence immunologiquement semblable correspondante.

Préférentiellement encore, ce peptide possède l'une ou l'autre des caractéristiques suivantes :
- sa séquence N terminale, qui contient au moins 8 acides aminés, n'est pas immunologiquement reconnue par des anticorps formés contre la séquence RILAVERY contenue dans la région immunodominante de la gp41 de la souche VIH-1-LAI.
- il n'est pas reconnu par des anticorps formés contre le peptide SGKLIC de la souche VIH-1-LAI.
- il contient l'une ou l'autre des deux séquences suivantes :
   RLLALETLMONQQLLNLWGCRGKAICYTS (21)
   QNQQLLNLWGCRGKAICYTSVQWN (22)

### Synthèse de peptides VAU

Un peptide VAU a été préparé par la technique classique de synthèse peptidique en phase solide par la méthode Fmoc en « flux continu ». Le peptide a été préparé à l'aide d'un synthétiseur Milligen 9050 PEP en utilisant la résine "Millipore" PEG PAL, substituée par le premier résidu d'acide aminé C-terminal. Les chaines latérales des acides aminés sont protégées par les groupes suivants : Pmc pour l'arginine ; Trt pour l'asparagine, la glutamine et la cystéine ; Boc pour la lysine ; tBu ester pour l'acide glutamique ; tBu éther pour la sérine, la thréonine et la tyrosine. Les groupements temporaires Fmoc sont éliminés par une solution de pipéridine à 20% en DMF. Les réactions de couplage de chaque acide aminé sont réalisées avec 6 équivalents de DIPCDI et d'HOBT. Certains résidus nécessitent un double couplage notamment les arginines 1 et 23, les cystéines 19 et 26, l'asparagine 11, les glutamines 10, 12 et 13, l'alanine 4, l'isoleucine 9, les leucines 2, 3, 14 et 15.

Après couplage, la résine est séchée sous vide. Le peptide est clivé du support par le réactif K pendant 4 heures à température ambiante. Le peptide brut est précipité et lavé à l'éther éthylique. Le produit est purifié par chromatographie liquide sous haute pression (HPLC) sur un instrument WATERS LC PREP 4000 avec des cartouches WATERS Delta Pak C18 40x100mm, débit 30 ml/mn, gradient acétonitnte/TFA 0,1%. Les fractions contenant le peptide sont rassemblées, concentrées à l'évaporateur rotatif puis lyophilisées.

### Cyclisation

Le peptide (0,025mM) est dissous dans une solution d'acétate d'ammonium 10mM. Le pH est amené à 8,5 avec une solution d'ammoniaque 1 M. Le pH est réajusté après 3 ou 4 heures. La cyclisation est suivie par HPLC à 214 nm et 280 nm, colonne WATERS Delta Pak C18 5µ, gradient acétonitrile/TFA 0,1%. La cyclisation est terminée au bout de 15 heures. Le pH est amené à 6 à l'aide d'acide acétique 97-100%, la solution est lyophilisée puis purifiée dans les mêmes conditions que pour le peptide brut.

Le peptide est contrôlé par HPLC et par spectrométrie de masse selon la technique d'électrospray (spectrophotomètre FISON VG Trio 2000).
Fmoc : Fluoroenyl-9 methyloxycarbonyl
Pmc : Pentanemethyl-8 chromane sulfonyl-6
Trt : Tritryl
Boc : Tertbutyloxycarbonyl
tBU : tert butyl
DMF : Dimethylformamide
DIPCDl : Diisopropyl carbodiimide
HOBT : Hydroxy-1 benzotriazole
TFA : acide trifluoroacétique
Réactif K : Phénot/Eau/Thioanisote/Ethanedithiot/TFA:
2,5ml/2,5ml/2,5ml/1,5ml/41 ml

### Comparaison de la séquence d'acides aminés de l'enveloppe VIH-1_{(VAU)} avec la séquence correspondante d'autres virus HIV.

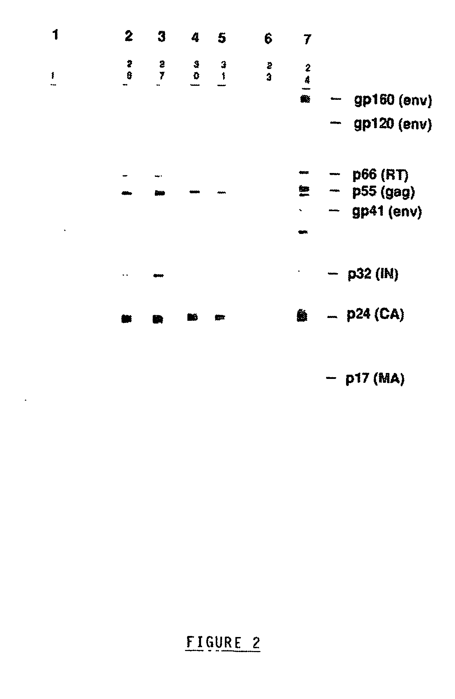

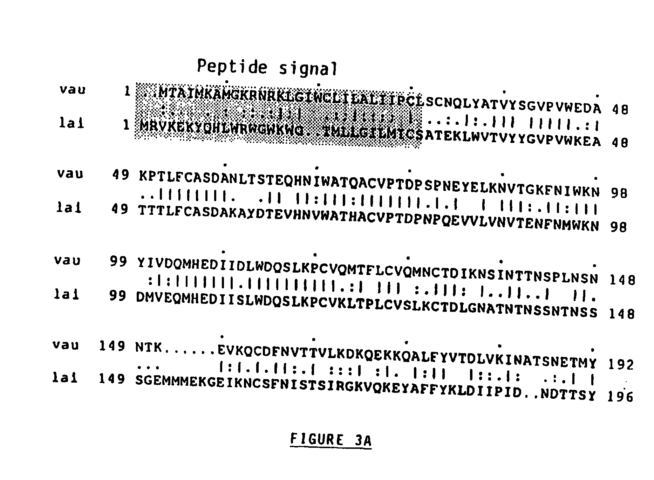

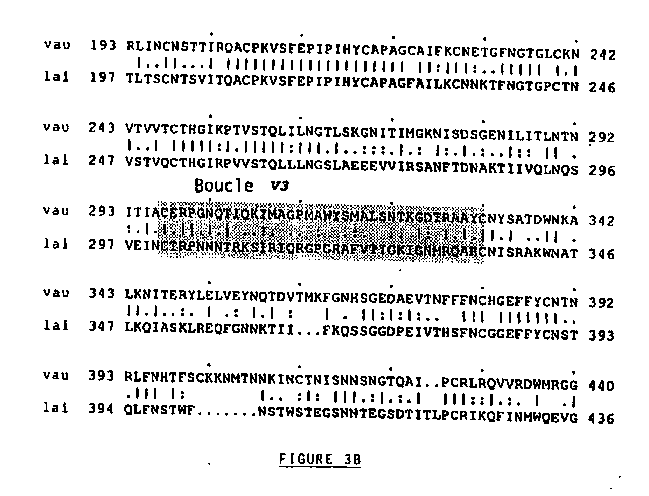

L'analyse en Westem-blot d'une série d'échantillons sériques provenant d'une patiente infectée par le VIH-1_{(VAU)} est présentée sur la figure 2. Des bandelettes de nitrocellulose, portant des protéines séparées par électrophorèse et provenant de particules de HIV purifiées (LAV BLOT, SANOFI DIAGNOSTICS PASTEUR), ont été mises à incuber avec des échantillons de sérum et leur réactivité a été évaluée selon les protocoles recommandés par le fabricant. Les résultats obtenus sont les suivants : Bandelette 1 : protéines spécifiques de VIH-2, qu'on a fait réagir avec un échantillon de sérum de la patiente VIH-1_{(VAU)} obtenu en février 1992. Bandelettes 2-7 : sérums positifs VIH-1 ; sérums de la patiente VIH-1_{(VAU)}: 2: obtenu en novembre 1990; 3 : en décembre 1990; 4 : en février 1991; 5 : en février 1992; 6 : témoin négatif; 7 : témoin positif (sérum d'un individu infecté par le VIH-1). Les noms et la taille des protéines (en kD) sont indiqués dans la marge.

La figure 3 montre un alignement de la séquence d'acides aminés de l'enveloppe de VIH-1_{(VAU)} avec la séquence correspondante de l'isolat de référence VIH-1 LAI (Wain-Hobson, et al. 1985). Les peptides signal, la boucle V3 et l'épitope immunodominant gp41 sont mis en relief par des rectangles hachurés. Le site de clivage entre la glycoprotéine d'enveloppe externe gp120 et la gp41 transmembranaire est indiqué par des flèches. Les lignes verticales entre les lettres d'acides aminés indiquent une identité complète, les deux-points (:) indiquent une forte homologie, et les points (.) indiquent une homologie limitée entre des acides aminés individuels. L'alignement a été effectué en utilisant le programme GAP du progiciel GCG-Wisconsin.

La version originale (1.0) des programmes GAP et BESTFIT a été écrite par Paul Haeberli à partir d'une étude détaillée des publications de Needleman et Vunsch (J. Mol. biol. 48, 443-453 (1970) et de Smith et Waterman (Adv. Appl. Math. 2; 482-489 (1981). Les alignements limités ont été mis au point par Paul Haeberli et ajoutés au progiciel pour constituer la version 3.0. Ils ont ensuite été fusionnés en un seul programme par Philip Marquess pour constituer la version 4.0. Les pénalités d'absence d'écart dans l'alignement des protéines ont été modifiées tel que suggéré par Rechid, Vingron et Argos (CABIOS 5 ; 107-113 (1989).

L'alignement de la figure 3 révèle de nombreuses zones de divergence poussée, avec quelques domaines conservés çà et là. Ces régions conservées correspondent grossièrement aux domaines également conservés dans les isolats VIH-1 classiques (Alizon, et al. 1986, Benn, et al. 1985). Parmi les domaines divergents, la boucle V3, appelée également déterminant principal de neutralisation (Javaherian, et al. 1990, Javaherian, et al. 1989, Matsushita, et al. 1988) est clairement l'un des plus divergents, bien que les deux cystéines définissant la boucle soient conservées. La séquence de la coiffe de la boucle, GPGRAF pour le VIH-1 LAI est GPMAWY chez le VIH-1_{(VAU)}. Ce motif de la coiffe est identique à celui de l'isolat du groupe O camerounais HIV_{ANT70} (Van den Heasevelde, et al. 1994), mais est différent de celui de l'autre isolat du groupe O, HIV_{MP5180} (Gürtler, et al. 1994), pour lequel le motif est GPMRWR.

Dans l'ensemble de l'enveloppe, 29 sites de N-glycosylation potentiels ont été identifiés au total, dont 13 sont conservés comparativement à d'autres protéines d'enveloppe VIH-1. On a également trouvé 19 cystéines conservées au total, ce qui indique que l'architecture globale de repliement de la protéine est conservée, mais on a trouvé 5 cystéines non conservées.

La figure 4 montre l'alignement multiple des peptides immunodominants dans le segment extracellulaire de la glycoprotéine d'enveloppe transmembranaire de différents isolats VIH-1. Toutes les séquences sont comparées à la séquence de référence VIH-1 LAI. Des traits d'union indiquent une identité au VIH-1LAI. L'alignement a été effectué à l'aide du programme PILEUP du progiciel GCG-Wisconsin.

Dans le programme PILEVP, la stratégie de rassemblement représentée par le dendrogramme est nommée UPGMA, qui signifie en anglais « Unweighted pair-group method using arithmetic averages» (Smith, P.H.A Sokal, R.R. (1973) dans Numerical Taxonomy (p.p. 230-234), W.H. Freeman and Company, San Francisco, Califomia, USA). Chaque alignement en pair de PILEVP utilise la méthode de Needleman et Wunsch (Journal of Molecular Biology 48 ; 443-453 (1970)).

Comme le montre la figure 4, la séquence d'acides aminés de l'épitope immunodominant de la portion extérieure de la protéine TM (Gnann, et al. 1987) est sensiblement différente de celle d'autres isolats VIH-1 et VIH-2. Toutefois, elle a gardé la plupart des acides aminés qui se sont révélés conservés entre les virus VIH-1 et VIH-2.

On a trouvé que certains acides aminés particuliers n'étaient conservés qu'entre les virus du groupe O : tel est le cas de la lysine en position 21 sur un peptide de 26 acides aminés, de la thréonine en position 7 et de l'asparagine en position 11. Ces différences pourraient expliquer l'absence de détection d'antigènes d'enveloppe VIH-1 classiques par un des sérums de la patiente VIH-1_{(VAU)} et également probablement par celui de patients infectés par d'autres virus de groupe O. Globalement, la comparaison entre les séquences d'enveloppe VIH-1 LAI et VIH-1_{(VAU)} a montré une identité de 50 %. La séquence d'enveloppe VIH-1_{(VAU)} a également été comparée à celle d'autres représentants HIV dont les deux membres du groupe O du VIH-1 décrits et séquencés : VIH-1_{ANT70} et VIH-1_{MVP5180}, et SIV. Les résultats de cette analyse, présentés sur le Tableau 1, établissent que le VIH-1_{(VAU)} appartient au groupe O. L'enveloppe VIH-1_{(VAU)} est identique à 70 % à l'enveloppe VIH-1_{ANT70} et identique à 71 % à VIH-1M_{VP5180.} Parmi les sous-types VIH-1 les plus courants, l'identité au niveau de l'enveloppe est comparable, allant de 74 % à 80 %.

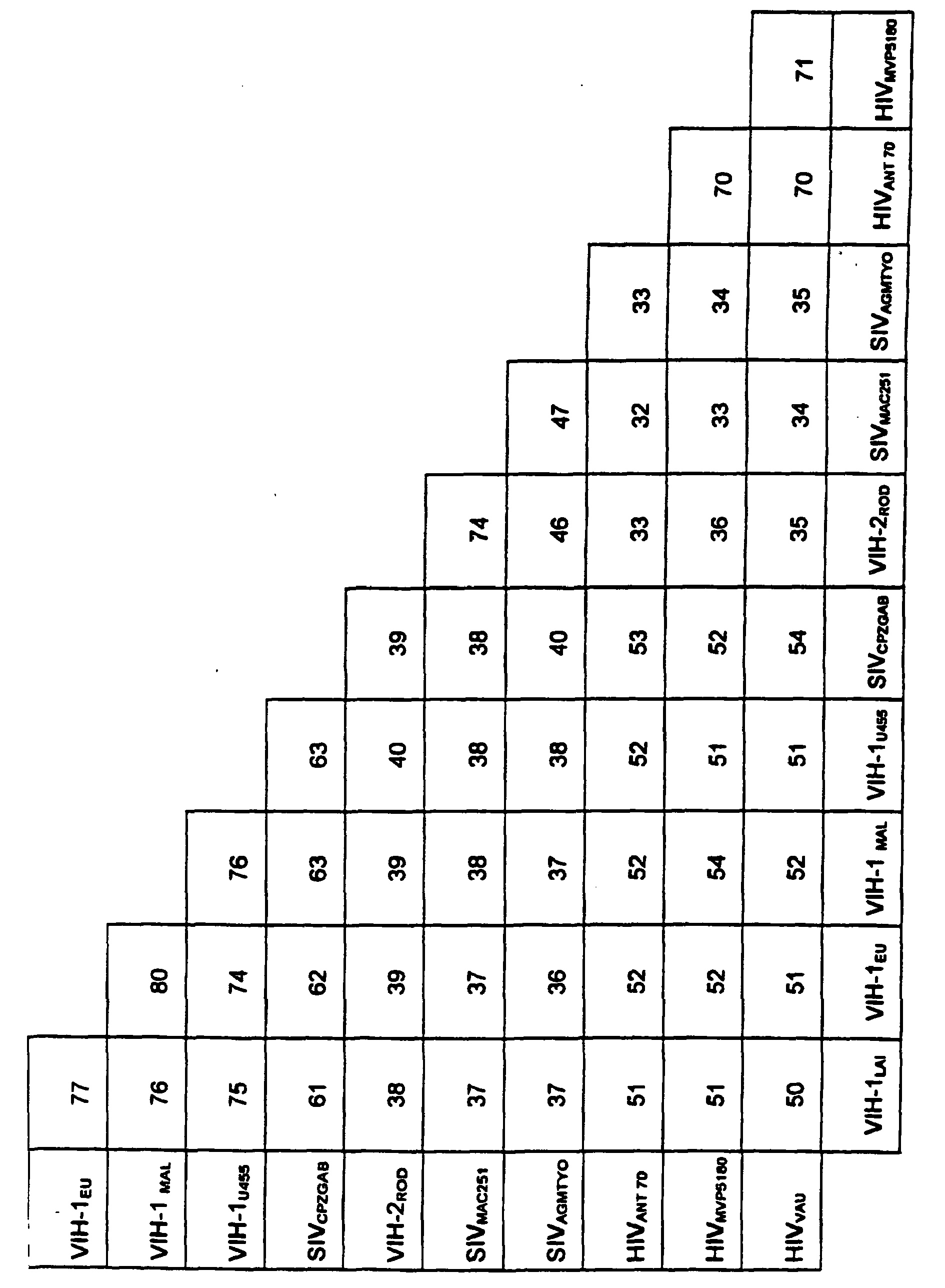

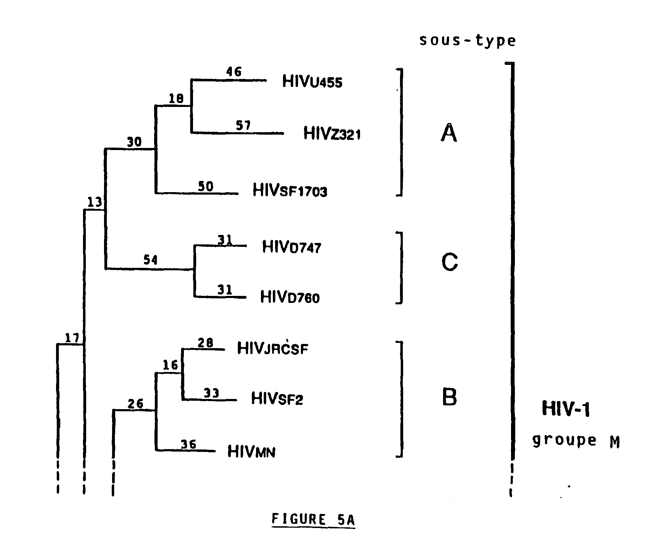

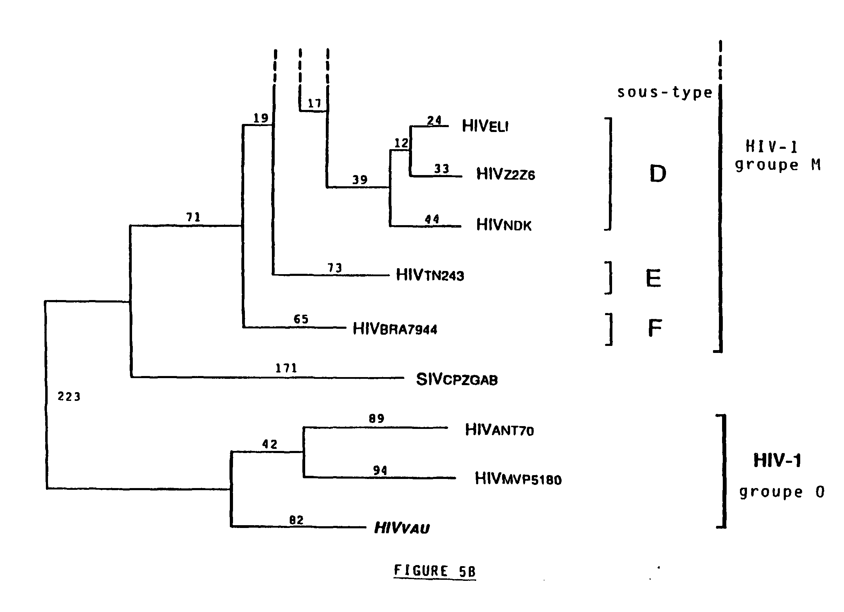

La relation entre le VIH-1_{(VAU)}, d'autres membres de la phylogénie des VIH-1 et les deux virus récemment décrits du groupe 0 a été analysée par construction d'un arbre phylogénétique de parcimonie non pondérée à l'aide de la séquence nucléotidique de la région transmembranaire *env.* Le résultat de cette analyse est représenté sur la fig. 5 dans laquelle les nombres indiquent le nombre de changements nucléotidiques. La figure 5 montre que le VIH-1_{(VAU)} est à peu près équidistant des deux autres virus du groupe O, et que, globalement, ces trois virus semblent être approximativement équidistants les uns des autres. En effet, le nombre de changements nucléotidiques entre le VIH-1_{MVP5180} et le VIH-1_{(VAU)} est de 218 dans le segment de génome analysé, alors que la distance est de 183 entre le VIH-1_{MVP5180} et le VIH-1_{ANT70}, et de 213 entre le VIH-1_{ANT70} et le VIH-1_{(VAU)}. Ce profil de divergence est très similaire à ce qu'on trouve dans l'ensemble des autres sous-types VIH-1, où le nombre de changements nucléotidiques uniques que l'on trouve entre deux sous-types distincts va de 157 (sous-type E au sous-type F) à 219 (sous-type A au sous-type D).

Le tableau 1 montre la comparaison des séquences d'enveloppe de différents virus apparentés au VIH-1. Les nombres indiquent le pourcentage d'identité en acides aminés entre des séquences d'enveloppe, tels qu'ils ont été calculés à l'aide du programme GAP du progiciel GCG-Wisconsin. ^{*}: pour le HIV_{ANT70}, seule la protéine d'enveloppe externe a été utilisée dans la comparaison.

### Compositions comprenant des antigènes VIH-1_{(VAU)}

De façon générale, l'invention concerne toute composition utilisable en détection in vitro de la présence dans un fluide biologique, notamment de personnes ayant été mises au contact du VIH-1_{(VAU)}, ou d'anticorps contre l'un au moins des antigènes VIH-1_{(VAU)}. Cette composition peut être appliquée au diagnostic sélectif de l'infection par un VIH-1 du groupe O, en mettant en oeuvre des techniques de diagnostic telles qu'elles sont décrites dans les demandes de brevet EP 84401.834 et EP 87400.151.4. Dans le contexte de la présente invention, on utilise tout constituant comprenant des déterminants antigéniques capables d'être reconnus par des anticorps produits contre le VIH-1_{(VAU)}, par exemple des antigènes ou peptides recombinants ou peptides synthétisés chimiquement définis à partir de la séquence de l'enveloppe VIH-1_{(VAU)}. A cet égard, l'invention concerne plus particulièrement des compositions contenant l'une au moins des protéines d'enveloppe du virus VIH-1_{(VAU)} On mentionnera, à titre d'exemples de compositions, celles qui contiennent des protéines, glycoprotéines ou peptides de la protéine d'enveloppe correspondant à l'ensemble de la région 590-620 de la protéine gp41 du VIH-1_{(VAU)} ou aux parties de cette région spécifiques du VIH-1_{(VAU)} tels que les peptides -TFIQN- ou -WGCKNR-.

L'invention concerne également des compositions associant des protéines et/ou glycoprotéines et/ou peptides VIH-1_{(VAU)}, recombinants ou synthétiques, avec des protéines et/ou glycoprotéines et/ou peptides VIH-1 et/ou VIH-2 et/ou d'un autre VIH-1 groupe 0 obtenus par extraction ou dans des lysats ou par recombinaison ou par synthèse chimique et/ou de peptides dérivés de ces protéines ou glycoprotéines et susceptibles d'être reconnus par des anticorps induits par le virus VIH-1 et/ou VIH-2 et/ou VIH-1, groupe O.

Les compositions diagnostiques contenant des déterminants antigéniques capables d'être reconnus par des anticorps dirigés contre le VIH-1_{(VAU)}, en particulier les compositions peptidiques, peuvent être incluses dans ou associées à des compositions ou des trousses déjà disponibles pour la détection de l'infection par des rétrovirus VIH-1 et/ou VIH-2, de façon à étendre le spectre de détection des trousses à la détection des rétrovirus VIH-1 du groupe O.

A titre d'exemples non limitatifs:
- soit des protéines du noyau (core), particulièrement les protéines gag, pol, VIH-1 et VIH-2 ou des peptides de celles-ci, et des protéines ou peptides d'enveloppe VIH-1_{(VAU)},
- soit des glycoprotéines d'enveloppe VIH-1, des glycoprotéines d'enveloppe VIH-2 et des glycoprotéines d'enveloppe VIH-1_{(VAU)},
- soit des mélanges de protéines et/ou glycoprotéines VIH-1, de protéines et/ou glycoprotéines VIH-2 et de protéines et/ou glycoprotéines d'enveloppe VIH-1_{(VAU)}.

Il est important de noter que, bien que les anticorps de patients infectés par des virus VIH-1 groupe 0 réagissent fortement avec des antigènes gag et pol de virus VIH-1 groupe M, leur réactivité est pratiquement nulle avec des antigènes d'enveloppe de virus du groupe M. Il est donc important que la composition de la présente invention comprenne au moins une protéine ou un peptide d'enveloppe VIH-1_{(VAU)} de façon à ce que ce virus puisse être détecté avec certitude.

De telles compositions, utilisées en diagnostic, aident par conséquent au diagnostic du sida ou des symptômes qui lui sont associés, qui s'étendent sur un plus large spectre des agents étiologiques responsables. Il va sans dire que l'utilisation de compositions de diagnostic qui ne contiennent que des protéines et/ou glycoprotéines d'enveloppe VIH-1_{(VAU)} n'en reste pas moins utile pour la détection plus sélective de la catégorie de rétrovirus qui peut être tenue pour responsable de la maladie.

### Procédés et trousses de diagnostic d'infections causées notamment par le virus VIH-1_{(VAU)}

La présente invention concerne un procédé de diagnostic in vitro de l'infection par les HIV, agents étiologiques du SIDA et de syndromes apparentés, qui comprend la mise en contact d'un sérum ou d'un autre milieu biologique, provenant d'un patient ou sujet faisant l'objet du diagnostic, avec une composition contenant au moins une protéine, une glycoprotéine ou un peptide VIH-1_{(VAU)}, et la détection d'une réaction immunologique éventuelle. Des exemples de telles compositions ont été décrits plus haut.

Des méthodes préférées mettent en jeu, par exemple, des réactions immunoenzymatiques de type ELISA ou d'immunofluorescence. Les détections peuvent se faire par immunofluoresence directe ou indirecte ou des dosages immunoenzymatiques directs ou indirects.

De telles détections comprennent par exemple :
- le dépôt de quantités déterminées de l'extrait ou des compositions antigéniques visées conformes à la présente invention dans les puits d'une microplaque ;
- l'introduction dans chaque puits d'un sérum dilué ou non, susceptible de contenir les anticorps dont la présence doit être détectée in vitro ;
- l'incubation de la microplaque ;
- le lavage soigneux de la microplaque avec un tampon approprié ;
- l'introduction dans les puits de la microplaque d'anticorps marqués spécifiques anti-immunoglobuline humaine, le marquage étant réalisé par une enzyme choisie parmi celles qui sont capables d'hydrolyser un substrat de telle sorte que ce dernier subit alors une modification de son absorption des radiations, au moins dans une bande de longueur d'onde déterminée, et
- la détection, de préférence de façon comparative par rapport à un témoin, de l'importance de l'hydrolyse du substrat en tant que mesure des risques potentiels ou de la présence effective de l'infection.

La présente invention est également relative à des nécessaires ou trousses de diagnostic de l'infection par le virus VIH-1_{(VAU)}, lesquels comprennent notamment :
- un extrait, une fraction plus purifiée, ou un antigène synthétique dérivé des types de virus indiqués ci-dessus, cet extrait fraction ou antigène marqué, par exemple, de façon radioactive, enzymatique, fluorescente ou autre,
- des anticorps antiimmunoglobulines humaines ou une protéine A (fixés de façon avantageuse sur un support insoluble dans l'eau) telles que des billes par exemple d'agarose) ou des puits de microplaque, etc.),
- éventuellement un échantillon de fluide biologique ou cellules obtenu à partir d'un sujet témoin négatif ;
- des tampons et, le cas échéant, des substrats pour la visualisation du marquage.

L'invention a aussi pour objet des compositions immunogènes capables d'induire la formation d'anticorps reconnaissant des antigènes pouvant être obtenus par synthèse chimique ou par recombinaison.

### Sérologie

La capacité des anticorps sériques de la patiente infectée par le VIH-1_{(VAU)} à réagir avec des préparations antigéniques VIH-1 a été évalué à l'aide de diverses trousses disponibles dans le commerce : SANOFI DIAGNOSTICS PASTEUR, (GENELAVIA MIXT) ABBOTT, WELLCOME, et BEHRING. La réactivité de ces anticorps avec différentes protéines VIH-1 a été examinée à l'aide de la trousse de Westem-blot de SANOFI DIAGNOSTICS PASTEUR, suivant les protocoles recommandés par le fabricant

Plus précisément, le sérum de la patiente a été examiné plusieurs fois à l'aide de trousses ELISA spécifiques VIH-1. Il a d'abord été testé et s'est avéré être positif en 1990, étant noté 7,33 (ce chiffre correspond au rapport de la DO mesurée sur la DO du bruit de fond) avec la trousse de SANOFI DIAGNOSTICS PASTEUR, 3,50 avec la trousse ABBOTT et 2,70 avec la trousse WELLCOME. Lors de l'utilisation des réactifs spécifiques à la fois du VIH-1 et du VIH-2, le sérum a été noté 1,42 avec la trousse Behring et 4,40 avec la trousse WELLCOME.

La capacité du sérum de la patiente à réagir à différentes dates avec différentes protéines de structure VIH-1 a été étudié à l'aide du dosage par immunoempreinte LAV BLOT VIH-1, test commercialisé par SANOFI DIAGNOSTICS PASTEUR. Comme le montre la figure 5 avec tous les échantillons de sérum testés, on n'a remarqué qu'une très faible réactivité du sérum avec les protéines *env* VIH-1 gp160 et gp120. Toutefois, le sérum a réagi fortement avec les protéines gag du VIH-1 p55 (précurseur de gag) et p24 (CA), et avec les produits pol p66 (RT) et p34 (IN). En immunoempreinte VIH-2, on n'a détecté qu'une très faible réactivité avec la p26 gag.

Ceci illustre que la détection d'anticorps spécifiques du groupe O avec des trousses de diagnostic sérique disponibles dans le commerce doit être soigneusement contrôlée. Bien que des anticorps sériques de patients infectés par des virus du groupe O présentent de fortes réactions croisées avec les antigènes gag et pol du groupe M, ils présentent peu ou pas de réactions avec les antigènes d'enveloppe de groupe M. On peut, par conséquent, supposer qu'une proportion significative de ces patients pourrait ne pas être détectée lors de l'utilisation de certaines trousses à base de réactifs antigéniques d'enveloppe du groupe M. En effet, dans une étude préliminaire récente de plusieurs sérums de patients infectés par le groupe O, il a été trouvé que la capacité de détecter des anticorps spécifiques du groupe O était très différente selon la trousse de détection utilisée (Loussert-Ajaka, I., Ly, T.D., Chaix, M.L., Ingrand, D., Saragosti, S., Courroucé, A.M., Brun-Vézinet, F. and Simon, F. (1994). VIH-1/VIH-2 seronegativity in VIH-1 subtype 0 infected patients. Lancet. 343, 1393-1394.). Ceci implique qu'une étude soignée et poussée de la réactivité d'un grand nombre de sérums du groupe O avec toutes les trousses de diagnostic disponibles sur la marché est nécessaire.

### Compositions comprenant des anticorps polyclonaux ou monoclonaux préparés à partir des antigènes recombinants ou synthétiques du virus VIH-1_{(VAU)}.

L'invention concerne un sérum susceptible d'être produit chez l'animal par inoculation à celui-ci du VIH-1_{(VAU)}, particulièrement les épitopes antigéniques du VIH-1_{(VAU)} et plus particulièrement les épitopes antigéniques de la protéine d'enveloppe du virus VIH-1_{(VAU)}. L'invention concerne plus particulièrement les anticorps polyclonaux plus spécifiquement orientés contre chacun des antigènes, notamment protéines ou glycoprotéines du virus. Elle concerne aussi des anticorps monoclonaux produits par différentes techniques, ces anticorps monoclonaux étant orientés respectivement plus spécifiquement contre les différentes protéines du VIH-1_{(VAU)}, particulièrement les protéines d'enveloppe du VIH-1_{(VAU)}.

Ces anticorps polyclonaux ou monoclonaux sont utilisables dans différentes applications. On mentionnera essentiellement leur utilisation pour neutraliser les protéines correspondantes, voire inhiber l'infectivité du virus entier. Ils peuvent également être utilisés par exemple pour mettre en évidence les antigènes viraux dans les préparations biologiques ou pour réaliser des opérations de purification des protéines et/ou glycoprotéines correspondantes, par exemple lors de l'utilisation dans des colonnes de chromatographie d'affinité.

A titre d'exemple, des anticorps anti-enveloppe ou des anticorps anti-gag sont des réactifs utilisables en diagnostic, en particulier pour la détection de particules VIH-1 groupe O par ELISA de capture d'antigène.

L'invention concerne des anticorps dirigés contre un ou des antigènes du virus VIH-1_{(VAU)} produits à partir de séquences d'acides aminés du VIH-1_{(VAU)}. Des techniques d'obtention d'anticorps à partir d'épitopes antigéniques semblables aux épitopes antigéniques du virus VIH-1_{(VAU)} de la présente invention ont été décrites précédemment.

La technique de préparation d'anticorps décrite dans la publication de ULMER et al., 1993 peut être utilisée par l'homme du métier pour préparer les anticorps de la présente invention, les modifications permettant d'adapter cette technique aux antigènes de la présente invention faisant partie des connaissances de l'homme du métier.

### Etude de l'immunoréactivité du peptide vau

L'immunoréactivité du peptide vau a été vérifiée, après avoir préparé des plaques ELISA expérimentales, selon un protocole établi pour un test de screening des anticorps anti-HIV. Ce test repose sur la détection d'une phase solide préparée avec le peptide mimant l'épitope immunodominant de la glycoprotéine d'enveloppe du virus VIH-1 groupe (ou sous-groupe) O, isolat VAU. La mise en oeuvre du test a été calquée sur le protocole proposé par la trousse GENELAVIA® MIXT, avec les réactifs de cette trousse.

Les données expérimentales rassemblées dans les deux tableaux des figures 21 et 22 démontrent que :
a) les quatre sérums prélevés chez des patients contaminés par le virus VIH-1 groupe (ou sous-groupe) O sont très réactifs sur le peptide vau ;
b) les dix sérums censés prélevés chez des patients contaminés par le virus VIH-1 (groupe ou sous-groupe) O, parmi les 19 sérums transmis par l'institut Pasteur de Yacoundé sont également fortement réactifs sur ce même peptide ;
c) les sérums (4 échantillons) prélevés chez des sujets contaminés par le virus VIH-1 sous-type B (en phase aiguë) ne sont pas réactifs sur le peptide vau ;
d) les sérums prélevés chez des donneurs de sang asymptomatiques (48 échantillons testés) ne sont pas réactifs sur le peptide vau ; Ces données expérimentales, bien que limitées (vu la paupicité des échantillons positifs en anticorps VIH-1 groupe (ou sous-groupe 0) attestent de la sensibilité et de la spécificité du peptide sélectionné.

De ce qui précède, il résulte que l'invention concerne aussi la détection du virus VIH-1_{(VAU)} ou d'un variant grâce à la mise en oeuvre des anticorps décrits précédemment dans un procédé faisant appel à différentes étapes, ces étapes étant spécifiquement prévues pour faire apparaître les propriétés caractéristiques du virus VIH-1_{(VAU)}.

L'invention concerne aussi la détection du virus VIH-1 _{(VAU)} par hybridation moléculaire.

D'une façon générale, ce procédé de détection du virus VIH-1(_{VAU}) ou d'un variant dans des échantillons de sérum ou d'autres liquides biologiques ou tissus, obtenus à partir de patients susceptibles d'être porteurs du virus VIH-1_{(VAU)} comprend les étapes suivantes :
- la fabrication d'au moins une sonde éventuellement marquée ;
- au moins une étape d'hybridation conduite dans des conditions permettant l'hybridation par mise en contact de l'acide nucléique de l'échantillon du patient suspect avec ladite sonde marquée et éventuelle immobilisation du complexe formé sur un support solide approprié,
- le cas échéant lavage dudit support solide avec une solution de lavage adéquate,
- la détection dudit complexe et donc de la présence ou non du virus VIH-1_{(VAU)} par méthode appropriée de détection connue de l'homme du métier.

Dans un autre mode de réalisation préféré du procédé selon l'invention, l'hybridation précitée est conduite dans des conditions non stringentes et le lavage du support (membrane) est réalisé dans des conditions adaptées à celles de l'hybridation.

En utilisant une sérologie ou une technique d'amplification génique telle que la Polymerase Chain Reaction (PCR) spécifique, l'importance de l'épidémie VIH-1 du groupe O a été évaluée précisément. Il a été trouvé que 5 à 10 % de patients infectés par un VIH-1 au Cameroun sont en réalité infectés par des virus du groupe O. Toutefois, à part l'isolat de virus décrit ici, la propagation de virus du groupe O en dehors de l'Afrique de l'Ouest Centrale n'a pas été documentée. La patiente chez qui le VIH-1_{(VAU)} a été isolé a toujours vécu en France et n'a jamais voyagé en Afrique. Jusqu'ici, nous n'avons aucune preuve précise concernant l'origine de son infection, mais ce cas indique qu'une certaine propagation des virus du groupe O en Europe s'est déjà produite.

L'invention concerne en outre un procédé de détection et de discrimination dans un échantillon biologique entre des anticorps caractéristiques d'un rétrovirus VIH-1 groupe (ou sous-groupe) O et des anticorps caractéristiques d'un rétrovirus du type VIH-1-M caractérisé par la mise en contact de cet échantillon biologique avec un peptide ne réagissant pas aux anticorps caractéristiques d'un rétrovirus du type VIH-1-M, notamment un choisi parmi les peptides (1), (2), (3), (4), (5bis), (5ter), (9) et (10) ci-dessus décrits.

Egalement, l'invention concerne un procédé de détection et de discrimination dans un échantillon biologique entre des anticorps caractéristiques d'un rétrovirus VIH-1 groupe (ou sous-groupe) O et des anticorps caractéristiques d'un rétrovirus du type VIH-1-M caractérisé par la mise en contact de cet échantillon biologique avec le peptide issu de l'un des virus VIH-1 M pris en considération dans les figures 8 et 9 homologue d'un peptide choisi parmi les peptides (1), (2), (3), (4), (5bis), (5ter), (9) et (10), la séquence de ce peptide homologue résultant des alignements verticaux de ses propres acides aminés successifs eux-mêmes contenus dans la séquence peptidique pertinente relative au virus VIH-1-M correspondant et représentée dans la figure 8 ou 9 avec les acides aminés successifs de la séquence du peptide choisi, telle qu'elle découle aussi de la figure 8 ou 9.

Selon la présente invention, le procédé de détection et de discrimination entre infection par un rétrovirus VIH-1 groupe (ou sous-groupe) 0 et un rétrovirus VIH-1 sous-groupe M est caractérisé par la mise en contact de sérum issu des personnes soumises à un test de diagnostic de SIDA, notamment avec le peptide RILAVERY.

En outre, le procédé de détection d'infection due soit à un rétrovirus VIH-1 sous-groupe O, soit VIH-1 sous-groupe M, est caractérisé par l'utilisation de mélanges de deux catégories de peptides, ceux de la première catégorie correspondant aux peptides (1), (2), (3), (4), (5bis). (5ter) (9) et (10).

Par ailleurs, le procédé de discrimination entre une infection due à un rétrovirus VIH-1-O DUR, et une infection due à un autre type de rétrovirus VIH-1-O, est caractérisé par la mise en contact de l'échantillon biologique étudié avec l'un quelconque des peptides (11) à (15) ou des peptides (17) à (20).

Alternativement il s'agit d'un procédé de discrimination entre une infection par un rétrovirus VIH-1 groupe (ou sous-groupe) O et un rétrovirus VIH-1 sous-groupe M mettant en oeuvre une sérine protéase dont l'action de clivage s'effectue au niveau d'un dipeptide SR et comprenant la détection d'un clivage ou d'un non-clivage de la boucle V3 de la gp 120 du rétrovirus selon que ce rétrovirus est un rétrovirus VIH-1 groupe (ou sous-groupe) 0 ou un rétrovirus VIH-1 sous-groupe M.

L'invention concerne en outre une composition pour la détection et la discrimination dans un échantillon biologique entre un rétrovirus VIH-1 sous-groupe M et un rétrovirus VIH-1 groupe (ou sous-groupe) O, comprenant un mélange de deux catégories de peptides, la première étant notamment ceux identifiés (1), (2), (3), (4), (5bis), (5ter), (9) et (10).

Entrant également dans le cadre de la présente invention les anticorps monoclonaux spécifiques des séquences de chacun des peptides (1) à (20).

L'invention concerne également un plasmide choisi parmi ceux qui ont été déposés à la CNCM le 24 Février 1995 sous les références I-1548, I-1549 et I-1550.

L'invention vise également des acides nucléiques contenant une séquence codant chacun des peptides (1) à (20) définis dans la présente invention.

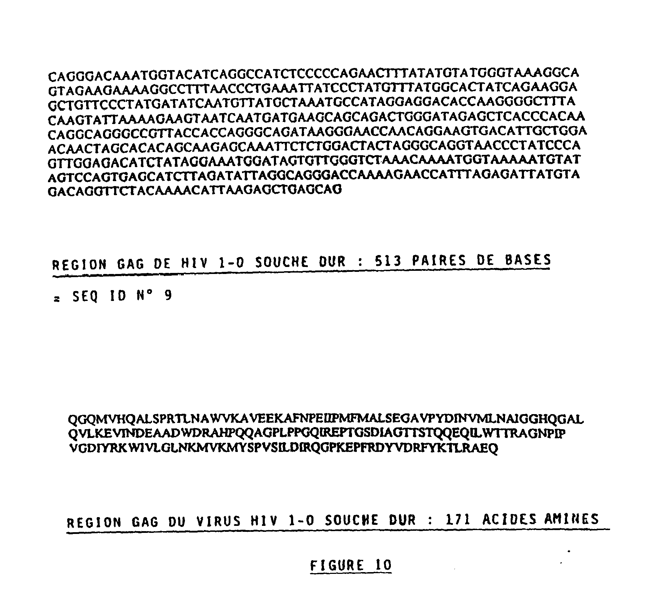

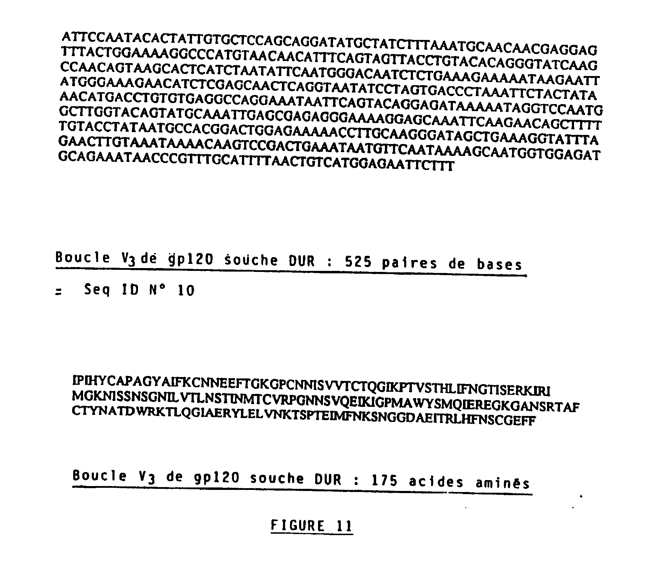

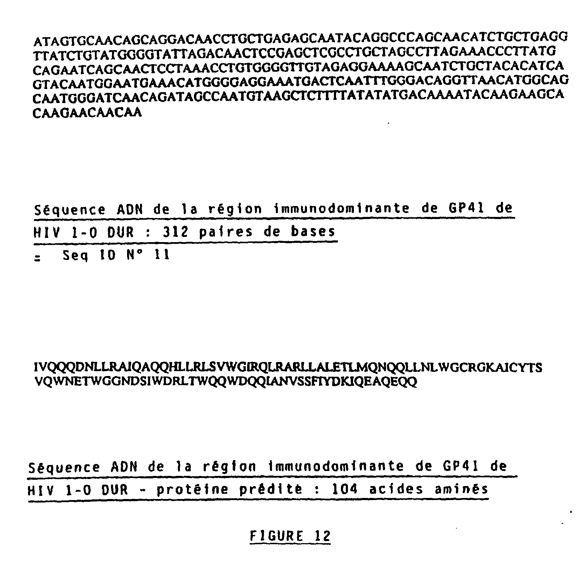

Parmi les séquences d'acides nucléiques préférées on choisira notamment les séquences nucléotidiques représentées aux figures 10, 11 ou 12.

L'invention a également trait aux vecteurs contenant un acide nucléique tel qu'il est défini ci-dessus.

L'invention vise également des cellules susceptibles de contenir l'un quelconque desdits acides nucléiques ou desdits vecteurs.

La présente invention concerne également un virus tel que celui déposé le 23 Février 1995 à la CNCM sous la référence I-1542.

Un virus entrant également dans le cadre de l'invention est un virus de même groupe que le précédent, caractérisé en ce que des peptides consensus de ce virus sont reconnus par des anticorps reconnaissant spécifiquement un polypeptide ou un peptide précédemment défini.

L'ARN génomique de ce virus entre également dans le cadre de l'invention.

Entre également dans le cadre de l'invention, un nécessaire ou trousse de détection d'anticorps dans le sérum ou tout autre échantillon biologique de patient susceptible d'être infecté par un rétrovirus humain du type HIV, caractérisé en ce qu'il comprend :
- au moins un polypeptide ou un peptide ayant notamment pour séquence une des séquences (1) à (20) précédemment décrites.
- des moyens permettant la réaction de formation du complexe immunologique entre le(s) polypeptide(s) ou le(s) peptide(s) et les anticorps éventuellement présents dans l'échantillon biologique à tester, par exemple si besoin un ou plusieurs tampons d'incubation,
- un échantillon témoin négatif,
- des moyens de révélation du complexe antigène/anticorps formé.

Egalement selon l'invention, cette trousse contient, en outre, au moins un polypeptide ou un peptide consensus dérivé d'une autre souche HIV ou d'un polypeptide ou d'un peptide comprenant,
soit une séquence d'acides aminés distincte de la séquence de ce polypeptide ou peptide dans laquelle un ou plusieurs acides aminés sont substitués par d'autres acides aminés sous réserve que le polypeptide ou le peptide conserve sa réactivité avec un antisérum contre le polypeptide ou le peptide consensus,
soit une séquence d'acides aminés dans laquelle un ou plusieurs acides aminés ont été supprimés ou ajoutés sous réserve que le polypeptide ou le peptide conserve sa réactivité avec un antisérum contre le polypeptide ou le peptide consensus.

Préférentiellement, une trousse selon l'invention contiendra, en outre, au moins un polypeptide ou un peptide dérivé d'une autre souche HIV, de préférence la souche HIV-LAI.

L'invention concerne également une composition polypeptidique pour le diagnostic in vitro d'une infection due à un rétrovirus selon l'invention, ou à un de ses variants ce diagnostic s'effectuant dans un échantillon biologique susceptible de contenir les anticorps formés après ladite infection. Cette composition est caractérisée en ce qu'elle comprend au moins l'un des peptides (1) à (20).

L'échantillon biologique peut être constitué notamment de sang, de plasma, de sérum ou de tout autre extrait biologique. Les compositions ci-dessus sont utilisables pour la détection d'anticorps dans un des échantillons biologiques susdits.

L'invention vise donc également une méthode de diagnostic in vitro d'une infection due spécifiquement à un rétrovirus du type HIV, caractérisée par les étapes de :
- mise en contact d'un échantillon biologique susceptible de contenir des anticorps produits à la suite d'une infection par un rétrovirus VIH-1 groupe (ou sous-groupe) O, avec un peptide tel que précédemment défini, ou avec une composition peptidique telle que précédemment définie, dans des conditions appropriées permettant la formation d'un complexe immunologique du type antigène/anticorps.
- détection de l'éventuelle présence du complexe.

L'invention concerne par ailleurs une composition immunogène, caractérisée en ce qu'elle comprend au moins un peptide en association avec un véhicule pharmaceutique acceptable pour la constitution de vaccins.

L'invention se rapporte également à un procédé de préparation des protéines de capside et des glycoprotéines gp41 et gp120 d'une souche rétrovirale selon l'invention, le procédé étant caractérisé par les étapes suivantes :
- lyse des cellules infectées par un rétrovirus VIH-1 selon l'invention et séparation du surnageant et des cellules infectées ou lyse des culots viraux préparés par centrifugation,
- dépôt de l'extrait cellulaire et/ou de l'extrait viral sur immuno-adsorbant contenant des anticorps purifiés, obtenus à partir d'un sérum de sujet infecté par un rétrovirus selon l'invention, et fixés avantageusement sur un support adapté, ledit sérum de sujet infecté ayant la capacité de réagir fortement avec des protéines d'enveloppe du virus selon l'invention,
- incubation en présence d'un tampon et pendant un temps suffisamment long pour obtenir la formation d'un complexe immunologique antigène/anticorps,
- lavage de l'immuno-adsorbant avec un tampon pour éliminer les molécules non retenues sur le support,
- récupération des protéines antigéniques recherchées.

Selon un premier mode de réalisation de ce procédé de préparation, la séparation et la récupération des protéines de capside et des glycoprotéines gp41 et gp120 de VIH-1 DUR sont faites par électrophorèse et par électroréduction des protéines.

Selon un autre mode de réalisation de ce procédé de préparation, la récupération des protéines est obtenue par :
- élution des protéines fixées sur l'immuno-adsorbant ci-dessus,
- purification des produits ainsi élués sur une colonne de chromatographie contenant, fixés sur le support de séparation, des anticorps reconnaissant les protéines de capside et les glycoprotéines gp41 et gp120 de VIH-1 groupe (ou sous-groupe) O DUR.
   Entre également dans le cadre de l'invention, un procédé de production d'un polypeptide ou d'un peptide selon l'invention, ce polypeptide ou peptide étant obtenu
- soit par l'expression d'un acide nucléique de l'invention,
- soit par synthèse chimique, par addition d'acides aminés jusqu'à l'obtention de ce polypeptide ou de ce peptide.
   Les principes et les procédés classiques du génie génétique peuvent être ici utilisés ( « Molecular cloning », Sambrook, Fritsch, Maniatis, CSH 1989)
   Entre également dans le cadre de l'invention, un procédé de production d'un acide nucléique précédemment défini, pouvant être produit soit par isolement à partir d'un virus de l'invention, soit par synthèse chimique, soit en utilisant des techniques d'amplification in vitro des acides nucléiques à partir d'amorces spécifiques.
   Des amorces oligonucléotidiques également selon l'invention ont une séquence consistant en au moins huit nucléotides consécutifs des séquences nucléotidiques suivantes:

ATT CCA ATA CAC TAT TGT GCT CCA -3'
AAA GAA TTC TCC ATG ACT GTT AAA -3'
GGT ATA GTG CAA CAG CAG GAC AAC-3'
AGA GGC CCA TTC ATC TAA CTC-3'

Ces amorces peuvent être utilisées lors d'un processus d'amplification génique, par exemple par PCR ou une technique équivalente, d'une séquence nucléotidique codant pour un peptide de l'invention. Des tests effectués avec ces amorces ont fourni des résultats concluants.

Egalement, l'invention concerne une trousse permettant l'amplification par PCR ou une technique équivalente ci-dessus décrite.

Entre également dans le cadre de la présente invention, un procédé de détection de la présence dans un échantillon biologique d'acide(s) nucléique(s) caractéristique(s) d'une rétrovirus VIH-1 groupe (ou sous-groupe) O DUR, y inclus d'un rétrovirus selon l'invention. Ce procédé comprend une mise en contact d'un ADNc formé à partir d'ARN(s) contenu(s) dans cet échantillon biologique, dans des conditions permettant l'hybridation de cet ADNc avec le génome rétroviral, et la réalisation d'une amplification génique sur cet échantillon viral.

L'invention concerne également un lysat viral tel qu'il est obtenu par lyse des cellules infectées par un virus selon l'invention

Un extrait protéique d'une souche VIH-1 (DUR) (ou VIH-1_{(VAU)}) contenant notamment un polypeptide ou un peptide tel que précédemment défini entre également dans le cadre de l'invention.

L'invention concerne des peptides particuliers issus de structure de VIH-1 groupe (ou sous-groupe) O DUR ou de variants de ce rétrovirus et qui permettent
soit de discriminer, selon le cas ,
- de façon globale entre des VIH-1 appartenant à la catégorie 0 et des VIH-1 appartenant à la catégorie M,
- ou plus spécifiquement entre des virus appartenant au sous-groupe caractéristique du VIH-1 groupe (ou sous-groupe) O DUR et d'autres virus du sous-groupe O,
soit de au contraire de reconnaître la plupart, sinon tous les rétrovirus à la fois du groupe (ou sous-groupe) 0 et du sous-groupe M.

Entrent également dans le cadre de l'invention les peptides correspondants et issus de protéines de structure correspondantes d'autres virus du VIH-1 groupe (ou sous-groupe) O ou VIH-1 sous-groupe M, en particulier ceux dérivés des protéines de structure GAG, gp120 et gp41 dont des parties sont indiquées dans les dessins, ces peptides homologues découlant de leur mise en alignement, comme il résulte aussi des dessins avec les peptides issus du VIH-1 groupe (ou sous-groupe) O DUR , plus particulièrement identifiés dans le présent texte.

Par voie de symétrie, certains peptides homologues peuvent être mis en oeuvre dans ceux des essais qui permettent de procéder aux susdites discriminations, étant alors entendu qu'ils sont alors utilisés en lieu et place des peptides correspondants, issus des protéines de structure GAG, gp120 et gp41.

### Détermination d'oligonucléotides spécifiques du groupe O.

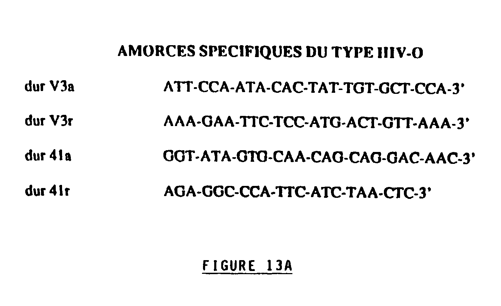

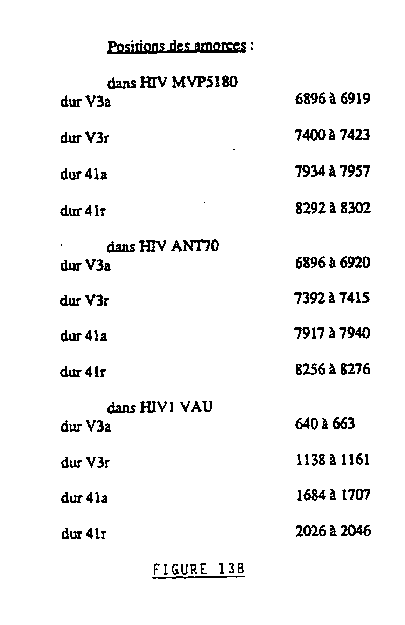

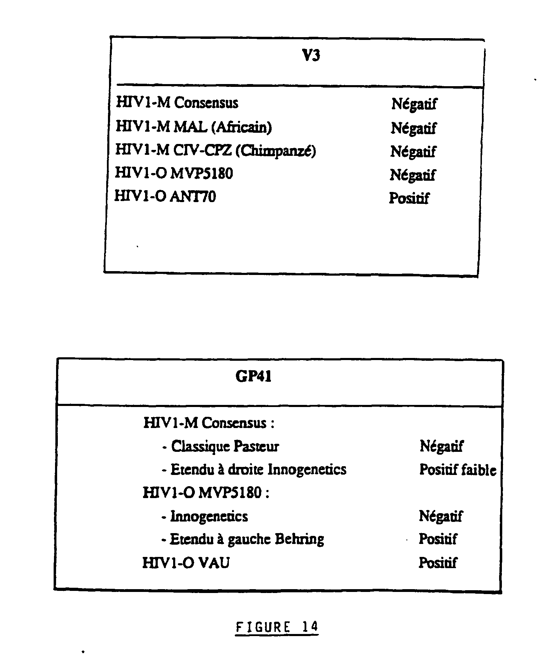

A l'aide de la séquence VAU et de sa corrélation aux séquences MVP5180 et ANT70, on a défini des amorces oligonucléotidiques s'efforçant d'être spécifiques du sous-groupe O dans son entièreté pour la région V3 et la région gp41. Celles-ci ont permis d'amplifier la souche DUR et ont en conséquence constitué une solution au problème d'amplification rencontré. La position ainsi que la séquence de ces amorces HIV sous-groupe O sont représentées à la figure 13. Ces amorces permettent l'obtention d'une bande d'amplification visible en coloration au bromure d'éthidium avec une seule étape de 30 cycles de PCR. Des séquences partielles ont été obtenues:
- GAG : 513 paires de bases (171 acides aminés) = Seq ID N°9
- gp 120 boucle V3 : 525 paires de bases (75 acides aminés) = Seq ID N°10
- gp41 région immunodominante : 312 paires de bases (104 acides aminés) = Seq ID N° 11.

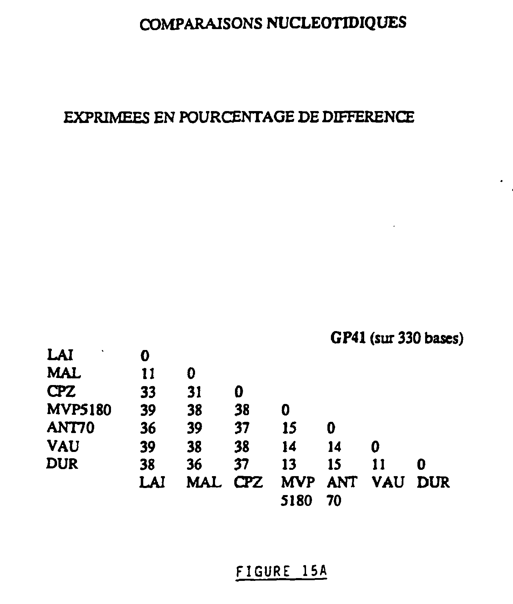

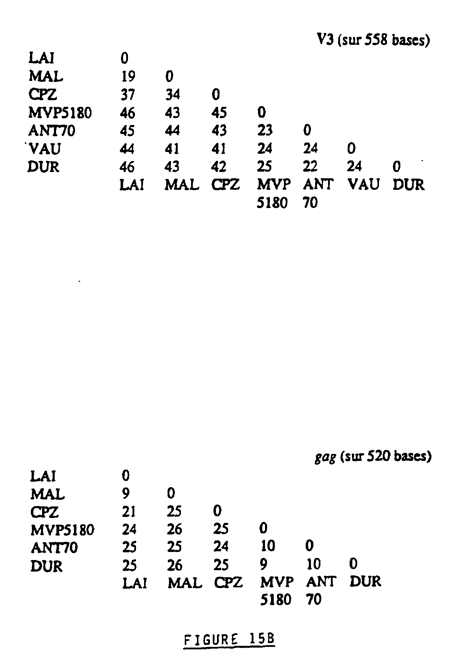

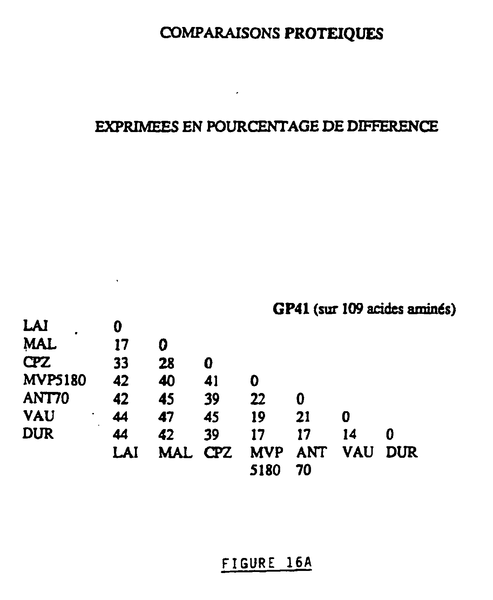

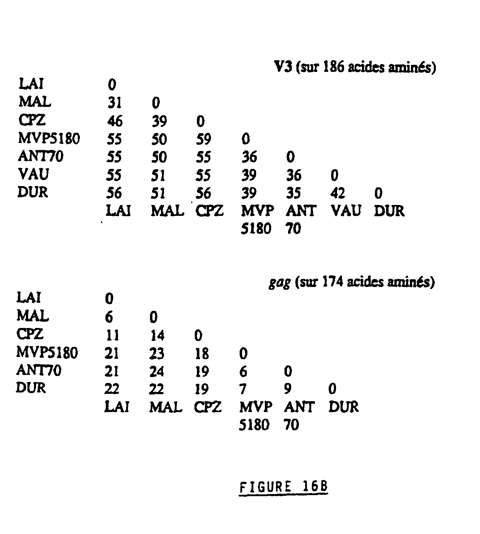

Les comparaisons nucléotidiques (figure 15) et protidiques (figure 16) des séquences DUR aux séquences MVP5180, ANT et VAU pour le sous-groupe O, LAI pour le consensus VIH-1, MAL séquence africaine VIH-1 représentative, et CPZ pour le CIV du chimpanzé gabonais, montrent que DUR est aussi éloigné des autres VIH-1 groupe (ou sous-groupe) O publiés que ceux-ci le sont entre eux.

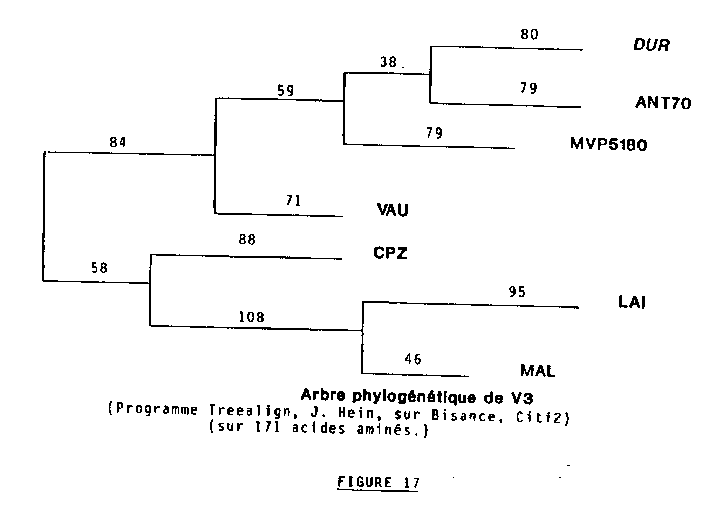

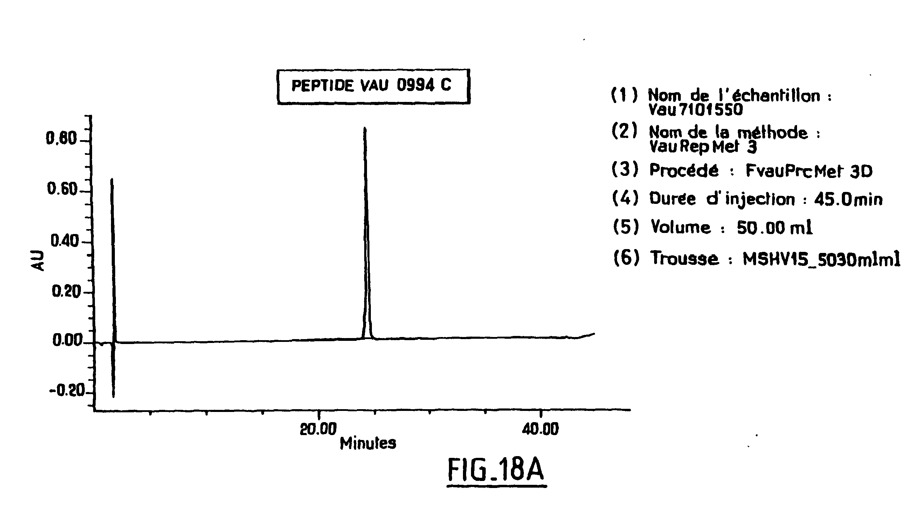

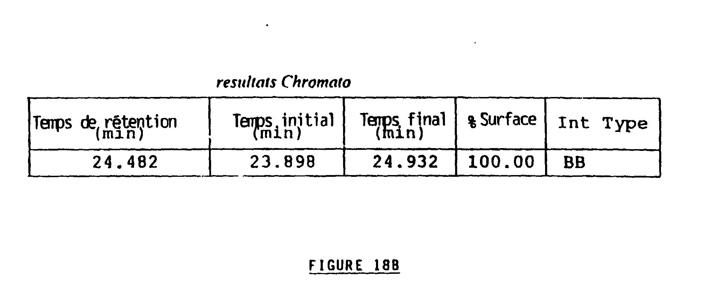

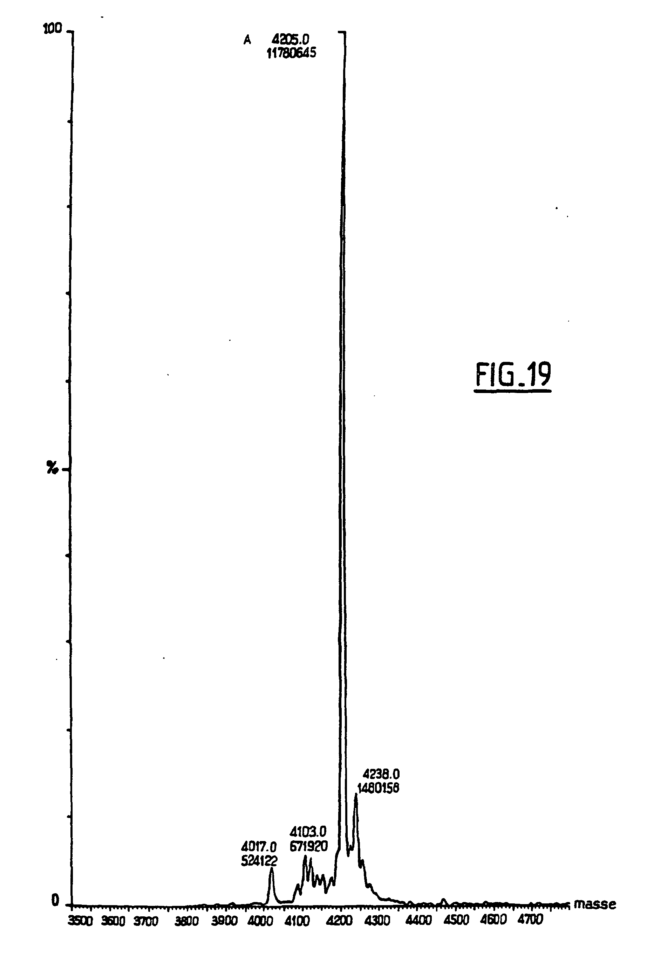

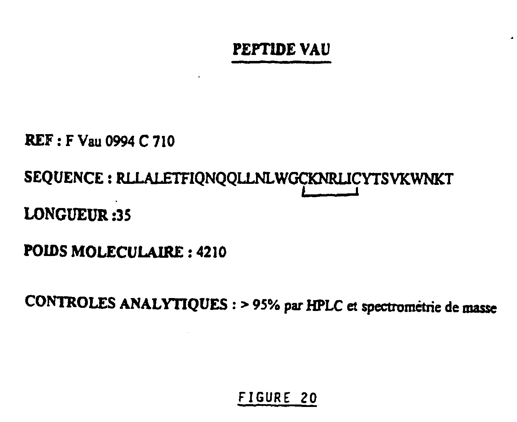

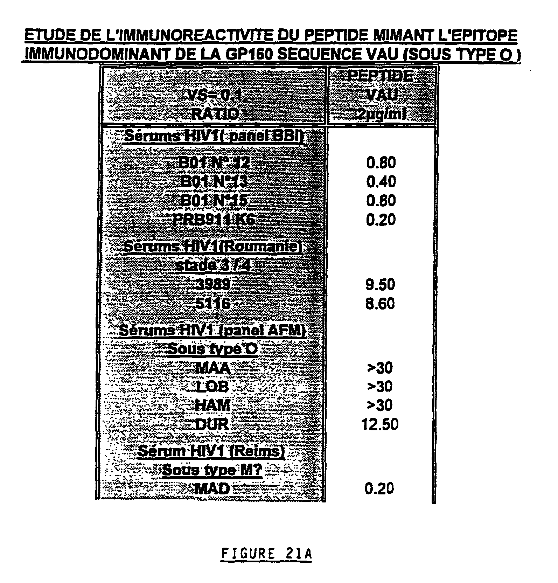

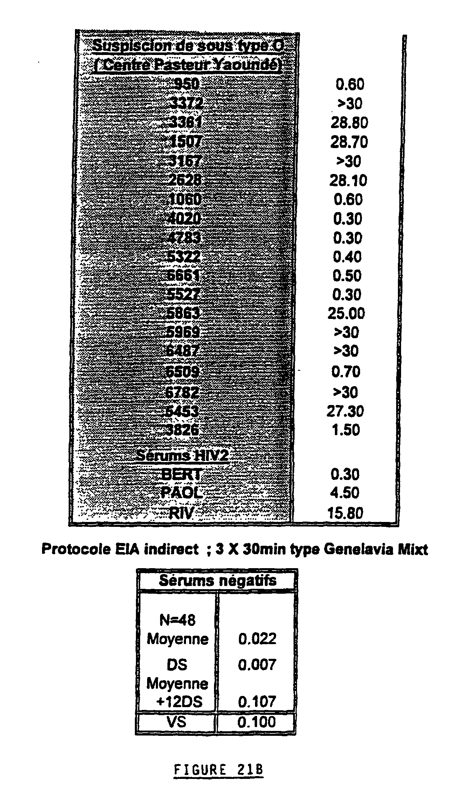

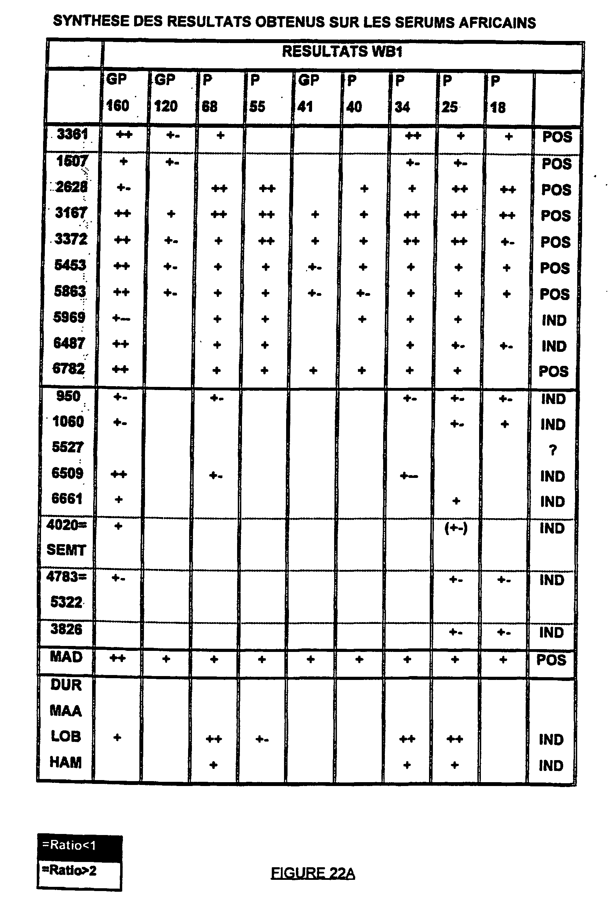

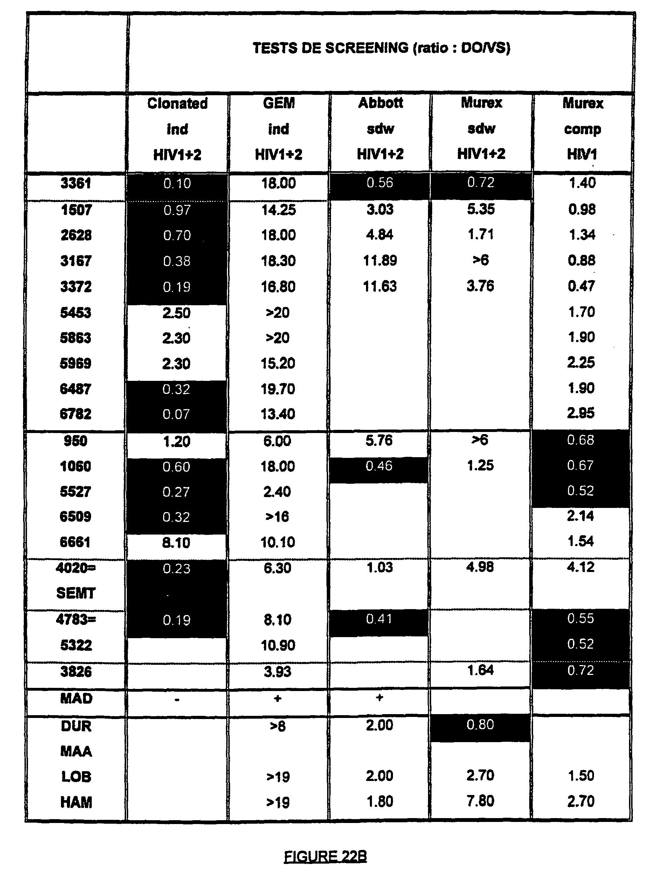

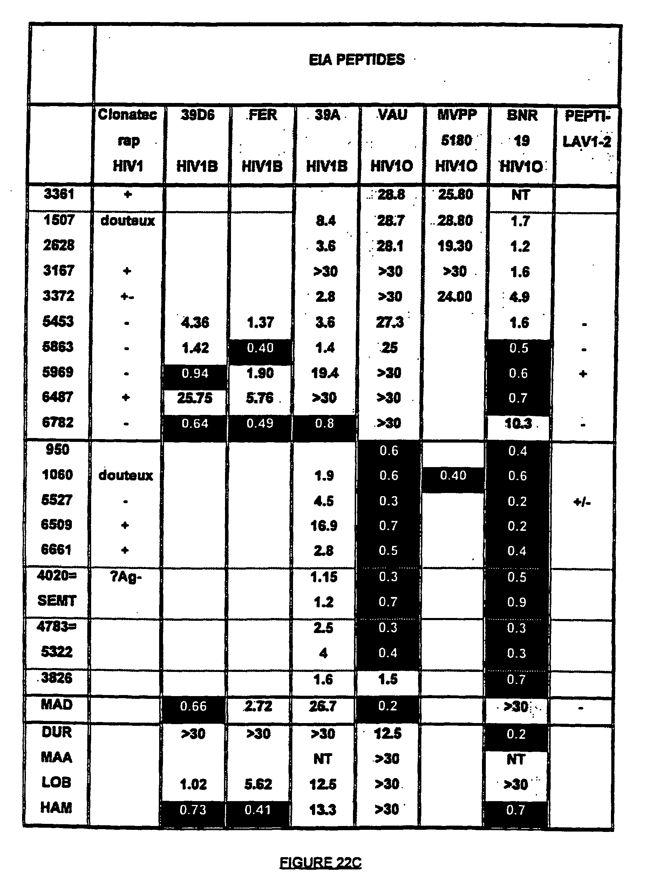

Les différences sont moindres dans la région GAG et maximales dans la région de la boucle V3 de gp120, où la comparaison protéique atteint 40% de différence (figure 16). Les arbres phylogéniques confirment d'une part que la souche DUR fait partie du sous-groupe O, et d'autre part l'importance des différences entre les diverses souches O décrites, sans pour autant que des embranchements de sous-types ne se dégagent nettement (figure 17).

### Comparaison des séquences GAG :

Lors de la comparaison de la séquence GAG obtenue aux deux autres souches O publiées ANT70 et MVP5180 ainsi qu'aux séquences représentatives du groupe M (figure 8), on a pu constater qu'il existe un consensus O sur plusieurs zones, distinct du consensus M dans les mêmes zones. On peut aussi relever deux zones hyper-variables, plus variables pour O que pour M, et quelques différences ponctuelles pour l'une ou l'autre souche. Néanmoins, les régions SPRT....SEGA, MLNAI....KEVIN, GPLPP....QQEQI, VGD....SPV paraissent discriminatives du consensus O versus le consensus M.

Les régions QQA et LWTTRAGNP sont des régions hyper-variables. La souche VIH-1 groupe (ou sous-groupe) O DUR se singularise en trois positions vis-à-vis du consensus M et O (L pour et 2 fois pour E) et prend un acide aminé spécifique en trois positions hyper-variables isolées V position L9 ; A position A77 ; L position 110.

En outre, il est possible de définir dans la région GAG des segments communs au groupe O et au groupe M tels que SPRTLNAWVK, GSDIAGTTST et QGPKEPFRDYVDRF.

### Comparaison des séquences de la boucle V3

Cette étude comparative a révélé des différences considérables atteignant 56% de différences protéiques avec le consensus VIH-1 sous-groupe M, et 35 à 42% avec les autres VIH-1 groupe (ou sous-groupe) O.

Les alignements de séquences peptidiques des régions de la boucle V3 de gp120 et de la région immunodominante de gp41 est donné à la figure 9. La séquence de l'intérieur de la boucle V3 de la souche DUR diffère substantiellement de celle du consensus VIH-1 sous-groupe M. Elle partage le motif GPMAWYSM avec les souches VAU et ANT70 mais pas avec la souche MVP qui présente deux substitutions : R pour A et R pour Y.

Les parties gauche et droite du reste de la boucle V3 sont nettement différentes de tous les autres HIV connus et ne laissent pas supposer d'autres réactivités croisées. De plus, la boucle V3 de DUR est plus longue d'un acide aminé que les autres séquences O, elles-mêmes plus longues d'un autre acide aminé que les séquences de groupe VIH-1 M.

### Comparaison des alignements concernant la région immunodominante de la gp41

La « mini boucle » de la souche DUR, de séquence CRGKAIC, s'est avérée être très spécifique de cette souche : elle constituerait une épitope (voir figure 9). En outre, cette séquence serait susceptible d'intervenir dans la modification des conditions de dépliage de la glycoprotéine gp41, et en conséquence dans l'infectuosité de la souche.

Une longue séquence de 11 acides aminés flanquant à gauche cette boucle est identique à la séquence VAU. On peut noter un polymorphisme de la souche DUR pour une position S ou T selon les clones analysés.

Sont également représentés dans la figure 9 des peptides correspondants issus d'autres souches rétrovirales connues.

La souche DUR permet également de définir un consensus HIV sous-groupe O de la région gp41 dont plusieurs régions homologues suffisamment longues pourraient être utilisées. Ces régions homologues sont entre autres: RL^{*}ALET, QNQQ, LWGC, CYTV (* représentant un acide aminé variable) .

### Corrélations sérologiques:

L'antisérum anti-DUR ne réagit pas avec les peptides de la boucle V3 du consensus VIH-1-M, du VIH-1 MAL, du VIH-1 CPZ ou du VIH-1 groupe (ou sous-groupe) O MVP5180, mais réagit cependant avec le peptide de la boucle V3 de l'VIH-1-O ANT70. Concernant la région immunodominante gp41, il ne réagit pas avec le consensus VIH-1 sous-groupe M « classique », mais réagit cependant, faiblement mais de façon surprenante, avec le consensus VIH-1 sous-groupe M étendu à droite.

### REFERENCES

Agut, H., Candotti, D., Rabanel, B., Huraux, J., Remy, G., Ingrand, D., Tabary, T., Chippaux, C., Chamaret, S., Guétard, D., Dauguet, C. et Montagnier, L. (1992). Isolation of atypical VIH-1-related rétrovirus from AIDS patient (L'isolement de rétrovirus atypique apparenté au VIH-1 de patient atteint du SIDA). Lancet. 340, 681-682.

Alizon, M., Wain-Hobson, S., Montagnier, L. et Sonigo, P. (1986). Genetic variability of the AIDS virus : nucleotide séquence analysis of two isolates from African patients (Variabilité génétique du virus du SIDA : analyse de la séquence nucléotidique de deux isolats de patients africains). Cell. 46, 63-74**.**

Barré-Sinoussi, F., Chermann, J.C., Rey, F., Nugeyre, M. T., Chamaret, S., Gruest, J., Dauguet, C., Axler-Blin, C., Brun-Vezinet, F., Rouzioux, C., Rozenbaum, W. et Montagnier, L. (1983). Isolation of a T-lymphotropic retrovirus from a patient at risk for acquired immune deficiency syndrome (AIDS) (Isolement d'un rétrovirus lymphotrope T d'un patient présentant un risque de symptôme de l'immunodéficience acquise (SIDA)). Science. 220, 868-871.

Benn, S., Rutledge, R., Folks, T. et al, e. (1985). Genomic heterogeneity from AIDS retroviral isolates from North America and Zaïre (Hétérogénéité génomique d'isolats rétroviraux de SIDA de l'Amérique du Nord et du Zaïre). Science. 230, 949-951.

Clavel, F. et Chameau, P. (1994). Fusion from without directed by Human immunodeficiency virus particles (Fusion de l'extérieur dirigée par des particules du virus de l'immunodéficience humaine). J. Virol. 68, 1179-1185.

Clavel, F., Guétard, D., Brun-Vézinet. F., Chamaret, S., Rey, M. A., Santos-Ferreira, M. O., Laurent, A. G., Dauguet, C., Katlama, C., Rouzioux, C., Klatzmann, D., Champalimaud, J. L. et Montagnier, L. (1986). Isolation of a new human rétrovirus from West African patients with AIDS (Isolement d'un nouveau rétrovirus humain de patients ouest-africains atteints du SIDA). Science. 233, 343-346.

De Cock, K. M., Adjorlolo, G., Epkini, E., Sibailly, T., Kouadio, J., Maran, M., Brattegaard, K., Vetter, K., Doorly, R. et Gayle, H. (1993). Epidemiology and transmission of VIH-2 : why there is no VIH-2 epidemic (Epidémiologie et transmission de VIH-2 : pourquoi il n'y a aucune épidémie de VIH-2). JAMA. 270, 2083-2086.

De Leys, R., Vanderborght, B., Vanden Haesevelde, M., Heyndrickx, L., van Geel, A., Wauters, C., Bemaerts, R., Saman, E., Nijs, P. et Willems, B. (1990). Isolation and partial characterization of an unusual human immunodeficiency rétrovirus from two persons of west-central African origin (Isolement et caractérisation partielle d'un rétrovirus inhabituel de l'immunodéficience humaine de deux personnes originaires de l'Afrique de l'Ouest Centrale). J Virol. 64, 1207-1216.

Gnann, J., Cormick, J., Michell, S., Nelson, J. et Oldstone, M. (1987). Synthetic peptide immunoassay distinguishes HIV type 1 and type 2 infections (L'immunodosage par peptide de synthèse distingue les infections de HIV du type 1 et du type 2. Science. 237, 1346-1349.

Grez, M., Dietrich, U., Balfe, P., von Briesen, H., Maniar, J., Mahambre, G., Delwart, E., Mullins, J. et Rubsamen-Waigmann, H. (1994). Genetic analysis of Human Immunodeficiency virus type 1 and 2 (VIH-1 and VIH-2) mixed infections in India reveals a recent spread of VIH-1 and VIH-2 from a single ancestor for each of these viruses (L'analyse génétique d'infections mixtes du virus de l'immunodéficience humaine du type 1 et 2 (VIH-1 et VIH-2) en Inde révèle une propagation récente de VIH-1 et de VIH-2 à partir d'un seul ancêtre pour chacun de ces virus). J. Virol. 68, 2161-2168.

Gürtler, L. G., Hauser, P. H., Eberle, J., von Brunn, A., Knapp, S., Zekeng, L., Tsague, J. M. et Kaptue, L. (1994). A new subtype of Human Immunodeficiency Virus type 1 (MVP-5180) from Cameroon (Un nouveau sous-type de virus de l'immunodéficience humaine du type 1 (MVP-5180) du Cameroun). J Virol. 68, 1581-1585.

Harada, S., Koyanagi, Y. et Yamamoto, N. (1985). Infection of HTLV-III/LAV in HTLV-I-carrying cells MT-2 and MT-4 and application in a plaque assay (Infection de HTLV-III/LAV dans des cellules MT-2 et MT-4 porteuses de HTLV-1 et application dans un dosage sur plage). Science. 229, 563-566.

Hirt, B. (1967). Selective extraction of polyoma DNA from infected mouse cell cultures (Extraction sélective d'ADN de polyome à partir de cultures de cellules de souris infectées). J. Mol. Biol. 26, 365-369.

Huct, T., Cheynier, R., Meyerhans, A., Roclants, G. et Wain-Hobson, S. (1990). Genetic organization of a chimpanzee lentivirus related to VIH-1 (Organisation génétique d'un lentivirus de chimpanzé apparenté au VIH-1). 345, 356-359.

Javaherian, K, Langlois, A. J., La Rosa, G. J., Profy, A. T., Bolognesi, D. P., Herlihy, W. C., Putney, S. D. et Matthews, T. J. (1990). Broadly neutralizing antibodies elicited by the hypervariable neutralizing déterminant of VIH-1 (Anticorps neutralisant un spectre large induit par le déterminant neutralisant hypervariable de VIH-1). Science. 250, 1590-1593.

Javaherian, K, Langlois, A J., Mc Danal, C., Ross, K. L., Eckler, L. 1., Jellis, C. L., Profy, A. T., Rusche, J. R., Bolognesi, D. P., Putney, S. D. et Matthews, T. J. (1989). Principal neutralizing domain of the human immunodeficiency virus type 1 envelope protein (Domaine neutralisant principal de la protéine d'enveloppe du virus de l'immunodéficience humaine de type 1). Proc. Natl. Acad. Sci. USA. 86, 6768-6772.

Louwagie, J., McCutchan, F., Peeters, M., Brennan, T., Sanders-Buell, E., Eddy, G., van der Groen, G., Fransen, K, Gershy-Damet, G. et Deleys, R. (1993). Phylogenetic analysis of gag genes from 70 international VIH-1 isolates provides evidence for multiple genotypes (L'analyse phylogénétique de gènes gag de 70 isolats internationaux de VIH-1 fournit la preuve de génotypes multiples). AIDS. 7, 769-80.

Louwagie, J., McCutchan, F., Van der Groen, G., Peeters, M., Fransen, K, Piot, P., Gershy-Damet, G., Roelants, G., Van Heuverswyn, II. et Eddy, G. (1992). Genetic comparison of VIH-1 isolates from Africa, Europe, and North America (Comparaison génétique d'isolats de VIH-1 de l'Afrique, de l'Europe et de l'Amerique du Nord). AIDS Res Hum Retroviruses. 8, 1467-9.

Matsushita, S. M., Robert-Guroff, M., Rusche, J., Koito, A., Hattori, T., Hoshino, H., Javaherian, K, Takatsuki, K. et Putney, S. (1988). Characterization of a Human immunodeficiency virus neutralizing monoclonal antibody and mapping of the neutralizing epitope. (Caractérisation d'un anticorps monoclonal neutralisant le virus de l'immunodéficience humaine et cartographie de l'épitope neutralisant). J. Virol 62, 2107-2114.

NKENGASONG, J.N. et al., AIDS 1993, Vol. 7, N° 11, pp. 1536-1538.

Rey, M. A., Krust, B., Laurent, A. G., Montagnier, L. et Hovanessian, A. G. (1989). Characterization of human immunodeficiency virus type 2 envelope glycoproteins : dimerization of the glycoprotein precursor during processing (Caractérisation des glycoprotéines d'enveloppe du virus de l'immunodéficience du type 2 : dimérisation du précurseur glycoprotéique au cours de la maturation). J. Virol. 63, 647-658.

ULMER J.B. et al., Science Vol. 259, Mars 1993, pp. 1745-1749.

Vanden Heasevelde, M., Decourt, J. L., de Leys, R. J., Vanderborght, B., van der Groen, G., van Heuverswijn, H. et Saman, E. (1994). Genomic cloning and complete sequence analysis of a highly divergent African Human Immunodeficiency Virus isolate (Clonage génomique et analyse de la séquence complète d'un isolat africain très divergeant du virus de l'immunodéficience humaine). J Virol. 68, 1586-1596.

Vartanian, J.-P., Meyerhans, A, Asjö, B. et Wain-Hobson, S. (1991). Selection, recombination, and G→A hypermutation of VIH-1 genomes (Sélection, recombinaison, et hypermutation G→A de génomes de VIH-1). J. Virol. 65, 1779-1788.

Wain-Hobson, S., Sonigo, P., Danos, O., Cole, S. et Alizon, M. (1985). Nucleotide séquence of the AIDS virus, LAV (Séquence nucléotidique du virus du SIDA, LAV). Cell 40, 9-17.

## Revendications

1. Protéine de rétrovirus VIH-1 groupe (ou sous-groupe) O, ou polypeptide ou peptide comprenant au moins une partie de ladite protéine, susceptible d'être reconnue par des anticorps isolables à partir de sérum obtenu suite à une infection par une souche VIH-1 groupe O VAU, ou une souche VIH-1 groupe (ou sous groupe) O DUR.

2. Protéine, polypeptide ou peptide selon la revendication 1, **caractérisé en ce que** ladite protéine est une protéine du virus VIH-1 groupe (ou sous-groupe) O (DUR), déposé le 23 février 1995 à la CNCM sous la référence I-1542 ou un polypeptide ou peptide comprenant au moins une partie de ladite protéine ou un peptide dont la séquence se distingue de celle du précédent par substitution,délétion ou addition d'acides aminés, ce peptide distinct conservant néanmoins les caractéristiques antigéniques du précédent.

3. Peptide selon la revendication 1 ou 2, comportant au moins 4 acides aminés consécutifs dont la séquence entière en acides aminés consécutifs est contenue dans la séquence GAG représentée à la figure 8 ou dans une séquence GAG immunologiquement semblable issue d'un variant du virus VIH-1 groupe (ou sous-groupe) O DUR, ladite séquence immunologiquement semblable étant reconnue par des anticorps reconnaissant aussi de façon spécifique au moins l'une des séquences AHPQQA, LWTTRAGNP contenues dans la séquence GAG de la figure 8.

4. Peptide selon la revendication 3, **caractérisé en ce qu'**il consiste en un peptide dont la séquence en acides aminés est contenue, soit dans l'une des séquences suivantes:
SPRTLNAWVKAVEEKAFNPEIIPMFMALSEGA (1),
MLNAIGGHOGALQVLKEVIN (2),
GPLPPGQIREPTGSDIAGTTSTQQEQI (3),
IPVGDIYRKWIVLGLNKMVKMYSPVSILDI (4),
QGPKEPFRDYVDRFYKTKLAE (5),
AHPQQA (5 bis),
LWTTRAGNP(5ter),
soit dans la séquence immunologiquement semblable correspondante, ce peptide comportant au moins 4 acides aminés consécutifs de l'une desdites séquences.

5. Peptide selon la revendication 4, **caractérisé en ce qu'**il consiste en un peptide dont la séquence en acides aminés est contenue soit dans l'une des séquences suivantes:
SPRTLNAWVK (6),
GSDIAGTTST (7),
QGPKEPFRDYVDRF (8),
soit dans la séquence immunologiquement semblable correspondante, ce peptide comportant au moins quatre acides aminés consécutifs de l'une desdites séquences.

6. Peptide selon la revendication 4, **caractérisé en ce qu'**il contient la séquence en acides aminés suivante: NPEI (9).

7. Peptide selon la revendication 4, **caractérisé en ce qu'**il contient la séquence en acides aminés suivante: AVEEKAFNPEIIPMFM (10).

8. Peptide selon la revendication 2, comportant au moins 4 acides aminés consécutifs, dont la séquence entière est contenue dans la séquence de la région de la boucle V3 de la gp120 représentée à la figure 9 ou dans la séquence correspondante immunologiquement semblable, issue d'un variant du virus VIH-1 groupe (ou sous-groupe) O DUR, ladite séquence immunologiquement semblable étant reconnue par des anticorps reconnaissant aussi de façon spécifique au moins l'une des séquences:
KEIKI (12),
EREGKGAN (13),
CVRPGNNSVKEIKI (14),
QIEREGKGANSR (15).

9. Peptide selon la revendication 8 contenant:
a) soit la séquenceCVRPGNNSVKEIKIGPMAWYSMQIEREGKGANSRTAFC (11) ou une partie de cette séquence qui contienne au moins 4 acides aminés,
b) soit une séquence d'acides aminés distincte de la séquence de a) dans laquelle un ou plusieurs acides aminés sont substitués par deux acides aminés sous réserve que le peptide conserve sa réactivité avec un antisérum contre le susdit peptide,
c) soit une séquence d'acides aminés distincte selon a) ou b) dans laquelle un ou plusieurs acides aminés ont été supprimés ou ajoutés sous réserve que le peptide conserve sa réactivité avec un antisérum contre le peptide de a),
d) soit la séquence ou partie de séquence immunologiquement semblable correspondante.

10. Peptide selon la revendication 9 qui contient la séquence KEIKI (12).

11. Peptide selon la revendication 9 qui contient la séquence EREGKGAN (13).

12. Peptide selon la revendication 9 ou 10 qui contient soit la séquence d'acides aminés CVRPGNNSVKEIKI (14), soit la séquence QIEREGKGANSR (15).

13. Peptide selon la revendication 9, qui comprend la séquence GPMAWYSM (16).

14. Peptide selon la revendication 2, comportant au moins 4 acides aminés consécutifs, dont la séquence entière est contenue dans la séquence de la région immunodominante de la gp41 représentée à la figure 9 ou dans la séquence correspondante immunologiquement semblable, issue d'un variant du virus VIH-1 groupe (ou sous-groupe) O DUR, ladite séquence immunologiquement semblable étant reconnue par des anticorps reconnaissant aussi de façon spécifique au moins l'une des séquences:
RLLALETLMQNQQL (17),
LNLWGCRGKAICYTSVQWNETWG (18),
CRGKAI (19),
SVQWN (20),
RLLALETLMQNQQLLNLWGCRGKAICYTS (21),
QNQQLLNLWGCRGKAICYISVQWN (22).

15. Peptide selon la revendication 14, contenant la séquence RLLALETLMQNQQL (17) LNLWGCRGKAICYTSVQWNETWG (18) ou partie de ce peptide contenant:
a) soit la séquence CRGKAI (19), soit la séquence SVQWN (20) dans laquelle Q est le cas échéant remplacé par un acide aminé différent, néanmoins différent aussi de K, soit les deux séquences à la fois,
b) soit une séquence d'acides aminés distincte de la séquence de a) dans laquelle un ou plusieurs acides aminés sont substitués par deux acides aminés sous réserve que le peptide conserve sa réactivité avec un antisérum contre le peptide de a),
c) soit une séquence d'acides aminés distincte selon a) ou b) dans laquelle un ou plusieurs acides aminés ont été supprimés ou ajoutés sous réserve que le peptide conserve sa réactivité avec un antisérum contre le peptide de a),
d) soit dans la séquence ou partie de séquence immunologiquement semblable correspondante.

16. Peptide selon la revendication 15, **caractérisé en ce que** sa séquence N terminale qui contient au moins 8 acides aminés, n'est pas immunologiquement reconnue par des anticorps formés contre la séquence RILAVERY contenue dans la région immunodominante de la gp41 de la souche VIH-1-LAI.

17. Peptide selon la revendication 15, **caractérisé en ce qu'**il n'est pas reconnu par des anticorps formés contre le peptide SGKLIC de la souche VIH-1-LAI.

18. Peptide selon la revendication 15, **caractérisé en ce qu'**il contient l'une ou l'autre des deux séquences suivantes:
RLLALETLMONQQLLNLWGCRGKAICYTS (21),
QNQQLLNLWGCRGKAICYTSVQWN (22).

19. Séquence nucléotidique, plus particulièrement ADN et fragments d'ADN clonés pouvant être obtenus à partir de l'ARN, de ADNc ou d'amorces utilisables pour l'amplification génique, dérivés de l'ARN ou de l'ADN du rétrovirus VIH-1 groupe (ou sous-groupe) O, ladite séquence nucléotidique étant **caractérisé en ce qu'**il comprend la séquence correspondant à l'une des séquences SEQ ID NOS :9, 10 ou 11, ainsi que toute portion de cette séquence notamment les séquences codant les protéines polypeptides ou peptides de l'une quelconque des revendications 4 à 18 ou variant de cette portion susceptible de s'hybrider avec l'ADN ou l'ARN correspondant du virus VIH-1 groupe (ou sous-groupe) O.

20. Séquence nucléotidique selon la revendication 19, **caractérisée en ce qu'**il s'agit d'ADN ou de fragments d'ADN obtenus à partir de l'ARN, de ADNc ou d'amorces pour l'amplification génique, dérivés de l'ARN ou de l'ADN du rétrovirus VIH-1 (DUR), ladite séquence nucléotidique comprenant l'enchaînement correspondant à SEQ ID NOS :9, 10 ou 11, ainsi que toute portion de cette séquence ou variant de cette portion susceptible d'hybrider avec l'ADN ou l'ARN correspondant du virus VIH-1 (DUR).

21. Oligonucléotide comprenant au moins 9 nucléotides, tel qu'obtenu à partir d'une séquence nucléotidique selon l'une quelconque des revendications 19 à 20, susceptible d'être utilisé comme amorce pour l'amplification génique d'un rétrovirus VIH-1 groupe (ou sous-groupe) O.

22. Oligonucléotide selon la revendication 21 ayant une séquence consistant en au moins neuf nucléotides consécutifs des séquences nucléotidiques suivantes:
ATT CCA ATA CAC TAT TGT GCT CCA -3',
AAA GAA TTC TCC ATG ACT GTT AAA -3',
GGT ATA GTG CAA CAG CAG GAC AAC-3',
AGA GGC CCA TTC ATC TAA CTC-3'.

23. Oligonucléotide selon la revendication 22 **caractérisé en ce qu'**il peut être utilisé lors d'un processus d'amplification génique d'une séquence nucléotidique codant un peptide selon l'une quelconque des revendications 2 à 18.

24. Séquence de nucléotides utilisable comme sonde, **caractérisé en ce qu'**elle hybride dans des conditions d'hybridation de forte stringence avec l'ADN produit par amplification génique au moyen d'amorces selon l'une quelconque des revendications 21 à 23.

25. Composition pour la détection de la présence ou non d'un rétrovirus VIH-1 groupe (ou sous-groupe) O, notamment le rétrovirus VIH-1 (VAU) et/ou VIH-1 (DUR) dans des échantillons de sérum ou d'autres liquides ou tissu biologique obtenu à partir de patients suspectés d'être porteurs d'un rétrovirus VIH-1 groupe (ou sous-groupe) O, ladite composition étant **caractérisée en ce qu'**elle comprend au moins une sonde obtenue à partir d'une séquence nucléotidique issue du génome du virus VIH-1 (DUR), l'ADN de VIH-1 (DUR) contenant la région ou une partie de la région env et une partie de la région GAG du virus VIH-1 (DUR) tel que défini la revendication 19 ou 20 et/ou une sonde obtenue à partir d'une séquence nucléotidique issue du génome du virus VIH-1 (VAU).

26. Composition selon la revendication 25, **caractérisée en ce que** ladite composition comprend également une sonde obtenue à partir d'une séquence nucléotidique issue de VIH-1 n'appartenant pas au sous groupe O et/ou de VIH-2.

27. Composition pour la détection de la présence ou non d'un rétrovirus VIH-1 groupe (ou sous-groupe) O, notamment le rétrovirus VIH-1 (VAU) et/ou le rétrovirus VIH-1 groupe (ou sous-groupe) O (DUR) dans un échantillon biologique, ladite composition étant **caractérisée en ce qu'**elle comprend au moins deux séquences nucléotidiques selon l'une quelconque des revendications 19 et 20, et au moins deux séquences nucléotidiques qui comprennent la séquence correspondant à SEQ ID NO:5, respectivement issues du génome des virus VIH-1 (DUR) et VIH-1 (VAU), utilisables en tant qu'amorces d'amplification notamment par PCR de l'ADN et/ou de l'ARN de rétrovirus VIH-1 du sous-groupe O et en particulier de VIH-1 (VAU) et VIH-1 (DUR).

28. Séquence nucléotidique **caractérisée en ce qu'**il s'agit d'une séquence d'ARN correspondant à une séquence d'ADN selon l'une quelconque des revendications 19 à 24.

29. Composition pour la détection *in vitro* de la présence dans un échantillon biologique humain d'anticorps anti-VIH-1 (VAU) et anti-VIH-1 (DUR), ladite composition comprenant au moins un antigène comprenant une protéine, une glycoprotéine, un polypeptide ou un peptide de la protéine d'enveloppe d'un rétrovirus VIH-1 (VAU) tel que défini dans la revendication 1, et/ou de la séquence comprenant l'enchaînement correspondant à SEQ ID NOS :9, 10 ou 11, ainsi que toute portion de cette séquence ou variant de cette portion susceptible de s'hybrider avec l'ADN ou l'ARN correspondant du virus VIH-1 (DUR).

30. Composition selon la revendication 29, **caractérisée en ce qu'**elle comprend en outre un antigène tel qu'une protéine, une glycoprotéine, un polypeptide ou un peptide d'un virus VIH-1 n'appartenant pas au sous-groupe et/ou d'un virus VIH-2 ou un peptide dérivé d'un virus VIH-1 n'appartenant pas au sous-groupe O et/ou d'un virus VIH-2 ayant un épitope susceptible d'être reconnu par les anticorps induits par le virus VIH-1 n'appartenant pas au sous-groupe O et/ou le virus VIH-2.

31. Composition selon la revendication 30, **caractérisée en ce que** les protéines et/ou glycoprotéines de VIH-1 n'appartenant pas au sous-groupe et/ou de VIH-2 sont des protéines gag, pol ou des peptides de celles-ci.

32. Composition selon la revendication 31, **caractérisée en ce que** les protéines et/ou glycoprotéines de VIH-1 n'appartenant pas au sous-groupe O et/ou de VIH-2 sont des glycoprotéines d'enveloppe.

33. Anticorps susceptible de reconnaître une protéine, un peptide ou un polypeptide dérivé de ladite protéine selon l'une quelconque des revendications 1 à 18.

34. Procédé de diagnostic *in vitro* d'une infection causée par le virus VIH-1 (VAU) et/ou par le virus VIH-1 (DUR), ledit procédé comprenant:
- la mise en contact d'un sérum ou d'un autre milieu biologique provenant d'un malade faisant l'objet du diagnostic avec au moins une des protéines, glycoprotéines d'enveloppe du virus VIH-1 (DUR) et/ou VIH-1 (VAU) ou d'un polypeptide ou d'un peptide issu d'une de ces protéines ou glycoprotéines respectivement selon l'une quelconque des revendications 2 à 18 et selon la revendication 1 ou une composition selon l'une quelconque des revendications 29 à 32, et
- la détection d'une réaction immunologique.

35. Réactif nécessaire à la réaction de Western blot (immunoempreinte) ou d'ELISA comportant une protéine ou glycoprotéine d'enveloppe du virus VIH-1 (DUR) et/ou VIH-1(VAU) ou d'un polypeptide ou d'un peptide issu d'une de ces protéines ou glycoprotéines respectivement selon l'une quelconque des revendications 2 à 18 et selon la revendication 1 ou une composition selon l'une quelconque des revendications 29 à 32

36. Utilisation d'une séquence nucléotidique selon la revendication 19 ou 20 pour induire *in vivo* la synthèse d'anticorps dirigés contre l'antigène codé par ladite séquence.

37. Composition immunogène selon l'une quelconque des revendications 29 à 32, capable d'induire des anticorps chez l'animal.

38. Trousse (kit) de diagnostic pour la détection *in vitro* sur un échantillon biologique, d'une infection par un rétrovirus VIH-1 du sous groupe O, par exemple d'un rétrovirus VIH-1 VAU), et/ou VIH-1 (DUR), **caractérisée en ce qu'**elle comprend:
- des amorces selon l'une quelconque des revendications 21 à 23 pour l'amplification génique d'un rétrovirus VIH-1 du sous-groupe O,
- des réactifs nécessaires à la réaction d'amplification génique.

39. Trousse de détection *in vitro,* sur un échantillon biologique, d'un rétrovirus VIH-1 du sous-groupe O, **caractérisée en ce qu'**elle comprend comme sonde éventuellement marquée, au moins une séquence nucléotidique selon l'une des revendications 19 à 23 et 28 ou une composition selon l'une quelconque des revendications 25 à 27, et éventuellement une autre sonde nucléotidique selon l'une quelconque des revendications 19 à 23 ou composition selon l'une quelconque des revendications 25 à 27, éventuellement immobilisée sur un support solide.

40. Trousse selon la revendication 39, **caractérisé en ce qu'**elle comprend également les réactifs nécessaires à la mise en oeuvre d'une hybridation.

41. Procédé de détection et de discrimination dans un échantillon biologique entre des anticorps caractéristiques d'un rétrovirus VIH-1 groupe (ou sous-groupe) O et des anticorps caractéristiques d'un rétrovirus VIH-1 sous-groupe M **caractérisé par** la mise en contact de cet échantillon biologique avec un peptide choisi parmi les peptides (1), (2), (3), (4), (5bis), (5ter), (9) et (10).

42. Procédé de détection et de discrimination dans un échantillon biologique entre des anticorps caractéristiques d'un rétrovirus VIH-1 groupe (ou sous-groupe) O et des anticorps caractéristiques d'un rétrovirus VIH-1 sous-groupe M **caractérisé par** la mise en contact de cet échantillon biologique avec un peptide issu de l'un des virus VIH-1 sous groupe M pris en considération dans les figures 8 et 9 et homologue d'un peptide choisi parmi ceux de la revendication 41, la séquence de ce peptide homologue résultant des alignements verticaux de ses propres acides aminés successifs,eux-mêmes contenus dans la séquence peptidique pertinente relative au virus VIH-1 sous-groupe M correspondant et représentée dans la figure 8 ou 9 avec les acides aminés successifs de la séquence du peptide choisi, telle qu'elle découle aussi de la figure 8 ou 9.

43. Procédé de détection et de discrimination entre infection par un rétrovirus VIH-1 groupe (ou sous-groupe) O et du type VIH-1 sous-groupe M **caractérisé par** la mise en contact de sérums issus des personnes soumises à un test de diagnostic de SIDA, avec le peptide RILAVERY.

44. Procédé de détection d'infection due soit à un rétrovirus VIH-1 groupe (ou sous-groupe) O, soit VIH-1 sous-groupe M, **caractérisé par** l'utilisation de mélanges de deux catégories de peptides, ceux de la première catégorie correspondant à ceux identifiés dans la revendication 41.

45. Procédé de discrimination entre une infection due à un rétrovirus VIH-1 groupe (ou sous-groupe) O DUR ou variant, et une infection due à un autre type de rétrovirus VIH-1 groupe (ou sous-groupe) O, **caractérisé par** la mise en contact de l'échantillon biologique étudié avec l'un quelconque des peptides suivants: les peptide (11), (12), (13), (14), (15), (17), (18), (19) et (20).

46. Vecteur contenant un acide nucléique dont la séquence nucléotidique correspond à l'une quelconque des séquences des revendications 19 à 24.

47. Vecteur selon la revendication 46, **caractérisé en ce que** c'est un plasmide.

48. Plasmide choisi parmi ceux qui ont été déposés à la CNCM le 24 Février 1995 sous les références I-1548, I-1549 et I-1550.

49. Cellule contenant un acide nucléique dont la séquence nucléotidique correspond à l'une quelconque des séquences des revendications 46 ou 47.

50. Virus déposé le 23 Février 1995 à la CNCM sous la référence I-1542.

51. Virus de même type ou sous type que le virus de la revendication 58 **caractérisé en ce que** des peptides consensus de ce virus sont reconnus par des anticorps reconnaissant spécifiquement un peptide selon l'une quelconque des revendications 2 à 18.

52. Trousse de détection *in vitro* d'anticorps contre HIV, contenant au moins un peptide selon l'une quelconque des revendications 2 à 18.

53. Trousse selon la revendication 52, contenant en outre au moins un peptide consensus dérivé d'une autre souche HIV comprenant:
- soit une séquence d'acides aminés distincte de la séquence de ce peptide dans laquelle un ou plusieurs acides aminés sont substitués par d'autres acides aminés sous réserve que le peptide conserve sa réactivité avec un antisérum contre le peptide consensus,
- soit une séquence d'acides aminés dans laquelle un ou plusieurs acides aminés ont été supprimés ou ajoutés sous réserve que le polypeptide ou le peptide conserve sa réactivité avec un antisérum contre le peptide consensus.

54. Trousse selon la revendication 52 ou 53, **caractérisée en ce que** l'autre souche HIV est une souche HIV-LAI.

55. Procédé de discrimination entre une infection par un rétrovirus VIH-1 groupe (ou sous-groupe) O et un rétrovirus VIH-1 sous groupe M mettant en oeuvre une sérine protéase dont l'action de clivage s'effectue au niveau d'un dipeptide SR et comprenant la détection d'un clivage ou d'un non clivage de la boucle V3 de la gp 120 du rétrovirus selon que ce rétrovirus est un rétrovirus VIH-1 groupe (ou sous-groupe) O ou un rétrovirus VIH-1 sous-groupe M.

56. Lysat viral tel qu'il est obtenu par lyse des cellules infectées par un virus selon la revendication 50 ou 51.

57. Extrait protéique de souche VIH-1 O (DUR) contenant notamment un peptide antigénique selon l'une quelconque des revendications 2 à 18.

58. Composition pour la détection et la discrimination dans un échantillon biologique entre un rétrovirus VIH-1 sous-groupe M et un rétrovirus VIH-1 groupe (ou sous-groupe) O, comprenant un mélange de deux catégories de peptides, la première étant ceux identifiés dans la revendication 41.

59. Peptide selon la revendication 4, **caractérisé en ce qu'**il consiste en un peptide dont la séquence en acides aminés est contenue, soit dans l'une des séquences suivantes:
IGGHQGALQ (23),
REPTGSDI (24),
soit dans une séquence immunologiquement semblable correspondante, ce peptide comportant au moins 4 acides aminés consécutifs de l'une desdites séquences.

60. Peptide selon la revendication 3, **caractérisé en ce qu'**il consiste en un peptide dont la séquence en acides aminés est contenue dans la séquence d'acides aminés suivante:
INDEAADWD (25),
soit dans une séquence immunologiquement semblable correspondante, ce peptide comportant au moins 4 acides aminés consécutifs de ladite séquence.

61. Acide nucléique codant les peptides des revendications 59 et 60.

62. Composition comprenant au moins un acide nucléique selon la revendication 61.

63. Utilisation d'au moins un acide nucléique selon la revendication 61 pour la détection et la discrimination entre des souches VIH-1 groupe M et VIH-1 groupe O.
